# EUROPÄISCHE PATENTSCHRIFT

(11) **EP 2 188 269 B1**
(45) Veröffentlichungstag und Bekanntmachung des Hinweises auf die Patenterteilung: **07.05.2014**
(21) Anmeldenummer: 08803005.1
(22) Anmeldetag: 12.08.2008
(51) Int. Cl.: C07D 243/08, C07D 295/14, C07C 311/29

(54) **ARYLSULFONAMIDE MIT ANALGETISCHER WIRKUNG**
ARYL SULFONAMIDES WITH ANALGESIC ACTIVITY
ARYLSULFONAMIDES A EFFET ANALGESIQUE

(30) Priorität: 14.08.2007 WO PCT/EP2007/058408; 21.02.2008 WO PCT/EP2008/052157; 26.02.2008 EP 08102043
(43) Veröffentlichungstag der Anmeldung: 26.05.2010
(73) Patentinhaber: Boehringer Ingelheim International GmbH, 55216 Ingelheim am Rhein (DE)
(72) Erfinder: HAUEL, Norbert, 55216 Ingelheim Am Rhein (DE); CECI, Angelo, 55216 Ingelheim Am Rhein (DE); DOODS, Henri, 55216 Ingelheim Am Rhein (DE); KAUFFMANN-HEFNER, Iris, 55216 Ingelheim Am Rhein (DE); KONETZKI, Ingo, 55216 Ingelheim Am Rhein (DE); SCHULER-METZ, Annette, 55216 Ingelheim Am Rhein (DE); WALTER, Rainer, 55216 Ingelheim Am Rhein (DE)
(74) Vertreter: Simon, Elke Anna Maria
(86) Internationale Anmeldenummer: PCT/EP2008/060562
(87) Internationale Veröffentlichungsnummer: WO 2009/021944

(56) Entgegenhaltungen:
- WO-A-02/053516
- WO-A-03/106428
- WO-A-2006/036664
- WO-A-2006/048209

## Beschreibung

Gegenstand der vorliegenden Erfindung sind Verbindungen der allgemeinen Formel **I** 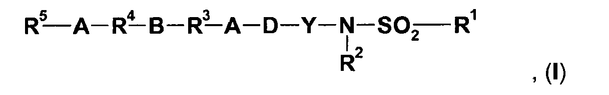 in der A, B, D, Y, **R¹, R², R³, R⁴** und **R⁵** wie nachstehend erwähnt definiert sind, deren Enantiomere, deren Diastereomere, deren Gemische und deren Salze, insbesondere deren physiologisch verträgliche Salze mit organischen oder anorganischen Säuren oder Basen, welche wertvolle Eigenschaften aufweisen, deren Herstellung, die die pharmakologisch wirksamen Verbindungen enthaltenden Arzneimittel, deren Herstellung und deren Verwendung. Strukturell ähnliche Analgetika werden in den Patentschriften WO2006/048209, WO02/053516,WO03/106428 und WO2006/36664 beschrieben.

### DETAILLIERTE BESCHREIBUNG DER ERFINDUNG

In der obigen allgemeinen Formel **I** bedeuten in einer ersten Ausführungsform
- **A**: eine Bindung,
- **B**: eine Bindung,
- **D-Y**: zusammen eine Gruppe ausgewählt aus 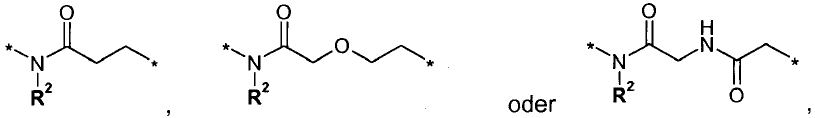
- **R¹**: die Gruppe 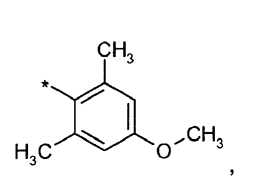
- **R²**: H oder C₁₋₃-Alkyl-, wobei jede Methylengruppe mit bis zu zwei und jede Methylgruppe mit bis zu drei Fluoratomen substituiert sein kann, oder auch H₃C-C(O)-,
- **R³**: eine C₄₋₆-Cycloalkylengruppe, die durch einen, zwei oder drei Reste **R^{3.1}** substituiert sein kann,
- **R^{3.1}**: -CH₃, -C₂H₅, *iso*-Propyl, *tert*-Butyl, -OH, F, Cl, Br, I,
- **R⁴**: einen gesättigten 6- oder 7-gliedrigen Diaza-Heterocyclus,
- **R⁵**: C₁₋₃-Alkyl- oder C₃₋₅-Cycloalkyl bedeutet,
deren Enantiomere, deren Diastereomere, deren Gemische und deren Salze, insbesondere deren physiologisch verträgliche Salze mit organischen oder anorganischen Säuren oder Basen.

Eine zweite Ausführungsform der vorliegenden Erfindung besteht in den Verbindungen der obigen allgemeinen Formel **I**, in denen
- **A**: eine Bindung,
- **B**: eine Bindung,
- **D-Y**: zusammen eine Gruppe ausgewählt aus 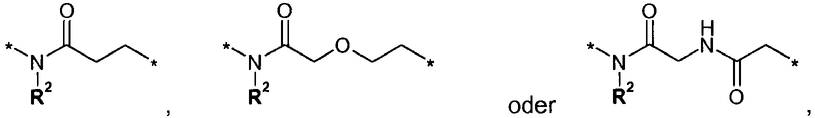
- **R¹**: die Gruppe 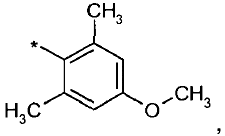
- **R²**: H oder C₁₋₃-Alkyl-, wobei jede Methylengruppe mit bis zu zwei und jede Methylgruppe mit bis zu drei Fluoratomen substituiert sein kann, oder auch H₃C-C(O)-,
- **R³**: eine C₄₋₆-Cycloalkylengruppe,
- **R⁴**: einen gesättigten 6- oder 7-gliedrigen Diaza-Heterocyclus,
- **R⁵**: C₁₋₃-Alkyl- oder C₃₋₅-Cycloalkyl bedeutet,
deren Enantiomere, deren Diastereomere, deren Gemische und deren Salze, insbesondere deren physiologisch verträgliche Salze mit organischen oder anorganischen Säuren oder Basen.

Eine dritte Ausführungsform der vorliegenden Erfindung besteht in den Verbindungen der obigen allgemeinen Formel **I,** in denen **A, B, D, Y, R¹, R², R⁴** und **R⁵** wie voranstehend unter der ersten Ausführungsform erwähnt definiert sind und
- **R³**: eine C₄₋₆-Cycloalkylengruppe, die durch einen, zwei oder drei Reste **R^{3.1}** substituiert sein kann, und
- **R^{3.1}**: -CH₃, -C₂H₅, *iso*-Propyl, *tert*-Butyl, -OH, F, Cl, Br oder I bedeutet,
mit der Maßgabe, dass die voranstehend erwähnte C₄₋₆-Cycloalkylengruppe in 1,3-Stellung mit dem restlichen Molekül verknpüft ist,
deren Enantiomere, deren Diastereomere, deren Gemische und deren Salze, insbesondere deren physiologisch verträgliche Salze mit organischen oder anorganischen Säuren oder Basen.

Eine vierte Ausführungsform der vorliegenden Erfindung besteht in den Verbindungen der obigen allgemeinen Formel **I,** in denen **A, B, D, Y, R¹, R², R⁴** und **R⁵** wie voranstehend unter der zweiten Ausführungsform erwähnt definiert sind und
- **R³**: eine C₄₋₆-Cycloalkylengruppe, die durch einen, zwei oder drei Reste **R^{3.1}** substituiert sein kann, und
- **R^{3.1}**: -CH₃, -C₂H₅, *iso*-Propyl, *tert*-Butyl, -OH, F, Cl, Br oder I bedeutet,
mit der Maßgabe, dass die voranstehend erwähnte C₄₋₆-Cycloalkylengruppe in 1,3-Stellung mit dem restlichen Molekül verknpüft ist,
deren Enantiomere, deren Diastereomere, deren Gemische und deren Salze, insbesondere deren physiologisch verträgliche Salze mit organischen oder anorganischen Säuren oder Basen.

Eine fünfte Ausführungsform der vorliegenden Erfindung besteht in den Verbindungen der obigen allgemeinen Formel **I**, in denen
- **A**: eine Bindung,
- **B**: eine Bindung,
- **D-Y**: zusammen eine Gruppe ausgewählt aus 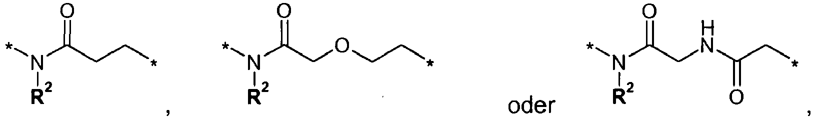
- **R¹**: die Gruppe 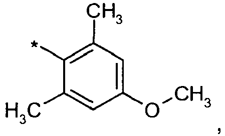
- **R²**: H oder C₁₋₃-Alkyl-, wobei jede Methylengruppe mit bis zu zwei und jede Methylgruppe mit bis zu drei Fluoratomen substituiert sein kann, oder auch H₃C-C(O)-,
- **R³**: eine C₄₋₆-Cycloalkylengruppe, die durch einen, zwei oder drei Reste **R^{3.1}** substituiert sein kann,
- **R^{3.1}**: -CH₃, -C₂H₅, *iso*-Propyl, *tert*-Butyl, -OH, F, Cl,
- **R⁴**: einen gesättigten 6- oder 7-gliedrigen Diaza-Heterocyclus,
- **R⁵**: H-, C₁₋₃-Alkyl- oder C₃₋₅-Cycloalkyl bedeutet,
deren Enantiomere, deren Diastereomere, deren Gemische und deren Salze, insbesondere deren physiologisch verträgliche Salze mit organischen oder anorganischen Säuren oder Basen.

Eine sechste Ausführungsform der vorliegenden Erfindung besteht in den Verbindungen der obigen allgemeinen Formel **I**, in denen
- **A**: eine Bindung,
- **B**: eine Bindung,
- **D-Y**: zusammen eine Gruppe ausgewählt aus 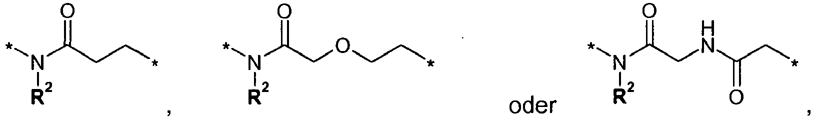
- **R¹**: die Gruppe 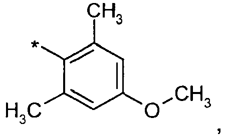
- **R²**: H oder C₁₋₃-Alkyl-, wobei jede Methylengruppe mit bis zu zwei und jede Methylgruppe mit bis zu drei Fluoratomen substituiert sein kann, oder auch H₃C-C(O)-,
- **R³**: eine C₄₋₆-Cycloalkylengruppe,
- **R⁴**: einen gesättigten 6- oder 7-gliedrigen Diaza-Heterocyclus,
- **R⁵**: H-, C₁₋₃-Alkyl- oder C₃₋₅-Cycloalkyl bedeutet,
deren Enantiomere, deren Diastereomere, deren Gemische und deren Salze, insbesondere deren physiologisch verträgliche Salze mit organischen oder anorganischen Säuren oder Basen.

Eine siebte Ausführungsform der vorliegenden Erfindung besteht in den Verbindungen der obigen allgemeinen Formel **I,** in denen **A, B, D, Y, R¹, R², R⁴** und **R⁵** wie voranstehend unter der fünften Ausführungsform erwähnt definiert sind und
- **R³**: eine C₄₋₆-Cycloalkylengruppe, die durch einen, zwei oder drei Reste **R^{3.1}** substituiert sein kann, und
- **R^{3.1}**: -CH₃, -C₂H₅, *iso*-Propyl, *tert*-Butyl, -OH, F oder Cl bedeutet,
mit der Maßgabe, dass die voranstehend erwähnte C₄₋₆-Cycloalkylengruppe in 1,3-Stellung mit dem restlichen Molekül verknpüft ist,
deren Enantiomere, deren Diastereomere, deren Gemische und deren Salze, insbesondere deren physiologisch verträgliche Salze mit organischen oder anorganischen Säuren oder Basen.

Eine achte Ausführungsform der vorliegenden Erfindung besteht in den Verbindungen der obigen allgemeinen Formel **I,** in denen **A, B, D, Y, R¹, R², R⁴** und **R⁵** wie voranstehend unter der fünften Ausführungsform erwähnt definiert sind und
- **R³**: eine C₄₋₆-Cycloalkylengruppe, die durch einen, zwei oder drei Reste **R^{3.1}** substituiert sein kann, und
- **R^{3.1}**: -CH₃, -C₂H₅, *iso*-Propyl, *tert*-Butyl, -OH, F oder Cl bedeutet,
mit der Maßgabe, dass die voranstehend erwähnte C₄₋₆-Cycloalkylengruppe in 1,3-Stellung mit dem restlichen Molekül verknpüft ist,
deren Enantiomere, deren Diastereomere, deren Gemische und deren Salze, insbesondere deren physiologisch verträgliche Salze mit organischen oder anorganischen Säuren oder Basen.

Als besonders bevorzugte Verbindungen der obigen allgemeinen Formel **I** seien beispielsweise folgende genannt:

| **Nr.** | **Struktur** |
|---|---|
| (1) | 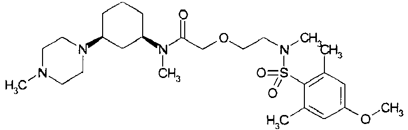 |
| (2) | 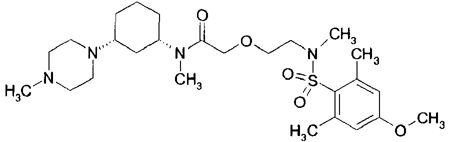 |
| (3) | 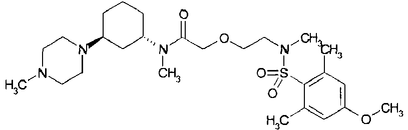 |
| (4) | 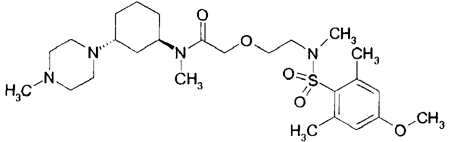 |
| (5) | 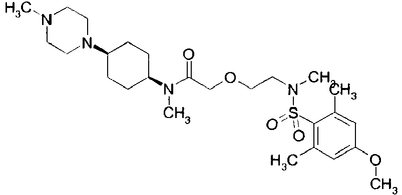 |
| (6) | 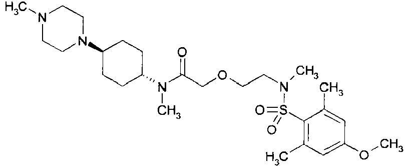 |
| (7) | 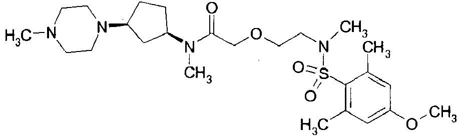 |
| (8) | 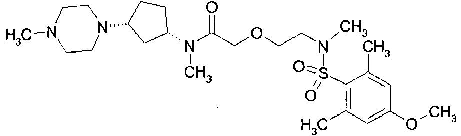 |
| (9) | 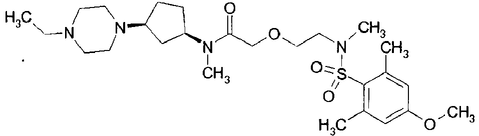 |
| (10) | 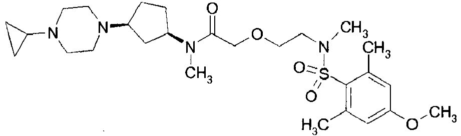 |
| (11) | 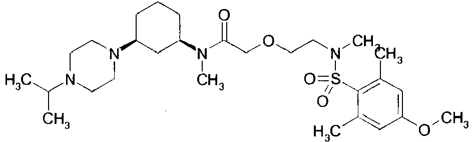 |
| (12) | 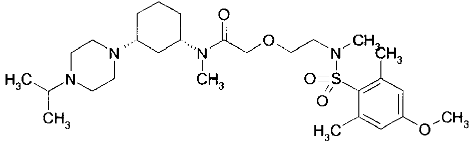 |
| (13) | 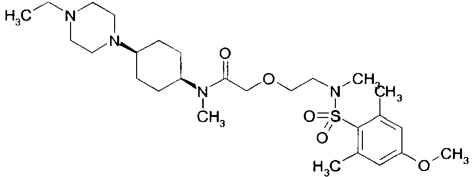 |
| (14) | 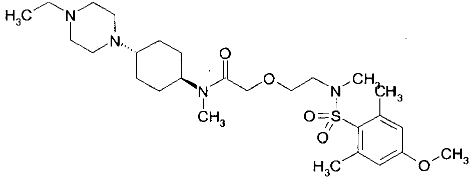 |
| (15) | 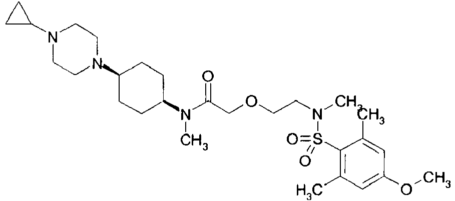 |
| (16) | 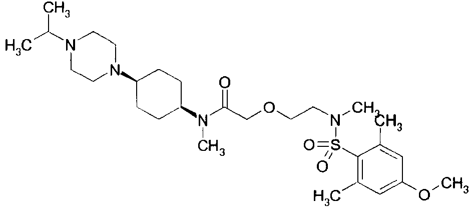 |
| (17) | 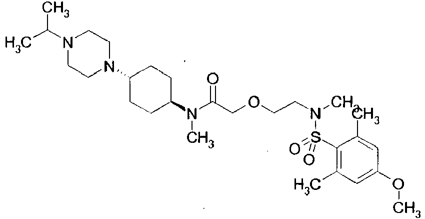 |
| (18) | 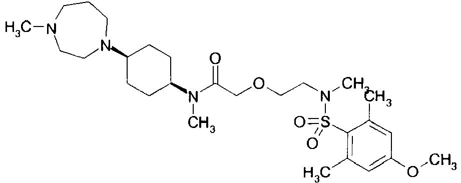 |
| (19) | 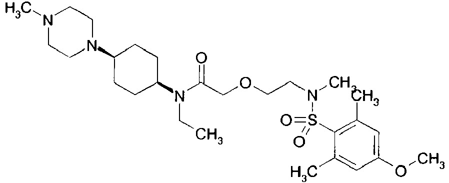 |
| (20) | 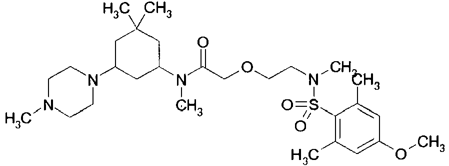 |
| (21) | 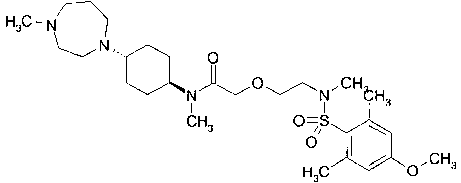 |
| (22) | 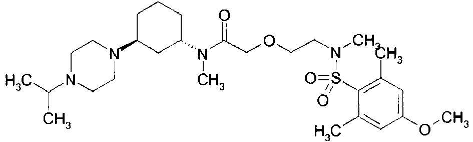 |
| (23) | 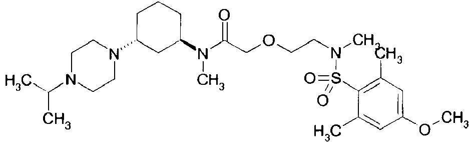 |
| (24) | 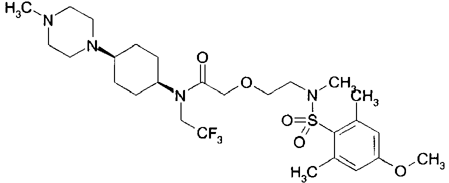 |
| (25) | 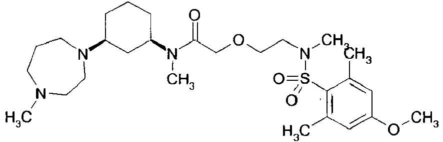 |
| (26) | 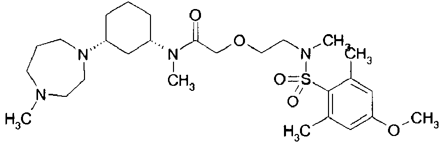 |
| (27) | 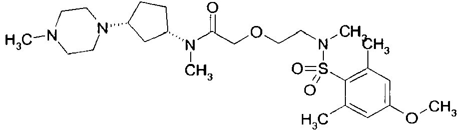 |
| (28) | 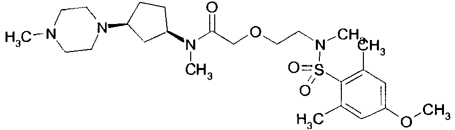 |
| (29) | 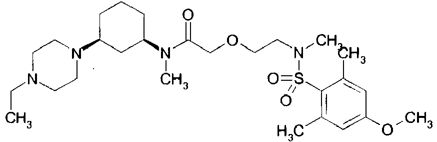 |
| (30) | 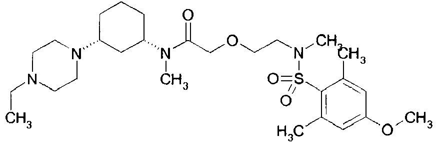 |
| (31) | 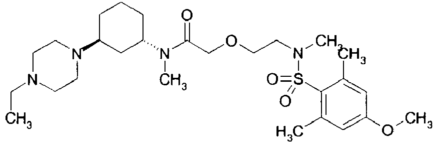 |
| (32) | 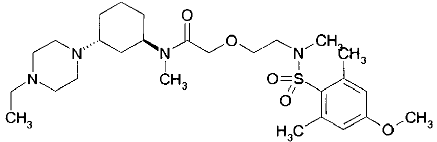 |
| (33) | 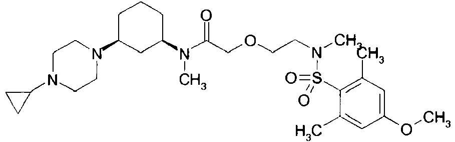 |
| (34) | 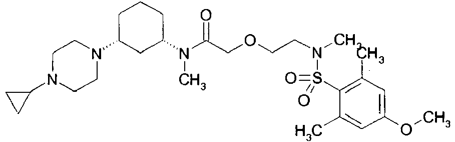 |
| (35) | 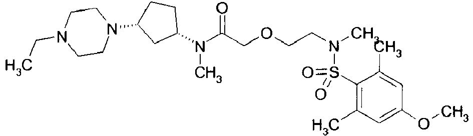 |
| (36) | 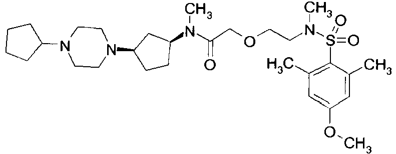 |
| (37) | 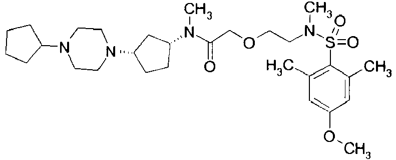 |
| (38) | 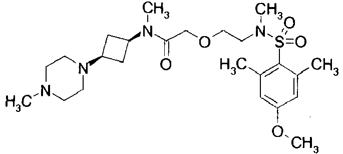 |
| (39) | 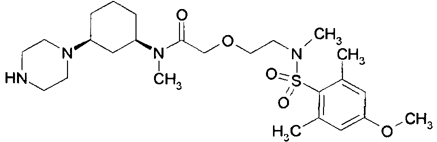 |
| (40) | 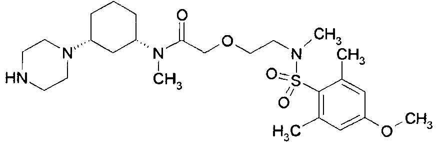 |
| (41) | 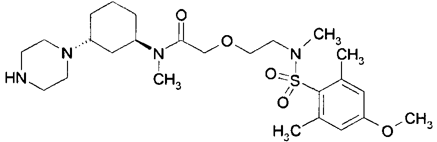 |
| (42) | 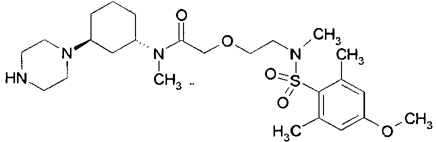 |
| (43) | 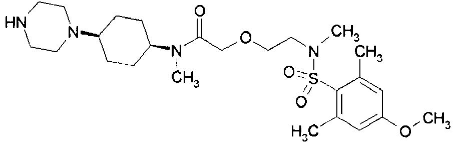 |

deren Enantiomere, deren Diastereomere, deren Gemische und deren Salze, insbesondere deren physiologisch verträgliche Salze mit organischen oder anorganischen Säuren oder Basen.

Als ganz besonders bevorzugte Verbindungen der obigen allgemeinen Formel **I** seien beispielsweise folgende genannt:

| **Nr.** | **Struktur** |
|---|---|
| (1) | 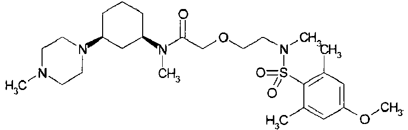 |
| (2) | 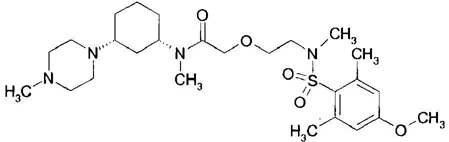 |
| (3) | 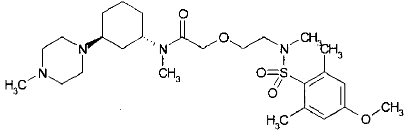 |
| (4) | 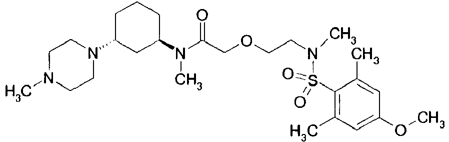 |
| (5) | 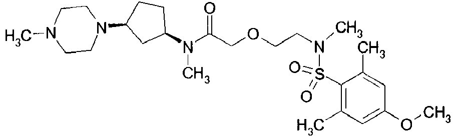 |
| (6) | 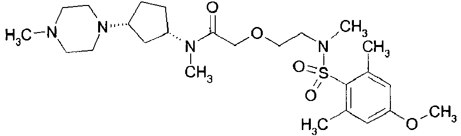 |
| (7) | 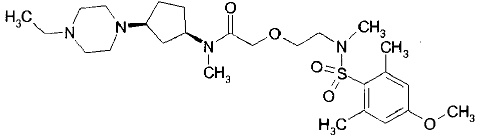 |
| (8) | 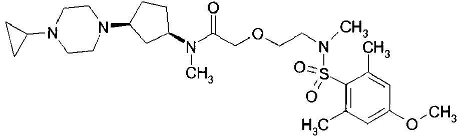 |
| (9) | 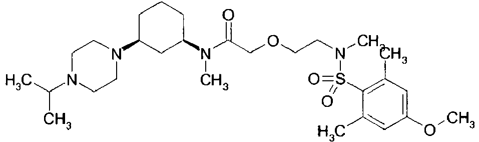 |
| (10) | 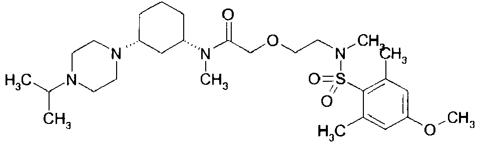 |
| (11) | 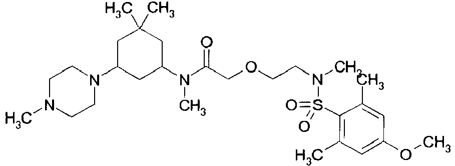 |
| (12) | 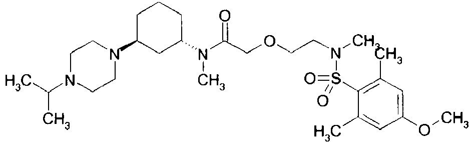 |
| (13) | 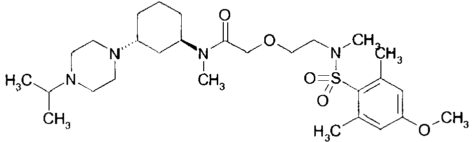 |
| (14) | 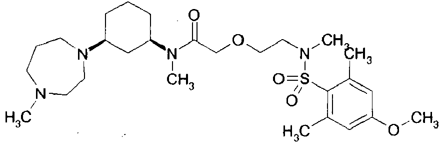 |
| (15) | 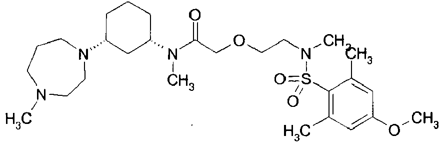 |
| (16) | 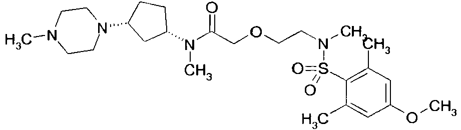 |
| (17) | 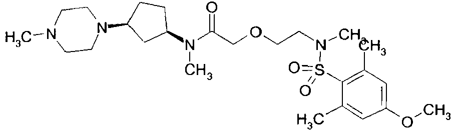 |
| (18) | 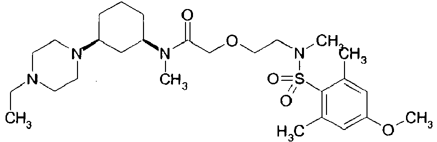 |
| (19) | 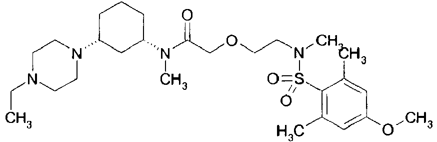 |
| (20) | 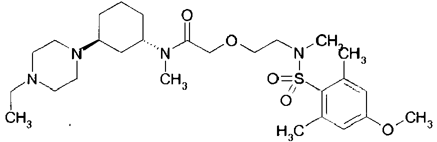 |
| (21) | 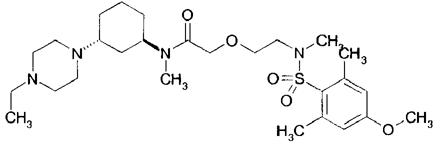 |
| (22) | 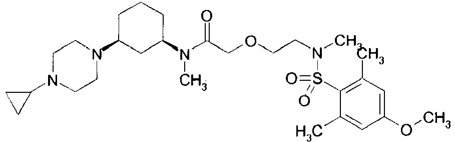 |
| (23) | 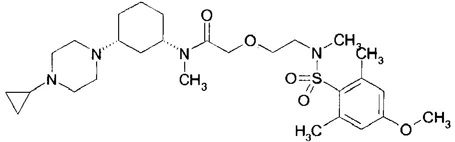 |
| (24) | 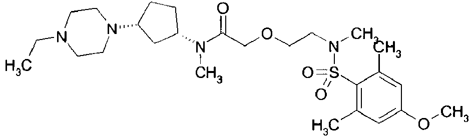 |
| (25) | 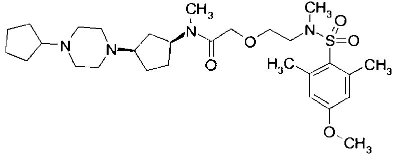 |
| (26) | 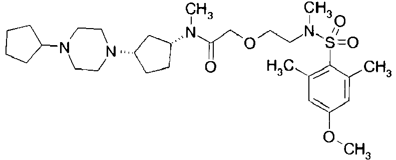 |
| (27) | 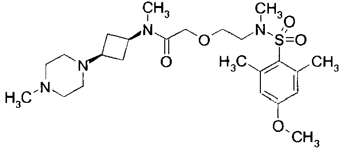 |
| (28) | 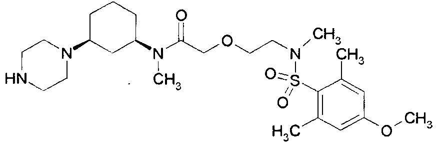 |
| (29) | 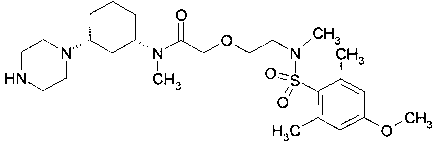 |
| (30) | 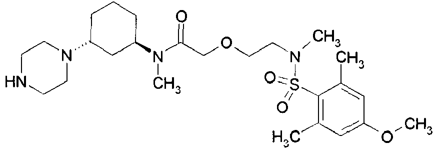 |
| (31) | 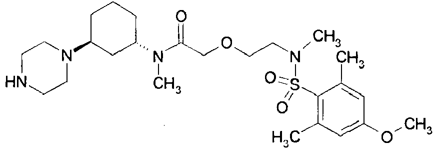 |

deren Enantiomere, deren Diastereomere, deren Gemische und deren Salze, insbesondere deren physiologisch verträgliche Salze mit organischen oder anorganischen Säuren oder Basen.

Ein weiterer Gegenstand der vorliegenden Erfindung betrifft die Verbindungen der allgemeinen Formel **Ia** 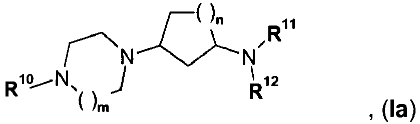 in der
- **m**: eine der Ziffern 1 oder 2,
- **n**: eine der Ziffern 0, 1 oder 2,
- **R¹⁰**: H, C₁₋₄-Alkyl, C₃₋₆-Cycloalkyl, C₁₋₄-Alkyl-O-C(O)-, Benzyl-O-C(O)- oder Benzyl, und
- **R¹¹, R¹²**: unabhängig voneinander
(a) H,
(b) C₁₋₄-Alkyl, C₃₋₆-Cycloalkyl,
(c) Benzyl,
(d) C₁₋₄-Alkyl-O-C(O)- oder Benzyl-O-C(O)- bedeuten,
deren Enantiomere, deren Diastereomere und deren Salze, vorzugsweise deren Hydrochloride, welche in besonderer Weise zur Herstellung von Verbindungen der allgemeinen Formel **I** geeignet sind.

Die Zwischenverbindungen der allgemeinen Formel **Ia** können analog den in der internationalen Patentanmeldung Nr. PCT/EP2007/058408 beschriebenen Verfahren hergestellt und gegebenenfalls nach bekannten Methoden in die Diastereomere oder Enantiomere getrennt werden.

Ein weiter bevorzugter Gegenstand der vorliegenden Erfindung betrifft die Verbindungen der allgemeinen Formel **Ia,** in der
- **m**: eine der Ziffern 1 oder 2,
- **n**: eine der Ziffern 0, 1 oder 2,
- **R¹⁰**: H, C₁₋₄-Alkyl, C₃₋₆-Cycloalkyl, C₁₋₄-Alkyl-O-C(O)-, Benzyl-O-C(O)- oder Benzyl, und
- **R¹¹**: (a) H,
(b) Benzyl,
(c) C₁₋₄-Alkyl-O-C(O)- oder Benzyl-O-C(O)-, und
- **R¹²**: (a) H,
(b) C₁₋₄-Alkyl, C₃₋₆-Cycloalkyl,
(c) Benzyl,
(d) C₁₋₄-Alkyl-O-C(O)- oder Benzyl-O-C(O)- bedeuten,
deren Enantiomere, deren Diastereomere und deren Salze, vorzugsweise deren Hydrochloride.

Die Verbindungen der allgemeinen Formel **Ia** stellen wertvolle Ausgangsstoffe zur Synthese der Verbindungen der allgemeinen Formel **I** dar, welche B1-antagonistische Eigenschaften besitzen.

Als weiter bevorzugte Verbindungen der allgemeinen Formel **Ia** seien beispielsweise folgende genannt:

| **Nr.** | **Struktur** |
|---|---|
| (1) | 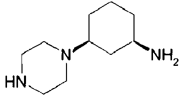 |
| (2) | 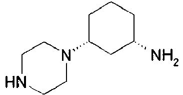 |
| (3) | 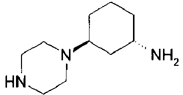 |
| (4) | 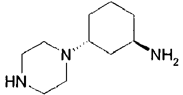 |
| (5) | 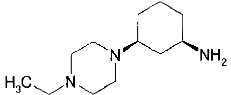 |
| (6) | 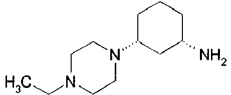 |
| (7) | 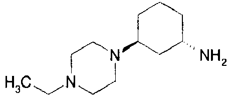 |
| (8) | 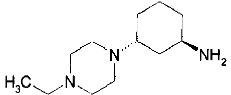 |
| (9) | 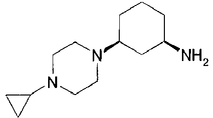 |
| (10) | 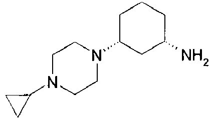 |
| (11) | 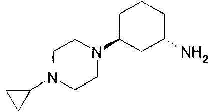 |
| (12) | 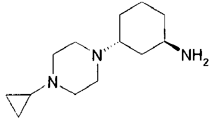 |
| (13) | 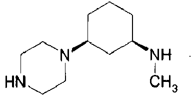 |
| (14) | 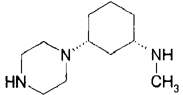 |
| (15) | 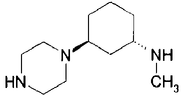 |
| (16) | 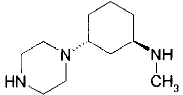 |
| (17) | 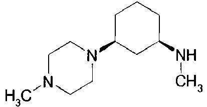 |
| (18) | 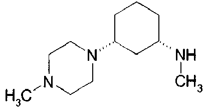 |
| (19) | 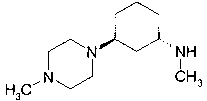 |
| (20) | 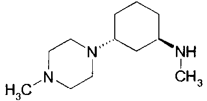 |
| (21) | 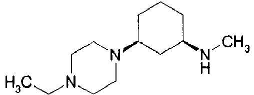 |
| (22) | 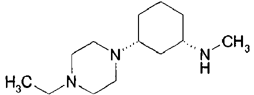 |
| (23) | 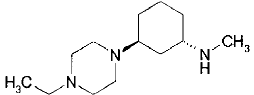 |
| (24) | 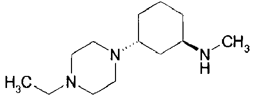 |
| (25) | 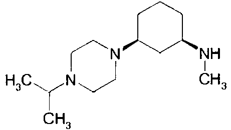 |
| (26) | 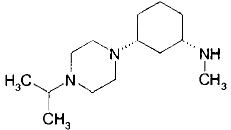 |
| (27) | 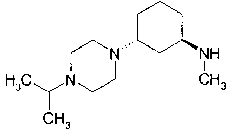 |
| (28) | 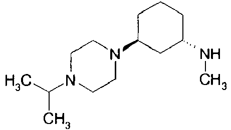 |
| (29) | 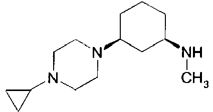 |
| (30) | 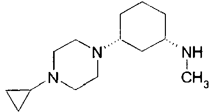 |
| (31) | 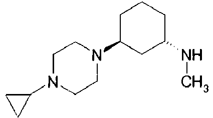 |
| (32) | 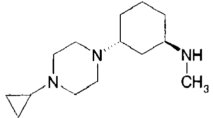 |
| (33) | 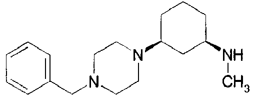 |
| (34) | 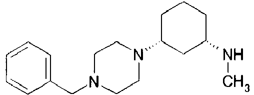 |
| (35) | 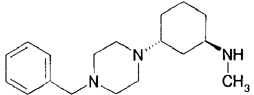 |
| (36) | 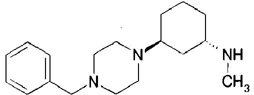 |
| (37) | 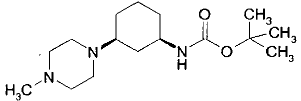 |
| (38) | 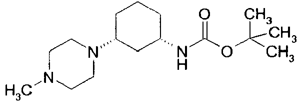 |
| (39) | 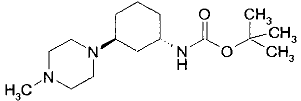 |
| (40) | 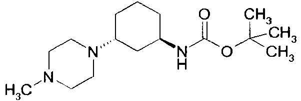 |
| (41) | 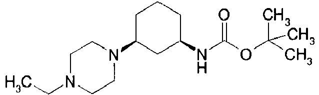 |
| (42) | 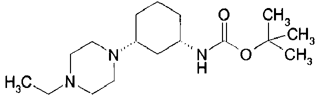 |
| (43) | 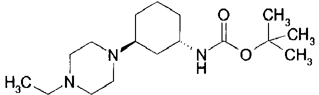 |
| (44) | 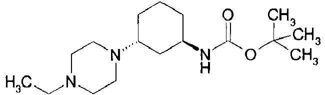 |
| (45) | 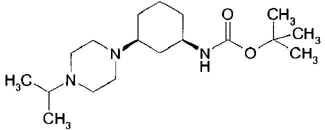 |
| (46) | 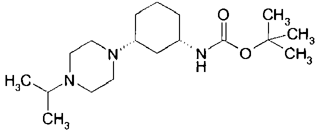 |
| (47) | 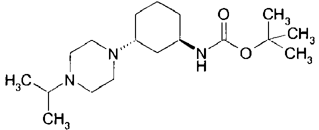 |
| (48) | 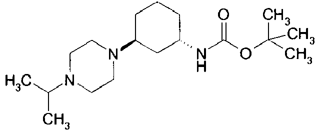 |
| (49) | 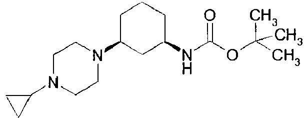 |
| (50) | 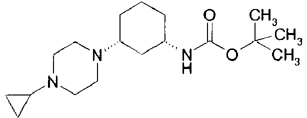 |
| (51) | 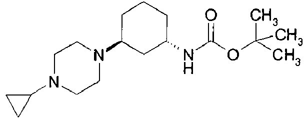 |
| (52) | 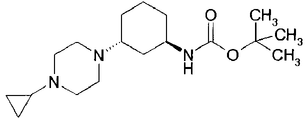 |
| (53) | 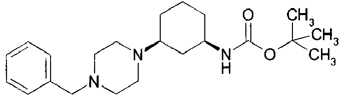 |
| (54) | 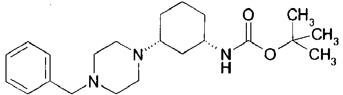 |
| (55) | 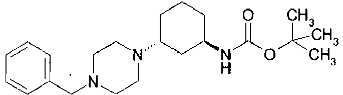 |
| (56) | 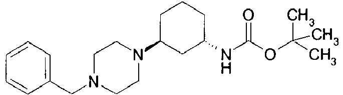 |
| (57) | 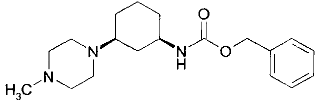 |
| (58) | 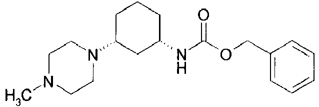 |
| (59) | 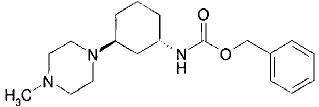 |
| (60) | 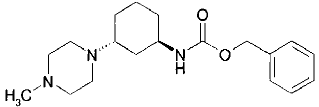 |
| (61) | 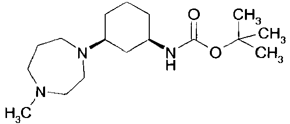 |
| (62) | 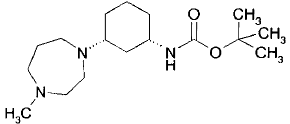 |
| (63) | 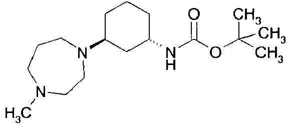 |
| (64) | 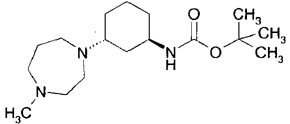 |
| (65) | 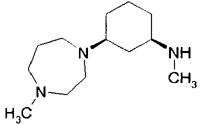 |
| (66) | 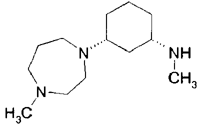 |
| (67) | 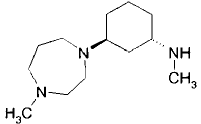 |
| (68) | 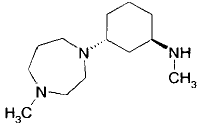 |
| (69) | 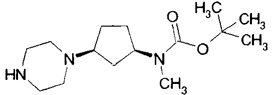 |
| (70) | 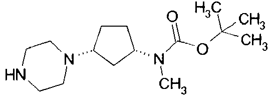 |
| (71) | 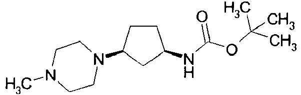 |
| (72) | 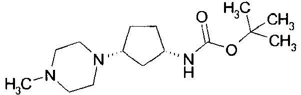 |
| (73) | 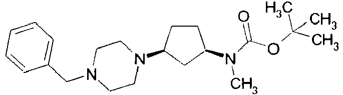 |
| (74) | 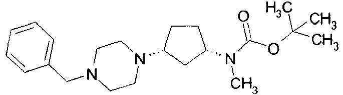 |
| (75) | 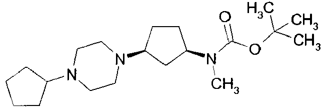 |
| (76) | 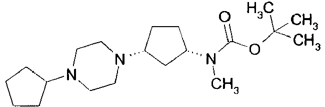 |
| (77) | 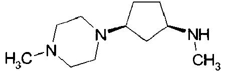 |
| (78) | 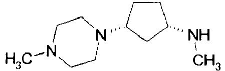 |
| (79) | 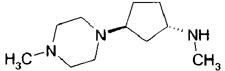 |
| (80) | 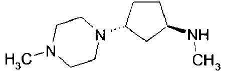 |
| (81) | 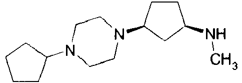 |
| (82) | 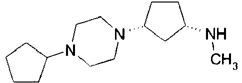 |
| (83) | 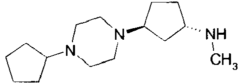 |
| (84) | 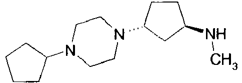 |
| (85) | 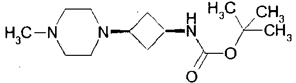 |
| (86) | 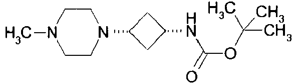 |
| (87) | 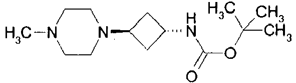 |
| (88) | 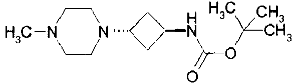 |
| (89) | 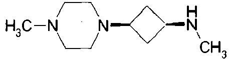 |
| (90) | 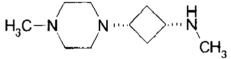 |
| (91) | 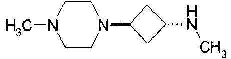 |
| (92) | 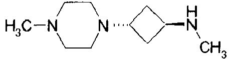 |

deren Enantiomere, deren Diastereomere.

Ein weiterer Gegenstand der vorliegenden Erfindung betrifft die Verwendung der voranstehend genannten Verbindungen der allgemeinen Formel **Ia,** in der m, n, **R¹** und **R²** wie voranstehend erwähnt definiert sind, als Zwischenprodukte zur Herstellung von Verbindungen der allgemeinen Formel **I**, in der **A, B, D, Y, R¹, R², R³**, **R⁴** und **R⁵** wie voranstehend erwähnt definiert sind.

### VERWENDETE BEGRIFFE UND DEFINITIONEN

Soweit nicht anders angegeben, sind alle Substituenten voneinander unabhängig. Sollten an einer Gruppe z.B. mehrere C₁₋₆-Alkylgruppen als Substituenten sein, so könnte im Fall von drei Substituenten C₁₋₆-Alkyl unabhängig voneinander einmal Methyl, einmal n-Propyl und einmal *tert*-Butyl bedeuten.

Im Rahmen dieser Anmeldung können bei der Definition von möglichen Substituenten, diese auch in Form einer Strukturformel dargestellt werden. Dabei wird, falls vorhanden, ein Stern (*) in der Strukturformel des Substituenten als der Verknüpfungspunkt zum Rest des Moleküls verstanden.

Ebenfalls mit vom Gegenstand dieser Erfindung umfasst sind die erfindungsgemäßen Verbindungen, einschließlich deren Salze, in denen ein oder mehrere Wasserstoffatome, beispielsweise ein, zwei, drei, vier oder fünf Wasserstoffatome, durch Deuterium ausgetauscht sind.

Unter dem Begriff "C₁₋₃-Alkyl" (auch soweit sie Bestandteil anderer Reste sind) werden Alkylgruppen mit 1 bis 3 Kohlenstoffatomen und unter dem Begriff "C₁₋₄-Alkyl" werden Alkylgruppen mit 1 bis 4 Kohlenstoffatomen verstanden. Beispielsweise werden hierfür genannt: Methyl, Ethyl, n-Propyl, *iso*-Propyl, n-Butyl, *iso*-Butyl und *tert*-Butyl. Gegebenenfalls werden für die vorstehend genannten Gruppen auch die Abkürzungen Me, Et, n-Pr, *i*-Pr, n-Bu, *i*-Bu, *t*-Bu etc. verwendet. Sofern nicht anders beschrieben, umfaßt die Definition Propyl alle denkbaren isomeren Formen des Restes. So umfasst Propyl n-Propyl und *iso*-Propyl.

Weiterhin umfassen die voranstehend genannten Begriffe auch solche Reste, in denen jede Methylengruppe mit bis zu zwei und jede Methylgruppe mit bis zu drei Fluoratomen substituiert sein kann.

Unter dem Begriff "C₃₋₅-Cycloalkyl" (auch soweit sie Bestandteil anderer Reste sind) werden cyclische Alkylgruppen mit 3 bis 5 Kohlenstoffatomen und unter dem Begriff "C₃₋₆-Cycloalkyl" werden cyclische Alkylgruppen mit 3 bis 6 Kohlenstoffatomen verstanden. Beispielsweise werden hierfür genannt: Cyclopropyl, Cyclobutyl, Cyclopentyl oder Cyclohexyl. Soweit nicht anders beschrieben, können die cyclischen Alkylgruppen substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Unter dem Begriff "C₄₋₆-Cycloalkylen" (auch soweit sie Bestandteil anderer Reste sind) werden cyclische Alkylengruppen mit 4 bis 6 Kohlenstoffatomen verstanden. Beispielsweise werden hierfür genannt: Cyclobutylen, Cyclopentylen oder Cyclohexylen. Soweit nicht anders beschrieben, können die cyclischen Alkylengruppen substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Eine C₄- oder eine C₅-Cycloalkylengruppe kann in 1,2-Stellung oder in 1,3-Stellung mit dem restlichen Molekül verknpüft sein, vorzugsweise in 1,3-Stellung. Eine C₆-Cycloalkylengruppe kann in 1,2-Stellung, in 1,3- Stellung oder in 1,4-Stellung mit dem restlichen Molekül verknüpft sein, vorzugsweise in 1,3-Stellung.

Unter dem Begriff "gesättigte Diaza-Heterocyclen" werden sechs- oder siebengliedrige heterocyclische Ringe verstanden, die zwei Stickstoffatome enthalten. Dabei ist der Ring über beide Stickstoffatome mit dem restlichen Molekül verknüpft. Als Beispiele werden genannt:

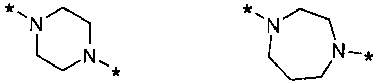

Verbindungen der allgemeinen Formel **I** können, sofern sie geeignete basische Funktionen enthalten, beispielsweise Aminogruppen, insbesondere für pharmazeutische Anwendungen in ihre physiologisch verträglichen Salze mit anorganischen oder organischen Säuren übergeführt werden. Als anorganische Säuren kommen hierfür beispielsweise Bromwasserstoffsäure, Phosphorsäure, Salpetersäure, Salzsäure, Schwefelsäure, Methansulfonsäure, Ethansulfonsäure, Benzolsulfonsäure oder *p*-Toluolsulfonsäure in Frage, als organische Säuren kommen beispielsweise Äpfelsäure, Bernsteinsäure, Essigsäure, Fumarsäure, Maleinsäure, Mandelsäure, Milchsäure, Weinsäure oder Zitronensäure in Betracht. Weiterhin können gegebenenfalls im Molekül vorhandene tertiäre Aminogruppen quarternisiert werden. Zur Umsetzung werden dabei Alkylhalogenide eingesetzt. Erfindungsgemäß bevorzugt wird für die Quartenisierung Methyliodid verwendet.

Weiterhin lassen sich die Verbindungen der allgemeinen Formel **I**, sofern sie geeignete Carbonsäurefunktionen enthalten, gewünschtenfalls in ihre Additionssalze mit anorganischen oder organischen Basen überführen. Als anorganische Basen kommen hierfür beispielsweise Alkali- oder Erdalkalimetallhydroxide, beispielsweise Natriumhydroxid oder Kaliumhydroxid, oder Carbonate, Ammoniak, Zink- oder Ammoniumhydroxide in Frage; als organische Amine kommen beispielsweise Diethylamin, Triethylamin, Ethanolamin, Diethanolamin, Triethanolamin, Cyclohexylamin oder Dicyclohexylamin in Betracht.

Die erfindungsgemäßen Verbindungen können als Racemate vorliegen, sofern sie nur ein Chiralitätselement besitzen, sie können aber auch als reine Enantiomere, d.h. in (R)- oder (S)-Form gewonnen werden.

Die Anmeldung umfasst jedoch auch die einzelnen diastereomeren Antipodenpaare oder deren Gemische, die dann vorliegen, wenn mehr als ein Chiralitätselement in den Verbindungen der allgemeinen Formel **I** vorhanden ist, sowie die einzelnen optisch aktiven Enatiomeren, aus denen sich die erwähnten Racemate zusammensetzen.

Gegenstand der Erfindung sind die jeweiligen Verbindungen gegebenenfalls in Form der einzelnen optischen Isomeren, Mischungen der einzelnen Enantiomeren oder Racemate, in Form der Tautomere sowie in Form der freien Basen oder der entsprechenden Säureadditionssalze mit pharmakologisch unbedenklichen Säuren - wie beispielsweise Säureadditionssalze mit Halogenwasserstoffsäuren - beispielsweise Chlor- oder Bromwasserstoffsäure - oder organische Säuren - wie beispielsweise Oxal-, Fumar-, Diglycol- oder Methansulfonsäure.

### HERSTELLVERFAHREN

Erfindungsgemäß erhält man die Verbindungen der allgemeinen Formel **I** nach an sich bekannten Verfahren, beispielsweise nach folgenden Verfahren:

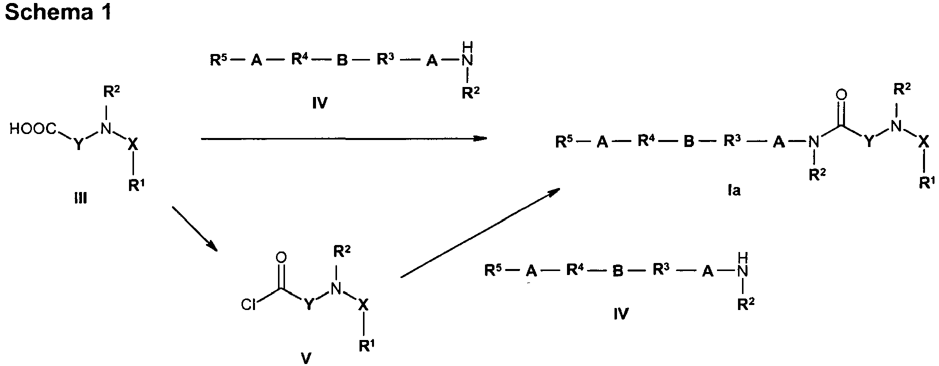

Die in Schema 1 dargestellte Verknüpfung von Carbonsäuren der allgemeinen Formel **III**, in der alle Reste wie voranstehend erwähnt definiert sind, mit Aminen der allgemeinen Formel **IV,** in der alle Reste wie voranstehend erwähnt definiert sind, unter Bildung von Carbonsäureamiden der allgemeinen Formel **Ia,** in der alle Reste wie voranstehend erwähnt definiert sind, kann mit herkömmlichen Methoden zur Amidbildung durchgeführt werden.

Die Kupplung wird bevorzugt unter Verwendung von aus der Peptidchemie bekannten Verfahren (siehe z. B. Houben-Weyl, Methoden der Organischen Chemie, Bd. 15/2) durchgeführt, wobei zum Beispiel Carbodiimide, wie z.B. Dicyclohexylcarbodümid (DCC), Diisopropylcarbodiimid (DIC) oder Ethyl-(3-dimethylamino-propyl)-carbodiimid, *O*-(1*H*-Benzotriazol-1-yl)-*N,N*-*N*',*N*'-tetramethyluronium-hexafluorphosphat (HBTU) oder -tetrafluorborat (TBTU) oder 1*H*-Benzotriazol-1-yl-oxy-tris-(dimethylamino)-phosphonium-hexafluorphosphat (BOP) eingesetzt werden. Durch Zugabe von 1-Hydroxybenzotriazol (HOBt) oder von 3-Hydroxy-4-oxo-3,4-di-hydro-1,2,3-benzotriazin (HOObt) kann die Reaktionsgeschwindigkeit gesteigert werden. Die Kupplungen werden normalerweise mit äquimolaren Anteilen der Kupplungskomponenten sowie des Kupplungsreagenz in Lösemitteln wie Dichlormethan, Tetrahydrofuran (THF), Acetonitril, Dimethylformamid (DMF), Dimethylacetamid (DMA), N-Methylpyrrolidon (NMP) oder Gemischen aus diesen und bei Temperaturen zwischen -30°C und +30°C, bevorzugt -20°C und +25°C, durchgeführt. Sofern erforderlich, wird als zusätzliche Hilfsbase Diisopropylethylamin (DIPEA) (Hünig-Base) bevorzugt.

Eine alternative Methode zur Verknüpfung besteht in der Überführung einer Carbonsäure der allgemeinen Formel **III,** in der alle Reste wie voranstehend erwähnt definiert sind, in ein Carbonsäurechlorid der allgemeinen Formel V, in der alle Reste wie voranstehend erwähnt definiert sind, und anschließender Umsetzung mit einem Amin der allgemeinen Formel **IV,** in der alle Reste wie voranstehend erwähnt definiert sind. Die Synthese eines Carbonsäurechlorids der allgemeinen Formel V erfolgt nach literaturbekannten Verfahren (siehe z. B. Houben-Weyl, Methoden der Organischen Chemie, Bd. E5/1).

Die als Ausgangsstoffe verwendeten Carbonsäuren der allgemeinen Formel **III,** in der alle Reste wie voranstehend erwähnt definiert sind, erhält man nach an sich literaturbekannten Verfahren, beispielsweise durch die in Schema 2 bis 7 dargestellten Synthesewege.

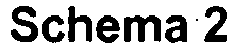

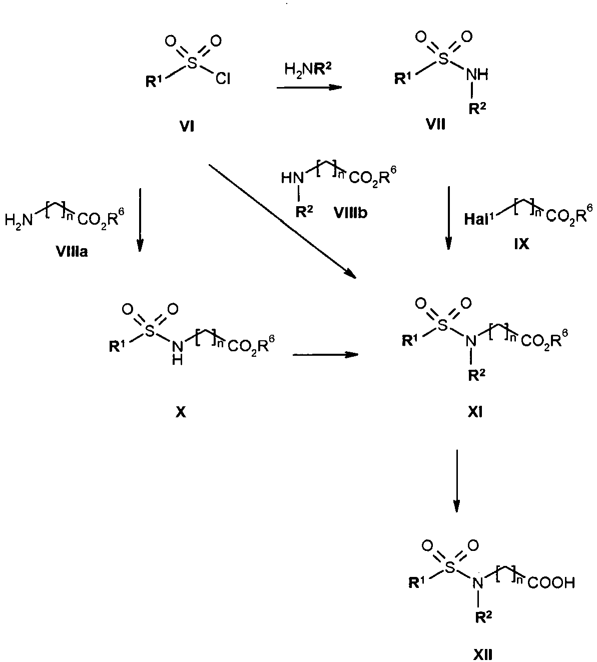

Die Sulfonsäurechloride der allgemeinen Formel **VI,** in der **R¹** wie voranstehend erwähnt definiert ist, sind entweder literaturbekannt oder käuflich erwerbbar. Sie werden nach Standard-Reaktionsbedingungen mit einem Amin der allgemeinen Formeln H₂N-**R²**, **VIIIa** oder **VIIIb** zu Sulfonsäureamiden der allgemeinen Formeln **VII, X** oder **XI** umgesetzt, wobei **R¹** und **R²** wie voranstehend erwähnt definiert sind und n eine Zahl 1, 2, 3 oder 4 und **R⁶** einen C₁₋₆-Alkylrest bedeuten. Die Umsetzung erfolgt gegebenenfalls in Gegenwart einer Base wie Triethylamin, DIPEA oder Pyridin und einem inerten Lösungsmittel wie Dichlormethan oder Tetrahydrofuran bei einer Temperatur von 0°C bis 100°C mit einer typischen Reaktionsdauer von einer bis 24 Stunden.

Die Umsetzung der Sulfonsäureamide der allgemeinen Formel **VII** mit einem Halogenid der allgemeinen Formel **IX,** in der **Hal¹** Chlor oder Brom bedeutet, erfolgt nach literaturbekannten Verfahren, beispielsweise mit Hilfe einer Base wie Kalium- oder Natriumcarbonat in Dimethylformamid oder Tetrahydrofuran bei 0°C bis 100°C.

Die Hydrolyse der Carbonsäurester der allgemeinen Formel **XI,** in der **R¹** und **R²** wie voranstehend erwähnt definiert sind, n eine Zahl 1, 2, 3 oder 4 und **R⁶** eine C₁₋₃-Alkylgruppe bedeuten, zu Carbonsäuren der allgemeinen Formel **XII,** in der **R¹** und **R²** wie voranstehend erwähnt definiert sind und n eine Zahl 1, 2, 3 oder 4 und **R⁶** eine C₁₋₃-Alkylgruppe bedeutet, wird unter bekannten Bedingungen durchgeführt, beispielsweise mit Lithium- oder Natriumcarbonat und Wasser in Methanol und/oder Tetrahydrofuran.

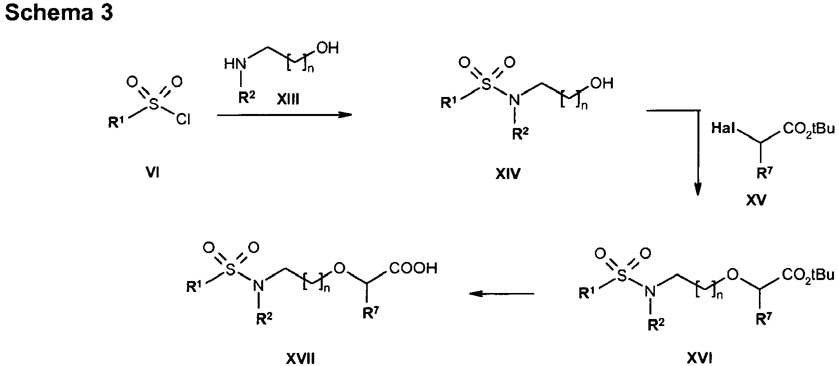

Die Herstellung von Sulfonsäureamiden der allgemeinen Formel **XIV** erfolgt wie unter Schema 2 beschrieben.

Die Alkylierung der Hydroxylfunktion der Sulfonsäureamide der allgemeinen Formel **XIV,** in der **R¹** und **R²** wie voranstehend erwähnt definiert sind mit der Maßgabe, dass **R²** kein Wasserstoffatom darstellt, und n eine Zahl 1, 2, 3 oder 4 und **R⁶** eine C₁₋₃-Alkylgruppe bedeutet, wird unter literaturbekannten Reaktionsbedingungen durchgeführt, beispielsweise unter 2-Phasen-Bedingungen mit Hilfe eines Phasentransfer-Katalysators in Gegenwart einer starken anorganischen Base wie Natronlauge oder Kalilauge und in einem inerten Lösungsmittel wie Toluol bei 0°C bis 100°C.

Die Spaltung des tert-Butylesters der allgemeinen Formel **XVI,** in der **R¹** und **R²** wie voranstehend erwähnt definiert sind, n eine Zahl 1, 2, 3 oder 4 und **R⁶** eine C₁₋₃-Alkylgruppe und **R¹** ein Wasserstoffatom oder eine C₁₋₃-Alkylgruppe bedeuten, erfolgt nach literaturbekannten Verfahren (siehe z.B. Philip J. Kocieński, Protecting Groups, 3rd Edition, 2005, Georg Thieme Verlag).

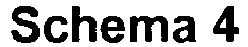

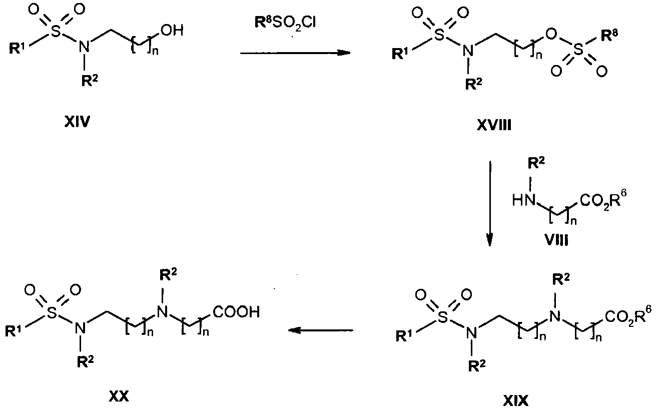

Die Sulfonierung der Hydroxylfunktion einer Verbindung der allgemeinen Formel **XIV,** in der **R¹** und **R²** wie voranstehend erwähnt definiert sind mit der Maßgabe, dass **R²** kein Wasserstoffatom darstellt, und n eine Zahl 1, 2, 3 oder 4 und **R⁶** eine C₁₋₃-Alkylgruppe bedeutet, mit einem Sulfonsäurechlorid der allgemeinen Formel **R⁸**SO₂Cl, in der **R⁸** eine C₁₋₃-Alkylgruppe oder eine ggf. durch C₁₋₃-Alkylgruppe substituierte Phenylgruppe darstellt, zu Verbindungen der allgemeinen Formel **XVIII,** in der alle Rest wie voranstehend erwähnt definiert sind, wird unter Standard-Reaktionsbedingungen durchgeführt, typischerweise in Gegenwart einer Base wie DMAP und/oder Pyridin und einem inerten Lösungsmittel wie Dichlormethan oder THF bei -5°C bis 35°C. Eine flüssige Base wie Pyridin kann als Base und gleichzeitig als Lösungsmittel verwendet werden.

Die nachfolgende Alkylierung der Amine mit der allgemeinen Formel **VII** zu Verbindungen der allgemeinen Formel **XIX,** in der **R¹** und **R²** wie voranstehend erwähnt definiert sind, n eine Zahl 1, 2, 3 oder 4 und **R⁶** eine C₁₋₃-Alkylgruppe und **R⁶** eine C₁₋₆-Alkylgruppe bedeuten, wird zweckmäßigerweise in einem Lösungsmittel wie Toluol, Chlorbenzol, Dimethylformamid, Dimethylsulfoxid (DMSO), Dichlormethan, Acetonitril oder Pyridin, beispielsweise bei Temperaturen zwischen 0°C und 150°C und zweckmäßigerweise in Gegenwart von Basen wie Pyridin, Triethylamin, DIPEA, Kaliumcarbonat, Kalium-tert-butylat oder Natriummethanolat durchgeführt, wobei das Alkylsulfonat als Abgangsgruppe dient.

Die Hydrolyse der Carbonsäureester der allgemeinen Formel **XIX** zu Carbonsäuren der allgemeinen Formel **XX** erfolgt wie unter Schema 2 beschrieben.

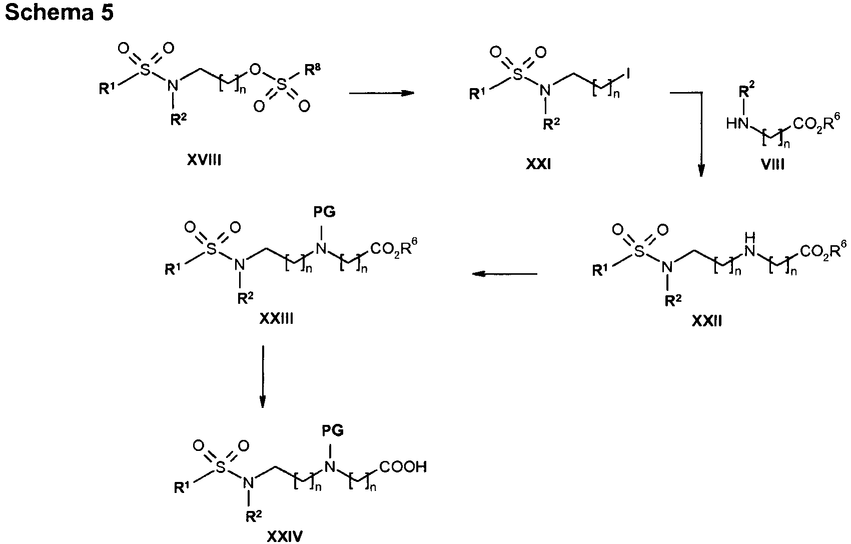

Die Finkelsteinstein-Reaktion von Verbindungen der allgemeinen Formel **XVIII,** in der **R¹** und **R²** wie eingangs erwähnt definiert sind, n eine Zahl 1, 2, 3 oder 4 und **R⁶** eine C₁₋₃-Alkylgruppe und **R⁸** eine C₁₋₃-Alkylgruppe oder eine ggf. durch C₁₋₃-Alkylgruppe substituierte Phenylgruppe bedeuten, zu Halogeniden der allgemeinen Formel **XXI,** in der **R¹** und **R²** wie eingangs erwähnt definiert sind und n eine Zahl 1, 2, 3 oder 4 und **R⁶** eine C₁₋₃-Alkylgruppe bedeutet, erfolgt unter bekannten Reaktionsbedingungen (siehe z.B. H. Finkelstein, Berichte der Deutschen Chemischen Gesellschaft 43, 1910, 1528).

Die anschließende Alkylierung des Glycinesters wird wie unter Schema 4 beschrieben durchgeführt (**R²** ≠ H).

Die Aminofunktion in den Verbindungen der allgemeinen Formel **XXIII** wird durch eine konventionelle Schutzgruppe **PG** nach bekannten Verfahren geschützt. Die ausgewählte Schutzgruppe ist eine, die unter nicht-hydrogenolytischen Bedingungen abgespalten werden kann. Eine bevorzugte Schutzgruppe ist die Boc-Gruppe. Eine Übersicht über die Chemie der Schutzgruppen findet sich in Theodora W. Greene und Peter G. M. Wuts, Protective Groups in Organic Synthesis, Second Edition, 1991, Verlag John Wiley and Sons sowie in Philip J. Kocieński, Protecting Groups, 3rd Edition, 2005, Georg Thieme Verlag.

Die Spaltung der Carbonsäureester der allgemeinen Formel **XXIII** zu Carbonsäuren der allgemeinen Formel **XXIV** erfolgt wie unter Schema 2 beschrieben.

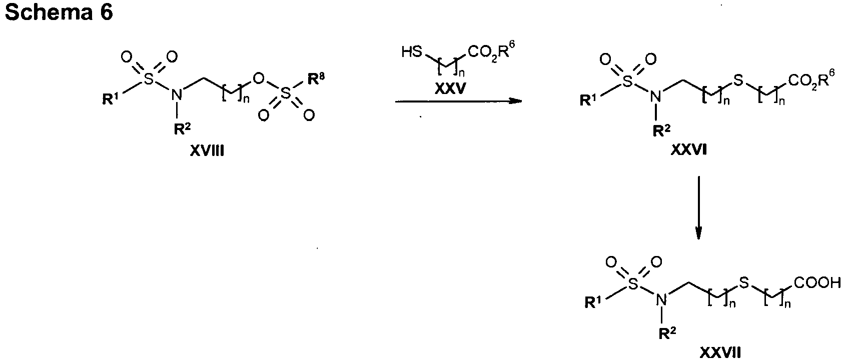

Die Alkylierung eines Thiols der allgemeinen Formel **XXV,** in der n eine Zahl 1, 2, 3 oder 4 und **R⁶** eine C₁₋₆-Alkylgruppe bedeutet, zu Verbindungen der allgemeinen Formel **XXVI,** in der **R¹** und **R²** wie voranstehend erwähnt definiert sind, n eine Zahl 1, 2, 3 oder 4 und **R⁶** eine C₁₋₆-Alkylgruppe bedeuten, wird zweckmäßigerweise in einem Lösungsmittel wie Toluol, Chlorbenzol, DMF, DMSO, Dichlormethan, Acetonitril oder Pyridin, beispielsweise bei Temperaturen zwischen 0°C und 150°C und zweckmäßigerweise in Gegenwart von Basen wie Pyridin, Triethylamin, DIPEA, Kaliumcarbonat, Kalium-tert-butylat oder Natriummethanolat durchgeführt, wobei das Alkylsulfonat als Abgangsgruppe dient.

Die Hydrolyse der Carbonsäureester der allgemeinen Formel **XXVI** zu Carbonsäuren der allgemeinen Formel **XXVII,** in der alle Reste wie voranstehend erwähnt definiert sind, erfolgt wie unter Schema 2 beschrieben.

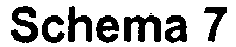

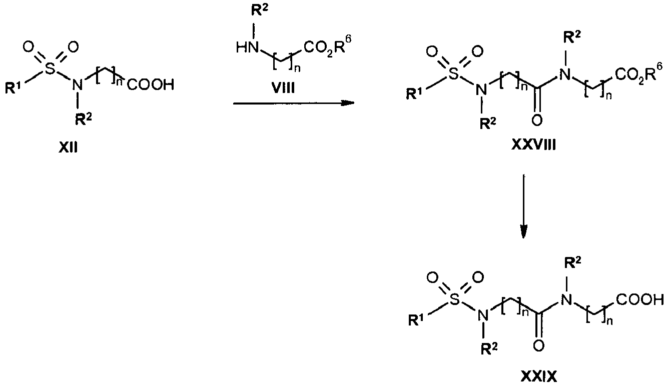

Die Amidknüpfung von Carbonsäuren der allgemeinen Formel **XII,** in der **R¹** und **R²** wie voranstehend erwähnt definiert sind und n eine Zahl 1, 2, 3 oder 4 bedeutet, und Aminosäuren der allgemeinen Formel **VIII,** in der **R¹** und **R²** wie voranstehend erwähnt definiert sind, n eine Zahl 1, 2, 3 oder 4 und **R⁶** eine C₁₋₆-Alkylgruppe bedeuten, zu Carbonsäureamiden der allgemeinen Formel **XXVIII,** in der **R¹** und **R²** wie voranstehend erwähnt definiert sind, n eine Zahl 1, 2, 3 oder 4 und **R⁶** eine C₁₋₆-Alkylgruppe bedeuten, wird wie unter Schema 1 beschrieben durchgeführt.

Wie unter Schema 2 ausgeführt, wird der Carbonsäureester der allgemeinen Formel **XXVIII** zu Carbonsäure der allgemeinen Formel **XXIX,** in der **R¹** und **R²** wie voranstehend erwähnt definiert sind und n eine Zahl 1, 2, 3 oder 4 bedeutet, gespalten.

Die als Ausgangsstoffe verwendeten Amine der allgemeinen Formel **IV** sind entweder käuflich erwerbbar, oder man erhält sie nach an sich literaturbekannten Verfahren, beispielsweise durch die in Schema 8 bis 12 dargestellten Synthesewege, wobei **R^{1.1}** wie voranstehend erwähnt definiert ist, **Hal¹** ein Chlor- oder Bromatom und **Hal²** ein Fluor-, Chlor- oder Bromatom oder einen Rest **R⁹** bedeuten.

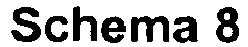

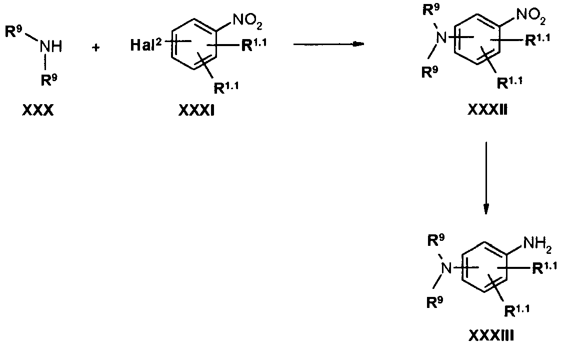

Die Reaktion eines Amins der allgemeinen Formel **XXX,** in der **R⁹** eine C₁₋₃-Alkylgruppe bedeutet, mit einem Halogen-nitrobenzol der allgemeinen Formel **XXXI,** in der **R^{1.1}** wie voranstehend erwähnt definiert ist und **Hal²** ein Fluor-, Chlor- oder Bromatom oder einen Rest **R⁹** bedeutet, erfolgt nach bekannten Verfahren, beispielsweise in einem Lösungsmittel wie Tetrahydrofuran, Dimethylformamid oder Dimethylsulfoxid und zweckmäßigerweise in Gegenwart einer passenden Base wie Triethylamin oder Kaliumcarbonat, bei einer Temperatur von 20°C bis 160°C. Ist das Amin der allgemeinen Formel **XXX** flüssig, kann die Reaktion auch ohne Lösungsmittel und zusätzlicher Base durchgeführt werden.

Die Reduktion der Nitrogruppe zu Anilinen der allgemeinen Formel **XXXIII,** in der **R^{1.1}** wie voranstehend erwähnt definiert ist und **R⁹** eine C₁₋₃-Alkylgruppe bedeutet, erfolgt nach Standard-Reaktionsbedingungen (siehe z.B. Richard C. Larock, Comprehensive Organic Transformations, 1989, VCH), vorzugsweise nach Standard-Bedingungen der katalytischen Hydrogenolyse mit einem Katalysator wie Palladium auf Kohle oder Raney-Nickel in einem Lösungsmittel wie Methanol oder Ethanol.

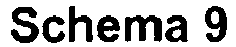

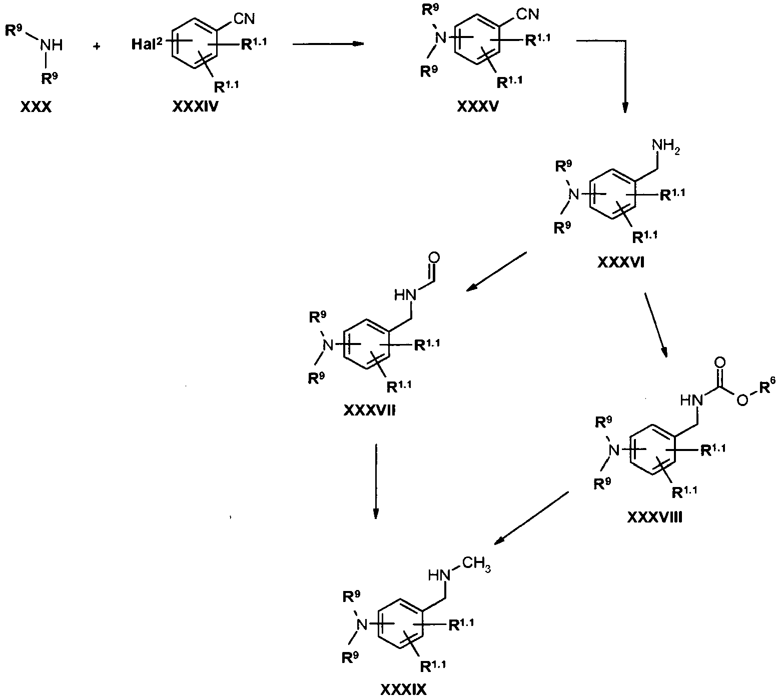

Die Umsetzung von Verbindungen der allgemeinen Formeln **XXX,** in der **R⁹** eine C₁₋₃-Alkylgruppe bedeutet, mit einer Verbindung der allgemeinen Formel **XXXIV,** in der **R^{1.1}** wie voranstehend erwähnt definiert ist und **Hal²** ein Fluor-, Chlor- oder Bromatom oder einen Rest **R⁹** bedeutet, zu Verbindungen mit der allgemeinen Formel **XXXV,** in der **R^{1.1}** wie voranstehend erwähnt definiert ist und **R⁹** eine C₁₋₃-Alkylgruppe bedeutet, wird wie unter Schema 8 beschrieben durchgeführt.

Die Reduktion eines Nitrils der allgemeinen Formel **XXXV** zu einem Amin der allgemeinen Formel **XXXVI,** in der **R^{1.1}** wie voranstehend erwähnt definiert ist und **R⁹** eine C₁₋₃-Alkylgruppe bedeutet, kann unter Standard-Bedingungen der katalytischen Hydrogenolyse mit einem Katalysator wie beispielsweise Raney-Nickel in einem Lösungsmittel wie ammoniakalischem Methanol oder Ethanol oder mit einem Reduktionsmittel wie Lithiumaluminiumhydrid oder Natriumborhydrid in einem Lösungmittel wie Tetrahydrofuran, gegebenenfalls in Gegenwart von Aluminiumchlorid, durchgeführt werden.

Die Formylierung eines Amins der allgemeinen Formel **XXXVI** zu einer Verbindung der allgemeinen Formel **XXXVII,** in der **R^{1.1}** wie voranstehend erwähnt definiert ist und **R⁹** eine C₁₋₃-Alkylgruppe bedeutet, erfolgt zweckmäßigerweise in einem Lösungsmittel wie Dichlormethan, beispielsweise bei Temperaturen von 40°C bis 70°C und in Gegenwart von Essigsäureanhydrid und Ameisensäure.

Die Carbamatbildung zu Verbindungen der allgemeinen Formel **XXXVIII,** in der **R^{1.1}** wie voranstehend erwähnt definiert ist, **R⁶** eine C₁₋₆-Alkyl- und **R⁹** eine C₁₋₃-Alkylgruppe bedeutet, wird nach bekannten Verfahren durchgeführt, beispielsweise mit einem Chlorameisensäureester oder Boc-Anhydrid in Gegenwart einer Base wie Triethylamin oder Natronlauge und einem Lösungsmittel wie THF oder Dioxan.

Die Reduktion des Formyls bzw. des Carbamates zu Verbindungen der allgemeinen Formel **XXXIX,** in der **R^{1.1}** wie voranstehend erwähnt definiert ist und **R⁹** eine C₁₋₃-Alkylgruppe bedeutet, erfolgt unter Standard-Reaktionsbedingungen, vorzugsweise mit einem Reduktionsmittel wie Lithiumaluminiumhydrid und in einem Lösungsmittel wie Tetrahydrofuran bei einer Temperatur von 50°C bis 100°C.

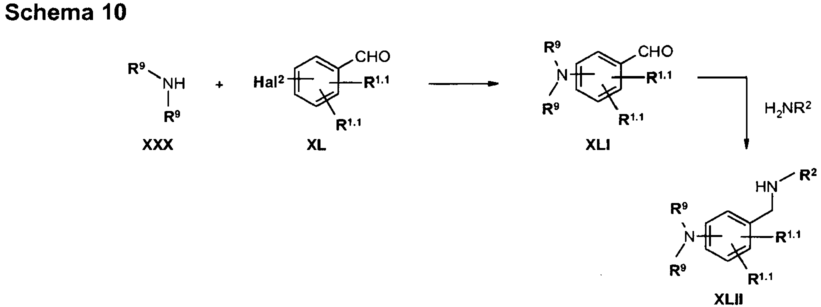

Der Halogen-Stickstoff-Austausch in Verbindungen der allgemeinen Formeln **XXX,** in der **R⁹** eine C₁₋₃-Alkylgruppe bedeutet, und **XL,** in der **R^{1.1}** wie voranstehend erwähnt definiert ist und **Hal²** ein Fluor-, Chlor- oder Bromatom oder einen Rest **R⁹** bedeutet, zur Herstellung von Verbindungen der allgemeinen Formel **XLI,** in der **R^{1.1}** wie voranstehend erwähnt definiert ist und **R⁹** eine C₁₋₃-Alkylgruppe bedeutet, wird wie unter Schema 8 beschrieben durchgeführt.

Die Reaktion von Benzaldehyden der allgemeinen Formel XLI, in der **R^{1.1}** wie voranstehend erwähnt definiert ist und **R⁹** eine C₁₋₃-Alkylgruppe bedeutet, mit einem Amin der allgemeinen Formel H₂N**R²**, in der **R²** wie voanstehend erwähnt definiert ist, zu einer Verbindung der allgemeinen Formel **XLII,** in der **R^{1.1}** und **R²** wie voranstehend erwähnt definiert sind und **R⁹** eine C₁₋₃-Alkylgruppe bedeutet, ist eine reduktive Aminierung. Sie erfolgt nach bekannten Verfahren, beispielsweise mit einem Reduktionsmittel wie Natriumtriacetoxyborhydrid, Natriumborhydrid oder Natriumcyanoborhydrid, zweckmäßigerweise in einem Lösungsmittel wie Tetrahydrofuran oder Dichlormethan, gegebenenfalls unter Zusatz von Essigsäure.

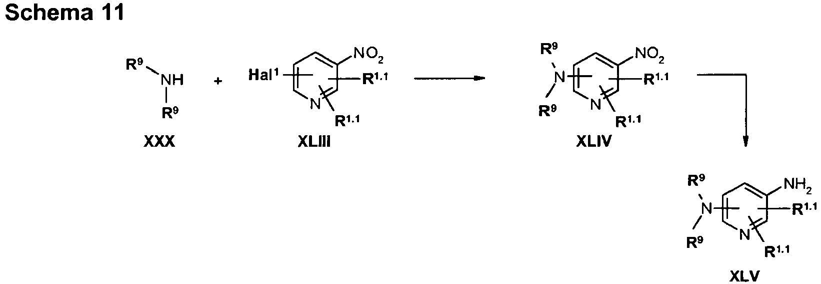

Die Reaktion eines Amins der allgemeinen Formel XXX, in der **R⁹** eine C₁₋₃-Alkylgruppe bedeutet, mit einem Halogen-nitropyridin der allgemeinen Formel **XLIII,** in der **R^{1.1}** wie voranstehend erwähnt definiert ist und **Hal¹** ein Chlor- oder Bromatom bedeutet, erfolgt nach bekannten Verfahren, beispielsweise in einem Lösungsmittel wie Tetrahydrofuran, Dichlormethan, Methanol oder DMSO und zweckmäßigerweise in Gegenwart einer passenden Base wie Triethylamin, Natronlauge oder Kaliumcarbonat und bei einer Temperatur von 20°C bis 100°C.

Die anschließende Reduktion der Nitrogruppe einer Verbindung der allgemeinen Formel **XLIV,** in der **R^{1.1}** wie voranstehend erwähnt definiert ist und **R⁹** eine C₁₋₃-Alkylgruppe bedeutet, zu Verbindungen der allgemeinen Formel XLV, in der **R^{1.1}** wie voranstehend erwähnt definiert ist und **R⁹** eine C₁₋₃-Alkylgruppe bedeutet, wird wie unter Schema 8 beschrieben durchgeführt.

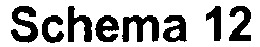

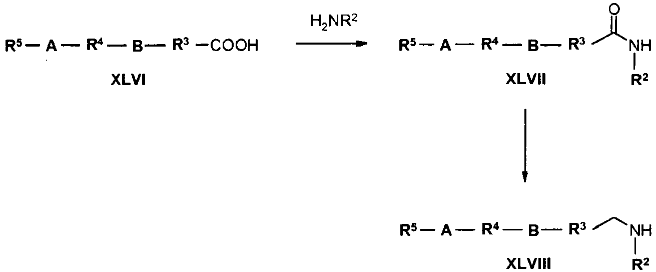

Die Amidknüpfung von Carbonsäuren der allgemeinen Formel **XLVI,** in der alle Reste wie voranstehend erwähnt definiert sind, und Aminen der allgemeinen Formel H₂N**R²**, in der **R²** wie voranstehend erwähnt definiert ist, zu Carbonsäureamiden der allgemeinen Formel **XLVII,** in der alle Reste wie voranstehend erwähnt definiert sind, wird wie unter Schema 1 beschrieben durchgeführt.

Die Reduktion von Carbonsäureamiden der allgemeinen Formel **XLVII** zu Aminen der allgemeinen Formel **XLVIII,** in der alle Reste wie voranstehend erwähnt definiert sind, erfolgt nach Standard-Reaktionsbedingungen, vorzugsweise in Gegenwart eines Reduktionsmittels wie Lithiumaluminiumhydrid und einem Lösungsmittel wie Tetrahydrofuran bei 40°C bis 100°C.

### Methodenbeschreibung zur hBK1-Rezeptorbindung

CHO-Zellen, die den hBK1-Rezeptor exprimieren, werden in "Dulbecco's modified Medium" kultiviert. Von konfluenten Kulturen wird das Medium entfernt, die Zellen werden mit PBS-Puffer gewaschen, abgeschabt und durch Zentrifugieren isoliert. Anschließend werden die Zellen in Suspension homogenisiert, das Homogenat zentrifugiert und resuspendiert. Nach Bestimmung des Proteingehalts wird die so erhaltene Membranpräparation bei -80°C eingefroren.

Nach dem Auftauen werden 200 µl des Homogenats (50 bis100 µg Protein/Assay) für 60 Minuten bei Raumtemperatur mit 0.5 bis 1.0 nM Kallidin (DesArg10,Leu9), [3,4-Prolyl-3,43H(N)] und ansteigenden Konzentrationen der Testsubstanz in einem Gesamtvolumen von 250 µl inkubiert. Die Inkubation wird durch rasche Filtration durch GF/B-Glasfaserfilter, die mit Polyethyleneimine (0.3%) vorbehandelt wurden, beendet. Die an das Protein gebundene Radioaktivität wird mit einem TopCount NXT gemessen. Als nichtspezifische Bindung wird die gebundene Radioaktivität in Gegenwart von 1.0 µM Kallidin (DesArg10,Leu9), [3,4-Prolyl-3,43H(N)] definiert. Die Analyse der Konzentrations-Bindungskurve erfolgt mit Hilfe einer computergestützten nichtlinearen Kurvenanpassung. Aus den so erhaltenen Daten wird für die Testsubstanz der entsprechende Kᵢ - Wert ermittelt.

Um zu zeigen, dass die Verbindungen der allgemeinen Formel **I** mit unterschiedlichen Strukturelementen gute bis sehr gute Bradikinin-B1-Rezeptor antagonistische Aktivitäten zeigen, werden in der folgenden Tabelle die Kᵢ-Werte angegeben, die gemäß dem voranstehenden Versuchsprotokoll erhalten wurden. Dazu wird angemerkt, dass die Verbindungen im Hinblick auf ihre unterschiedlichen strukturellen Elemente ausgewählt wurden und nicht um spezifische Verbindungen hervorzuheben:

| **Beispiel** | **Kᵢ [nM]** |
|---|---|
| (1) | 8.7 |
| (3) | 5.1 |

### INDIKATIONEN

Aufgrund ihrer pharmakologischen Eigenschaften eignen sich die neuen Verbindungen und deren physiologisch verträglichen Salze zur Behandlung von Krankheiten und Krankheitssymptomen, die wenigstens teilweise durch die Stimulierung von Bradykinin-B1-Rezeptoren hervorgerufen werden.

Aufgrund ihrer pharmakologischen Wirkung eignen sich die Substanzen zur Behandlung
(a) von **akuten Schmerzen** wie z.B. Zahnschmerzen, peri- und postoperativen Schmerzen, traumatischen Schmerzen, Muskelschmerzen, Verbrennungsschmerzen, Schmerzen bei Sonnenbrand, Trigeminusneuralgie, Schmerzen bei Koliken, sowie bei Spasmen des Magen-Darm-Trakts oder des Uterus;
(b) von **Eingeweideschmerzen** wie z.B. chronischen Beckenschmerzen, gynäkologischen Schmerzen, Schmerzen vor und während der Menstruation, Schmerzen bei Pankreatitis, bei peptischen Ulzera, bei interstitieller Zystitis, bei Nierenkolik, bei Angina pectoris, Schmerzen bei Kolon irritabile, bei nicht-ulzeröser Dyspepsie und bei Gastritis, nicht-kardialen Thoraxschmerzen und Schmerzen bei myokardialer Ischämie und Herzinfarkt;
(c) von **neuropathischen Schmerzen** wie z.B. schmerzhaften Polyneuropathien, Schmerzen bei diabetischer Neuropathie, AIDS-assoziierten neuropathischen Schmerzen, Schmerzen bei Lumbago, bei nicht-herpesassoziierter Neuralgie, bei Post-zoster Neuralgie, bei Nervenverletzungen, bei Schädel-Hirn-Trauma, Schmerzen bei Nervenchädigungen infolge von Toxinen oder Chemotherapie, Phantomschmerzen, Schmerzen bei multipler Sklerose, bei Nervenwurzelabriß und schmerzhaften traumatisch bedingten Einzelnervenschädigungen;
(d) von **entzündlichen / Schmerzrezeptor-vermittelten Schmerzen** im Zusammenhang mit Erkrankungen wie Osteoarthritis, rheumatoider Arthritis, rheumatischem Fieber, Tendo-Synovitis, Sehnenentzündungen, Gicht, Vulvodynie, Schädigungen und Erkrankungen der Muskeln und Faszien (muskuläre Verletzungen, Fibromyalgie), Osteoarthritis, juveniler Arthritis, Spondylitis, Gicht-Arthritis, Psoriasis-Arthritis, Fibromyalgie, Myositis, Migräne, Zahnerkrankungen, Influenza und anderen Virusinfektionen wie Erkältungen, systemischer Lupus erythematodes,
(e) von **Tumorschmerzen** im Zusammenhang mit Krebserkrankungen wie lymphatischer oder myeloischer Leukämie, Hodgkin Erkrankung, Non-Hodgkin Lymphomen, Lymphogranulomatose, Lymphosarkomen, soliden malignen Tumoren und ausgedehnten Metastasen;
(f) von **Kopfschmerz-Erkrankungen** wie z.B. Kopfschmerzen unterschiedlicher Ursache, Cluster-Kopfschmerz, Migräne (mit oder ohne Aura) und Spannungskopfschmerz.
   Weiterhin eigenen sich die Verbindungen zur Behandlung
(g) von entzündlichen Veränderungen im Zusammenhang mit Erkrankungen der Atemwege wie Asthma bronchiale, einschließlich allergischem Asthma (atopisch und nicht-atopisch) sowie Bronchospasmen bei Anstrengung, beruflich-bedingtem Asthma, viraler oder bakterieller Exazerbation einer bestehenden Asthma-Erkrankung und anderen nicht-allergisch bedingten asthmatischen Erkrankungen;
   chronisch obstruktiver Lungen-Erkrankung (COPD) einschließlich Lungenemphysem, akutem Atemnotsyndrom des Erwachsenen (ARDS), Bronchitis, Lungenentzündung, allergischer Rhinitis (saisonal und ganzjährig), vasomotorischer Rhinitis und Staublungen-Erkrankungen wie Aluminose, Anthrakose, Asbestose, Chalikose, Siderose, Silikose, Tabakose und Byssinose;
(h) von Entzündungserscheinungen bei Sonnenbrand und Verbrennungen, Ödemen nach Traumata durch Verbrennungen, Hirnödemen und Angioödemen, Darmerkrankungen einschließlich Morbus Crohn und Kolitis ulzerosa, Reizdarmsyndrom, Pankreatitis, Nephritis, Zystitis (interstitielle Zystitis), Uveitis; entzündlichen Erkrankungen der Haut (wie z.B. Psoriasis und Ekzeme), vaskulären Bindegewebserkrankungen, Lupus, Zerrungen und Frakturen;
(i) von Diabetes Mellitus und dessen Folgen (wie z.B. diabetische Vaskulopathie, diabetische Neuropathie, diabetische Retinopathie) und von diabetischen Symptomen bei Insulitis (z.B. Hyperglycämie, Diurese, Proteinurie und erhöhte renale Nitrit- und Kallikrein-Exkretion);
(j) von neurodegenerativen Erkrankungen wie der Parkinson'schen Erkrankung und der Alzheimer Erkrankung;
(k) von Sepsis und septischem Schock nach bakteriellen Infektionen oder nach Trauma;
(l) von Juckreiz verursachenden Syndromen und allergischen Hautreaktionen;
(m) von Osteoporose;
(n) von Epilepsie;
(o) von Verletzungen des Zentralnervensystems;
(p) von Wunden und Gewebeschädigungen;
(q) von Zahnfleischentzündungen;
(r) von benigner Prostata-Hyperplasie und hyperaktiver Blase;
(s) von Pruritus;
(t) von Vitiligo;
(u) von Störungen der Motilität von respiratorischen, genito-urinalen, gastro-intestinalen oder vaskulären Regionen und
(v) von post-operativem Fieber.

Neben der Eignung als Humantherapeutika, sind diese Substanzen auch nützlich in der veterinärmedizinischen Therapie von Haustieren, exotischen Tieren und Nutztieren.

### KOMBINATIONEN

Zur Behandlung von Schmerzen kann es vorteilhaft sein, die erfindungsgemässen Verbindungen mit belebenden Stoffen wie Koffein oder anderen schmerzlindernden Wirkstoffen zu kombinieren. Stehen zur Behandlung der Ursache der Schmerzen geeignete Wirkstoffe zur Verfügung, so können diese mit den erfindungsgemässen Verbindungen kombiniert werden. Sind unabhängig von der Schmerzbehandlung auch noch weitere medizinische Behandlungen angezeigt, zum Beispiel gegen Bluthochdruck oder Diabetes, so können auch die dafür nötigen Wirkstoffe mit den erfindungsgemässen Verbindungen kombiniert werden.

Für eine Kombinationstherapie kommen beispielsweise die folgenden Verbindungen in Frage:
Nicht-steroidale Antirheumatika (NSAR): COX-2 Hemmer wie Propionsäure-Derivate (Alminoprofen, Benoxaprofen, Bucloxinsäure, Carprofen, Fenhufen, Fenoprofen, Fiuprofen, Fiulbiprofen, Ibuprofen, Indoprofen, Ketoprofen, Miroprofen, Naproxen, Oxaprozin, Pirprofen, Pranoprofen, Suprofen, Tiaprofensäure, Tioxaprofen), Essigsäure-Derivate (Indomethacin, Acemetacin, Alcofenac, Isoxepac, Oxpinax, Sulindac, Tiopinac, Tolmetin, Zidometacin, Zomepirac) Fenaminsäure-Derivate (Meclofenaminsäure, Mefenaminsäure, Tolfenaminsäure), Biphenyl-Carboxylsäure-Derivate, Oxicame (Isoxicam, Meloxicam, Piroxicam, Sudoxicam und Tenoxicam), Salicylsäure-Derivate (Acetylsalicylsäure, Sulfasalazin, warum nicht auch Mesalazin, Olsalazin, und Pyrazolone (Apazon, Bezpiperylon, Feprazon, Mofebutazon, Oxyphenbutazon, Phenylbutazon, warum nicht auch Propyphenazon und Metamizol, und Coxibe (Celecoxib, Valecoxib, Rofecoxib, Etoricoxib).
Opiat Rezeptor Agonisten wie z. B. Morphin, Propoxyphen (Darvon), Tramadol, Buprenorphin.
Cannabinoid Agonisten wie z.B. GW-1000, KDS-2000, SAB-378, SP-104, NVP001-GW-843166, GW-842166X, PRS-211375.
Natriumkanalblocker wie z.B. Carbamazepin, Mexiletin, Lamotrigin, Pregabalin, Tectin, NW-1029, CGX-1002.
N-Typ Calciumkanalblocker wie z.B. Ziconitid, NMED-160, SP1-860.
Serotonerge und noradrenerge Modulatoren wie z.B. SR-57746, Paroxetin, Duloxetin, Clonidin, Amitriptylin, Citalopram.
Corticosteroide wie z.B. Betamethason, Budesonid, Cortison, Dexamethason, Hydrocortison, Methylprednisolon, Prednisolon, Prednison und Triamcinolon.
Histamin H1-Rezeptorantagonisten wie z.B. Bromophtniramint, Chlorpheniramin, Dexchlorpheniramin, Triprolidin, Clemastin, Diphenhydramin, Diphenylpyralin, Tripelennamin, Hydroxyzin, Methdijazin, Promethazin, Trimeprazin Azatadin, Cyproheptadin, Antazolin, Pheniramin, Pyrilamin, Astemizol, Terfenadin, Loratadin, Cetirizin, Desloratadin, fexofenadin, Levocetirizin.
Histamin H2-Rezeptor Antagonisten wie z.B. Cimetidin, Famotidin, und Ranitidin. Protonenpumpenhemmer wie z.B. Omeprazol, Pantoprazol, Esomeprazol.
Leukotrien Antagonisten und 5-Lipoxygenasehemmer wie z.B. Zafirlukast, Montelukast, Pranlukast und Zileuton.
Lokalanästhetika wie z.B. Ambroxol, Lidocain.
VR1 Agonisten und Antagonisten wie z.B. NGX-4010, WL-1002, ALGRX-4975, WL-10001, AMG-517.
Nikotinrezeptor Agonisten wie z.B. ABT-202, A-366833, ABT-594, BTG-102, A-85380, CGX1204.
P2X3-Rezeptor Antagonisten wie z.B. A-317491, ISIS-13920, AZD-9056.
NGF Agonisten und Antagonisten wie z.B. RI-724, RI-1024, AMG-819, AMG-403, PPH 207.
NK1 und NK2 Antagonisten wie z.B. DA-5018, R-116301, CP-728663, ZD-2249.
NMDA Antagonisten wie z.B. NER-MD-11, CNS-5161, EAA-090, AZ-756, CNP-3381. Kaliumkanal Modulatoren wie z.B. CL-888, ICA-69673, Retigabin.
GABA Modulatoren wie z.B. Lacosamid.
Serotonerge und noradrenerge Modulatoren wie z.B. SR-57746, Paroxetin, Duloxetin, Clonidin, Amitriptylin, Citalopram, Flibanserin.
Anti-Migräne Therapeutika wie z.B. Sumatriptan, Zolmitriptan, Naratriptan, Eletriptan.

Die zur Erzielung einer schmerzstillenden Wirkung erforderliche Dosierung beträgt bei intravenöser Gabe zweckmäßigerweise 0.01 bis 3 mg/kg Körpergewicht, vorzugsweise 0.1 bis 1 mg/kg, und bei oraler Gabe 0.1 bis 8 mg/kg Körpergewicht, vorzugsweise 0.5 bis 3 mg/kg, jeweils 1 bis 3 x täglich. Die erfindungsgemäß hergestellten Verbindungen können intravenös, subkutan, intramuskulär, intrarektal, intranasal, durch Inhalation, transdermal oder oral verabreicht werden, wobei zur Inhalation insbesondere Aerosolformulierungen geeignet sind. Sie können gegebenenfalls zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen, Lösungen, Dosieraerosole oder Zäpfchen eingearbeitet werden.

### EXPERIMENTELLER TEIL

Für die hergestellten Verbindungen liegen in der Regel IR-, ¹H-NMR und/oder Massenspektren vor. Die bei den Fliessmitteln angegebenen Verhältnisse beziehen sich auf Volumeneinheiten der jeweiligen Lösungsmittel. Die angegebenen Volumeneinheiten bei Ammoniak beziehen sich auf eine konzentrierte Lösung von Ammoniak in Wasser. Soweit nicht anders vermerkt sind die bei den Aufarbeitungen der Reaktionslösungen verwendeten Säure-, Basen- und Salzlösungen wässrige Systeme der angegebenen Konzentrationen.

Zu chromatographischen Reinigungen wird Kieselgel der Firma Millipore (MATREX*™*, 35-70 µm) oder Alox (E. Merck, Darmstadt, Aluminiumoxid 90 standardisiert, 63-200 µm, Artikel-Nr: 1.01097.9050) verwendet.

In den Versuchsbeschreibungen werden die folgenden Abkürzungen verwendet:
- Alox: Aluminiumoxid
- BOC: tert-Butoxycarbonyl
- DC: Dünnschichtchromatogramm
- DCM: Dichlormethan
- DIPEA: Diisopropylethylamin
- DMAP: 4-Dimethylaminopyridin
- DMF: Dimethylformamid
- DMSO: Dimethylsulfoxid
- EE: Essigester
- HCl: Salzsäure
- MeOH: Methanol
- NaOH: Natronlauge
- TEA: Triethylamin
- tert: tertiär
- TBTU: 2-(1*H*-Benzotriazol-1-yl)-1,1,3,3-tetramethyluronium-tetrafluorborat
- TFA: Trifluoressigsäure
- THF: Tetrahydrofuran

Folgende analytische HPLC-Methode wurde verwendet:
Methode 1:
Säule: Zorbax Stable Bond C18, 3.5 µM, 4.6 x 75 mm
Detektion: 230 - 360 nm
Fließmittel A: Wasser / 0.1 % Ameisensäure
Fließmittel B: Acetonitril / 0.1 % Ameisensäure
Gradient:

| Zeit in min | %A | %B | Flussrate in mL/min |
|---|---|---|---|
| 0.0 | 95.0 | 5.0 | 1.6 |
| 0.1 | 95.0 | 5.0 | 1.6 |
| 4.5 | 10.0 | 90.0 | 1.6 |
| 5.09 | 10.0 | 90.0 | 1.6 |
| 5.5 | 90.0 | 10.0 | 1.6 |

Methode 2:
Säule: Merck Cromolith Speed ROD RP18e, 4.6 x 50 mm
Detektion: 190 - 400 nm
Fließmittel A: Wasser / 0.1 % Ameisensäure
Fließmittel B: Acetonitril / 0.1 % Ameisensäure
Gradient:

| Zeit in min | %A | %B | Flussrate in mL/min |
|---|---|---|---|
| 0.0 | 90.0 | 10.0 | 1.5 |
| 4.5 | 10.0 | 90.0 | 1.5 |
| 5.0 | 10.0 | 90.0 | 1.5 |
| 5.5 | 90.0 | 10.0 | 1.5 |

Methode 3:
Säule: Merck Cromolith Flash RP18e, 25 x 4.6 mm
Detektion: 190 - 400 nm
Fließmittel A: Wasser / 0.1 % Ameisensäure
Fließmittel B: Acetonitril / 0.1 % Ameisensäure
Gradient:

| Zeit in min | %A | %B | Flussrate in mL/min |
|---|---|---|---|
| 0.0 | 90.0 | 10.0 | 1.6 |
| 2.7 | 10.0 | 90.0 | 1.6 |
| 3.0 | 10.0 | 90.0 | 1.6 |
| 3.3 | 90.0 | 10.0 | 1.6 |

Methode 4:
Säule: Waters Xbridge C18, 2.5 µM, 3.0 x 30 mm
Detektion: 230 - 360 nm
Fließmittel A: Wasser / 0.1 % Ammoniak
Fließmittel B: Acetonitril / 0.1 % Ammoniak
Gradient:

| Zeit in min | %A | % B | Flussrate in mL/min |
|---|---|---|---|
| 0.0 | 95.0 | 5.0 | 1.4 |
| 1.6 | 50.0 | 5.0 | 1.4 |
| 1.8 | 10.0 | 90.0 | 1.4 |
| 2.0 | 10.0 | 90.0 | 1.4 |
| 2.2 | 95 | 10 | 1.4 |

Methode 5:
Säule: Waters Xbridge C18, 2.5 µM, 3.0 x 30 mm
Detektion: 230 - 360 nm
Fließmittel A: Wasser / 0.1 % Ammoniak
Fließmittel B: Acetonitril / 0.1 % Ammoniak
Gradient:

| Zeit in min | %A | %B | Flussrate in mL/min |
|---|---|---|---|
| 0.0 | 95.0 | 5.0 | 1.4 |
| 1.8 | 10.0 | 90.0 | 1.4 |
| 2.0 | 10.0 | 90.0 | 1.4 |
| 2.0 | 10.0 | 90.0 | 1.4 |

Methode 6:
Säule: Waters Xbridge C18, 2.5 µM, 3.0 x 30 mm
Detektion: 230 - 360 nm
Fließmittel A: Wasser / 0.1 % Ammoniak
Fließmittel B: Acetonitril / 0.1 % Ammoniak
Gradient:

| Zeit in min | %A | %B | Flussrate in mL/min |
|---|---|---|---|
| 0.0 | 95.0 | 5.0 | 1.4 |
| 0.8 | 10.0 | 90.0 | 1.4 |
| 2.0 | 10.0 | 90.0 | 1.4 |
| 2.2 | 95.0 | 5.0 | 1.4 |

Die folgenden präparativen Methoden wurden zur Umkehrphasen-Chromatographie verwendet:
Methode 7:
Säule: Varian C18 Microsorb, 10 µM, 50 x 160 mm
Detektion: UV gesteuert
Fließmittel A: Wasser / 0.2 % TFA
Fließmittel B: Acetonitril
Gradient:

| Zeit in min | %A | %B | Flussrate in mL/min |
|---|---|---|---|
| 0.0 | 90.0 | 10.0 | 100.00 |
| 1.0 | 90.0 | 10.0 | 100.00 |
| 1.5 | 90.0 | 10.0 | 100.00 |
| 13.0 | 0 | 100.0 | 100.00 |
| 15.5 | 0 | 100.0 | 100.00 |
| 15.75 | 90.0 | 10.0 | 100.00 |
| 18.3 | 90.0 | 10.0 | 100.00 |

Methode 8:
Säule: Merck Chromolith-prep RP 18 e, 100 x 25 mm
Detektion: UV gesteuert
Fließmittel A: Wasser / 0.1 % TFA
Fließmittel B: Acetonitril / 0.1 % TFA
Gradient:

| Zeit in min | %A | %B | Flussrate in mL/min |
|---|---|---|---|
| 0.0 | 90.0 | 10.0 | 20.00 |
| 7.5 | 10.0 | 90.0 | 20.00 |
| 9.0 | 10.0 | 90.0 | 20.00 |
| 10.0 | 90.0 | 10.0 | 20.00 |

Methode 9:
Säule: Varian Pursuit XRs, 10 µM, 50 x 250 mm
Detektion: UV gesteuert
Fließmittel A: Wasser / 0.2 % TFA
Fließmittel B: Acetonitril
Gradient:

| Zeit in min | %A | %B | Flussrate in mL/min |
|---|---|---|---|
| 0.0 | 90.0 | 10.0 | 100.00 |
| 1.0 | 90.0 | 10.0 | 100.00 |
| 1.5 | 90.0 | 10.0 | 100.00 |
| 13.0 | 0 | 100.0 | 100.00 |
| 15.5 | 0 | 100.0 | 100.00 |
| 15.75 | 90.0 | 10.0 | 100.00 |
| 18.3 | 90.0 | 10.0 | 100.00 |

Methode 10:
Säule: Varian C18 Pursuit XRs, 10 µM, 50 x 250 mm
Detektion: UV gesteuert
Fließmittel A: Wasser / 0.2 % Ammoniak
Fließmittel B: Acetonitril
Gradient:

| Zeit in min | %A | %B | Flussrate in mL/min |
|---|---|---|---|
| 0.0 | 90.0 | 10.0 | 100.00 |
| 1.0 | 90.0 | 10.0 | 100.00 |
| 1.5 | 90.0 | 10.0 | 180.00 |
| 13.0 | 0 | 100.0 | 180.00 |
| 15.5 | 0 | 100.0 | 180.00 |
| 15.75 | 90.0 | 10.0 | 180.00 |
| 19.0 | 90.0 | 10.0 | 180.00 |

Methode 11:
Säule: Waters XBridge C18, 5 µM, 50 x 150 mm
Detektion: MS oder UV gesteuert
Fließmittel A: Wasser / 0.3 % Ammoniak
Fließmittel B: Acetonitril
Gradient:

| Zeit in min | %A | %B | Flussrate in mL/min |
|---|---|---|---|
| 0.0 | 90.0 | 10.0 | 120.00 |
| 2.0 | 90.0 | 10.0 | 120.00 |
| 9.0 | 5.0 | 95.0 | 120.00 |
| 15.0 | 5.0 | 95.0 | 120.00 |
| 15.5 | 95.0 | 5.0 | 120.00 |
| 17.5 | 95.0 | 5.0 | 120.00 |

Methode 12:
Säule: Varian Microsorb C18, 10 µM, 50 x 160 mm
Detektion: MS oder UV gesteuert
Fließmittel A: Wasser / 0.1 % TFA
Fließmittel B: Methanol
Gradient:

| Zeit in min | %A | %B | Flussrate in mL/min |
|---|---|---|---|
| 0.0 | 95.0 | 5.0 | 150.00 |
| 1.15 | 95.0 | 5.0 | 150.00 |
| 12.4 | 2.0 | 98.0 | 150.00 |
| 14.0 | 2.0 | 98.0 | 150.00 |
| 14.05 | 95.0 | 5.0 | 150.00 |
| 15.3 | 95.0 | 5.0 | 150.00 |

Die folgenden HPLC-Methoden wurden zur präparativen Enantiomerentrennung verwendet:
- Methode 13:: Säule: Daicel OJ-H, 250 x 4.6 mm, 5 µm
Detektion: 230 - 360 nm
Fließmittel: n-Hexan + 0.2 % Diethylamin / Ethanol = 70:30
Flußrate: 12 ml/min
Gradient: isokratisch
- Methode 14:: Säule: Daicel AD-H, 250 * 4,6 mm, 5 µm, 10 °C
Detektion: 230 -360 nm
Fließmittel: n-Hexan + 0,2 % Diethylamin / i-Propyl = 85 :15
Flußrate: 1 ml/min
Gradient: isokratisch

Folgende Mikrowellen-Apparatur wurde verwendet: Biotage EmrysOptimizer™

### Herstellung der Endverbindungen

### Beispiel 1

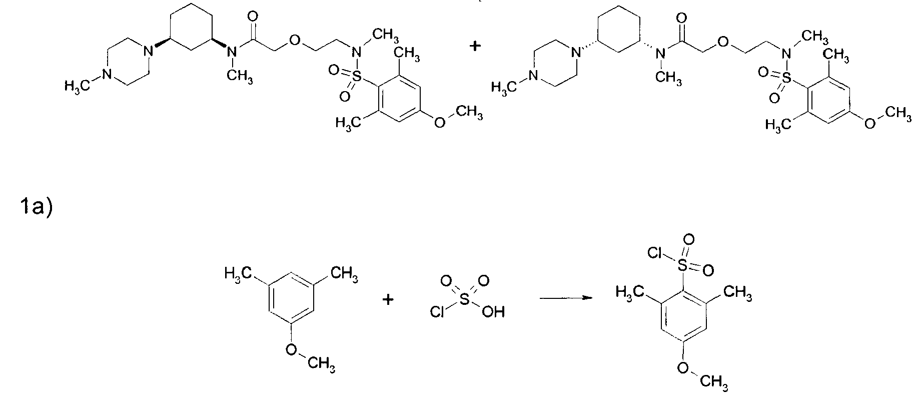

Eine Mischung aus 2.0 g (14.69 mmol) 3,5-Dimethylanisol und 20 ml Dichlormethan wurde unter Eisbadkühlung mit 5.85 ml (88.0 mmol) Chlorsulfonsäure versetzt. Die Reaktionsmischung wurde danach 20 min bei Raumtemperatur gerührt und anschließend auf 50 ml Eiswasser gegossen. Man extrahierte mit 100 ml Dichlormethan. Die organischen Extrakte wurden mit 5%iger Natriumhydrogencarbonat-Lösung gewaschen, über Natriumsulfat getrocknet und bis zur Trockene eingeengt.
C₉H₁₁ClO₃S (234.70)
[M+H]+ = 234/236
DC: Kieselgel, Petrolether / Ethylacetat 9:1, Rf-Wert = 0.46

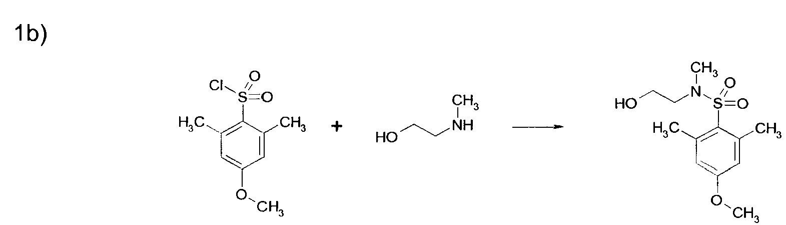

1.69 g (21.1 mmol) N-Methylaminoethanol (BASF) und 6.68 ml (47.9 mmol) Triethylamin wurden in 100 ml Dichlormethan gelöst. Bei 0°C wurden 4.50 g (19.2 mmol) Produkt aus 1 a) gelöst in 50 ml Dichlormethan zugetropft. Man entfernte die Kühlung und rührte 1.5 Stunden bei Raumtemperatur. Das Reaktionsgemisch wurde anschließend mit 1 N Salzsäure und 5-prozentiger Natriumhydrogencarbonatlösung gewaschen. Die organische Phase wurde über Natriumsulfat getrocknet und bis zur Trockene eingeengt.
C₁₂H₁₉NO₄S (273.35)
[M+H]+ = 274
DC: Kieselgel, Dichlormethan / Ethanol 19:1, Rf-Wert = 0.43

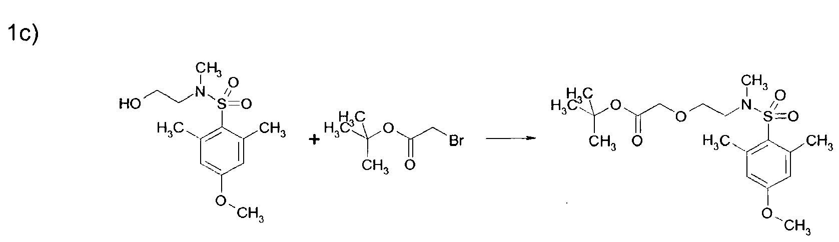

Eine Mischung aus 5.15 g (18.8 mmol) Produkt aus 1 b), 1.75 g (6.60 mmol) Tetrabutylammoniumchlorid (Fluka) und 80 ml Toluol wurde bei 0°C zuerst mit 100 ml 35%iger Natronlauge, dann mit 4.18 ml (28.3 mmol) Bromessigsäure-tert-butylester in 20 ml Toluol versetzt. Die Reaktionsmischung wurde anschließend 1.5 Stunden bei Raumtemperatur gerührt, dann mit Diethylether verdünnt. Nach der Phasentrennung wurde die organische Phase viermal mit Wasser neutral gewaschen, über Natriumsulfat getrocknet und im Vakuum bis zur Trockene eingeengt. Das so erhaltene Rohprodukt wurde durch Säulenchromatographie über Kieselgel (Elutionsmittel: Petrolether / Ethylacetat 4:1) gereinigt. C₁₈H₂₉NO₆S (387.49)
[M+H]+ = 388
DC: Kieselgel, Petrolether / Ethylacetat 7:3, Rf-Wert = 0.59

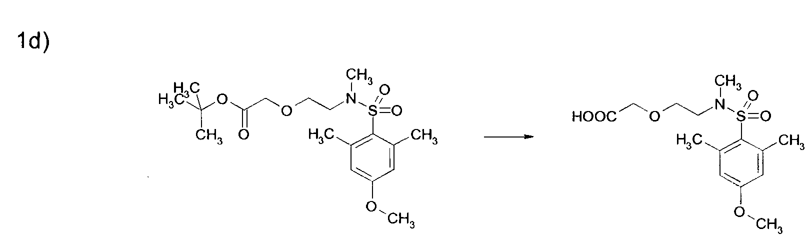

Eine Mischung aus 6.80 g (17.6 mmol) Produkt 1c), 8 ml TFA und 100 ml Dichlormethan wurde 2.5 Stunden bei Raumtemperatur gerührt. Die Reaktionsmischung wurde danach im Vakuum bis zur Trockene eingeengt. Man versetzte den Rückstand mit 1 N Natronlauge und extrahierte zweimal mit Ethylacetat (organische Extrakte verworfen). Die wässrige Phase wurde mit 2M Salzsäure angesäuert, dann nochmals mit Ethylacetat extrahiert. Die organischen Extrakte wurden mit Wasser gewaschen, über Natriumsulfat getrocknet und im Vakuum bis zur Trockene eingeengt.
C₁₄H₂₁NO₆S (331.29)
[M+H]+ = 332
analytische HPLC (Methode 1): Retentionszeit = 3.4 min

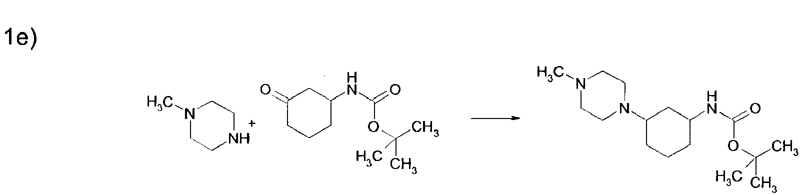

2.6 ml (23.4 mmol) 1-Methylpiperazin, 1.0 g (4.69 mmol) 3-Amino-N-tert-butyloxycarbonylcyclohexanon (AB Chem) und 2.7 ml (49 mmol) Eisessig wurden in 10 ml Methanol gelöst und 30 Minuten bei Raumtemperatur gerührt. Danach gab man portionsweise 1.99 g (9.38 mmol) Natriumtriacetoxyborhydrid zu und rührte 2 Stunden bei Raumtemperatur. Anschließend wurde die Reaktionslösung mit Hydrogencarbonatlösung versetzt und mit Dichlormethan extrahiert. Die organische Phase wurde im Vakuum vom Lösungsmittel befreit und der Rückstand an RP-Phase (Varian C18 XRS, präparative HPLC-Methode 10) chromatographiert.
C₁₆H₃₁N₃O₂ (297.44)
[M+H]+ = 298

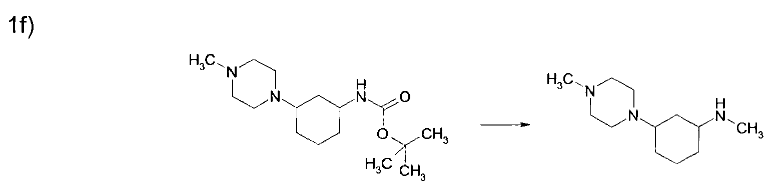

8.57 ml (8.57 mmol) einer 1 M Lösung von Lithiumaluminiumhydrid in Toluol wurden in 8 ml THF gelöst und bei Raumtemperatur langsam mit 850 mg (2.86 mmol) Produkt 1e) gelöst in 2 ml THF versetzt. Die Reaktionslösung wurde 2 Stunden bei 75°C gerührt. Anschließend gab man 1 N Natronlauge zu und Wasser. Der Niederschlag wurde abgesaugt und die Reaktionslösung bis zur Trockene eingeengt.
C₁₂H₂₅N₃ (211.35)
[M+H]+ = 212
analytische HPLC (Methode 2): Retentionszeit = 0.29 min

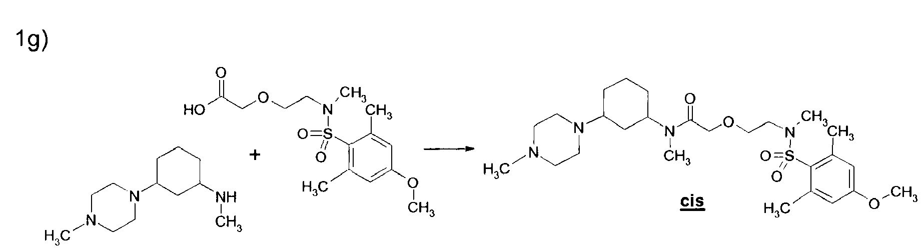

360 mg (1.01 mmol) Produkt 1d), 384 mg (1.20 mmol) TBTU und 151 µl (1.09 mmol) Triethylamin wurden in 5 ml DMF gelöst und 5 Minuten bei Raumtemperatur gerührt. Dann wurden 460 mg (2.18 mmol) Produkt 1f) zugegeben. Man rührte 2 Stunden bei Raumtemperatur und entfernte anschließend das Lösungsmittel im Vakuum. Zur Trennung des *cis*/*trans*-Gemisches wurde der Rückstand an RP-Phase (Merck Chromolith Speed ROD) chromatographiert (Wasser + 0. 1 % Ameisensäure / Acetonitril + 0.1% Ameisensäure = 90:10 -> 0:100). Man erhielt so das racemische Gemisch der *cis-*Isomeren:
C₂₆H₄₄N₄O₅S (524.72)
[M+H]+=525
analytische HPLC (Methode 2): Retentionszeit = 1.53 min

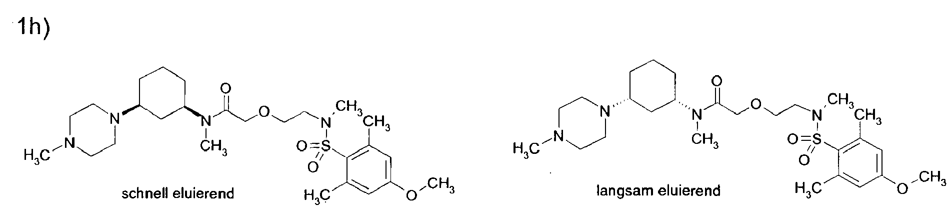

31 mg Produkt 1g (*cis*-Diastereomer) wurden nach HPLC-Methode 13 an chiraler Phase in die Enantiomere aufgetrennt.

HPLC (Methode 13): Retentionszeiten = 7.3 min (schnell eluierendes Enantiomer), 9.7 min (langsam eluierendes Enantiomer)

### Beispiel 2

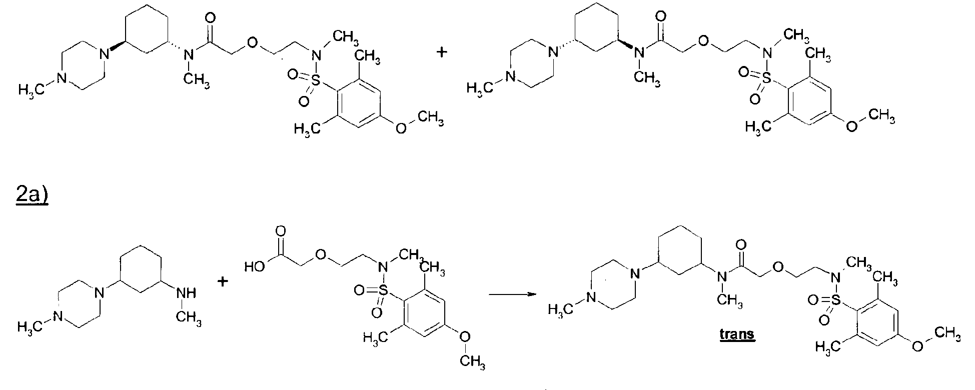

Analog Beispiel 1g) wurde die Titelverbindung aus Produkt 1d) und Produkt 1f) hergestellt. Zur Trennung des *cis*/*trans*-Gemisches wurde der Rückstand an RP-Phase (Merck Chromolith Speed ROD) chromatographiert (Wasser + 0.1% Ameisensäure / Acetonitril + 0.1% Ameisensäure = 90:10 -> 0:100). Man erhielt so das racemische Gemisch der *trans-*Isomeren:
*trans-*Diastereomer: C₂₆H₄₄N₄O₅S (524.72)
[M+H]+ = 525
analytische HPLC (Methode 2): Retentionszeit = 1.41 min

### Beispiel 3

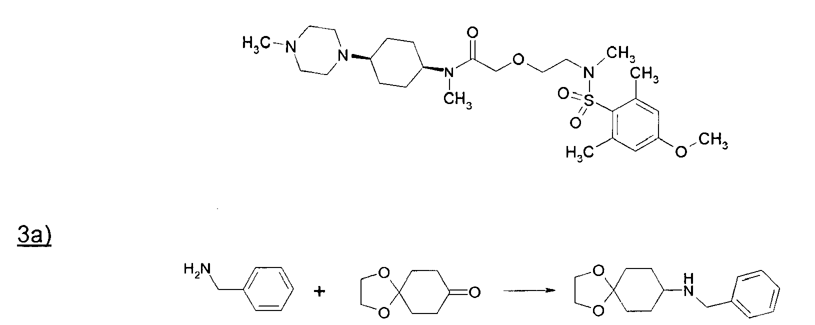

110 ml (1 mol) Benzylamin und 156.2 g (1 mol) Cyclohexandion-monoethylenketal, gelöst in 0.8 l Toluol, wurden 2 h am Wasserabscheider gekocht. Danach wurde das Reaktionsgemisch eingeengt und der Rückstand in 1 l EtOH aufgenommen. Die Lösung wurde bei 15-20°C portionsweise mit 23 g (0.61 mol) Natriumborhydrid versetzt und über Nacht gerührt. Danach wurde das Reaktionsgemisch eingeengt und der Rückstand mit 500 ml Wasser versetzt und zweimal mit je 400 ml Ether extrahiert. Die organische Phase wurde mit Wasser gewaschen, getrocknet und eingeengt. Der Rückstand wurde im Hochvakuum destilliert.
C₁₅H₂₁NO₂ (247.33)
[M+H]⁺ = 248
Siedetemperatur = 125-127°C (bei 0.06 mbar)

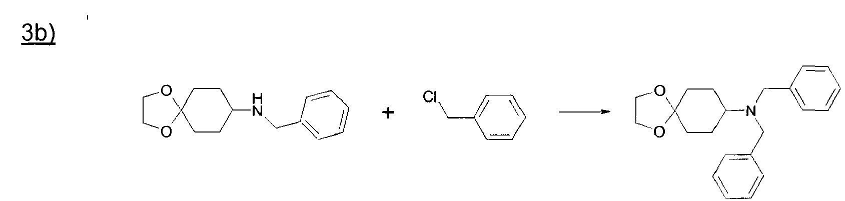

208 g (0.84 mol) Produkt 3a), 114 g (0.9 mol) Benzylchlorid, 138 g (1.0 mol) Kaliumcarbonat und 14 g (0.08 mol) Kaliumjodid wurden in 400 ml *N*-Methylpyrrolidon suspendiert und 24 h bei 80 °C gerührt. Danach wurde der Ansatz abgekühlt und mit 5 l Wasser versetzt. Die ausgefallenen Kristalle wurden abgesaugt, mit Wasser gewaschen und in DCM aufgenommen. Die organische Phase wurde getrocknet und eingeengt. Die Kristalle wurden aus MeOH umkristallisiert.
C₂₀H₂₃NO (293.40)

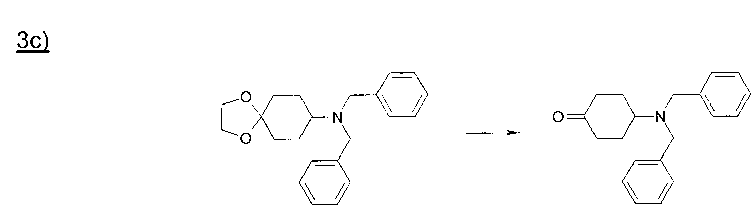

200g (0.44 mol) Produkt 3c) wurden in 400 ml Wasser suspendiert, mit 100 ml 37 %iger Salzsäure versetzt und bei 60°C 2 h gerührt. Danach wurde die Lösung abgekühlt und mit 200 g (1.45 mol) Kaliumcarbonat alkalisch gestellt. Die ausgefallenen Kristalle wurden abgesaugt und mit Wasser gewaschen. Die Kristalle wurden dann in DCM gelöst, das organische Lösungsmittel getrocknet und eingeengt. Der Rückstand wurde aus Petrolether kristallisiert.
C₂₀H₂₃NO (293.40)
[M+H]⁺ = 294

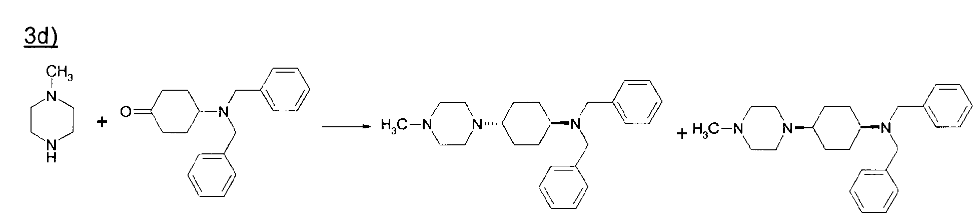

1 ml (9.02 mmol) 1-Methylpiperazin und 2.65 g (9.02 mmol) Produkt 3c) wurden in 40 ml THF gelöst und 1 h bei 50°C gerührt. Danach wurden bei RT 2.87 g (13.52 mmol) Natriumtriacetoxyborhydrid zugegeben und anschließend 2 h bei RT gerührt. Der Ansatz wurde dann mit ca. 150 ml DCM versetzt, mit NaHCO₃-Lösung ausgeschüttelt und nochmals dreimal mit DCM extrahiert. Die organische Phase wurde getrocknet und eingeengt. Der Rückstand wurde über präparative HPLC (Methode 10) gereinigt. C₂₅H₃₅N₃ (377.57)
[M+H]⁺ = 378
analytische HPLC (Methode 3): Retentionszeit = 0.68 min

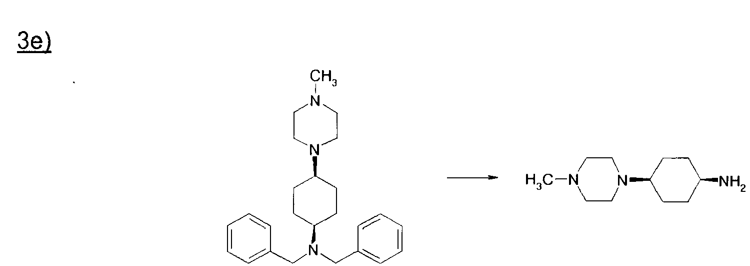

644 mg (1.063 mmol) Produkt 3d) (*cis*-Isomer) wurden in 25 ml MeOH gelöst und mit 150 mg Katalysator Palladium/C versetzt. Das Reaktionsgemisch wurde 5 h bei RT und 50 psi in einer Wasserstoffatmosphäre geschüttelt. Anschließend wurde der Katalysator abfiltriert und das Filtrat eingedampft.
C₁₁H₂₃N₃ x 2C₂HF₃O₂ (425.37)
[M+H]⁺ = 198

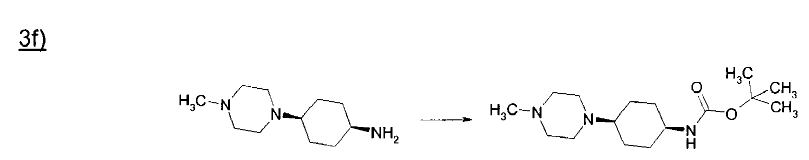

384 mg (0.9 mmol) Produkt 3e) und 0.63 ml (4.51 mmol) TEA wurden in 5 ml DMF gelöst und mit 223.4 mg Di-tert-butyl-dicarbonat versetzt. Das Reaktionsgemisch wurde über Nacht gerührt. Dann wurde das Lösungsmittel im Vakuum abgedampft und der Rückstand über präparative HPLC (Methode 11) gereinigt.
C₁₆H₃₁N₃O₂ (297.44)
[M+H]⁺ = 298
analytische HPLC (Methode 3): Retentionszeit = 0.29 min

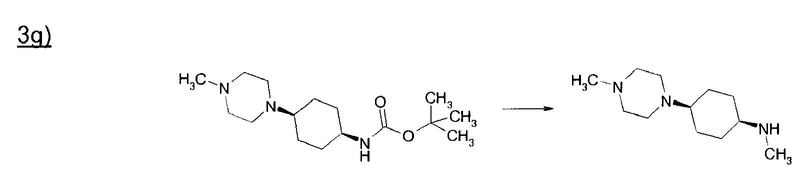

Zu 1.61 ml (1.61 mmol) einer 1 M Lithiumaluminumhydrid-Lösung in THF wurden bei RT unter Stickstoffatmosphäre 160 mg Produkt 3f), gelöst in 10 ml THF, langsam zugetropft und anschließend 3.5 h bei 75°C gerührt. Dann wurde die abgekühlte Lösung mit wenig Wasser verrührt, mit 4 ml 1M NaOH versetzt und filtriert. Das Lösungsmittel wurde abgedampft.
C₁₂H₂₅N₃ (211.35)
[M+H]⁺ = 212
analytische HPLC (Methode 3): Retentionszeit = 0.30 min

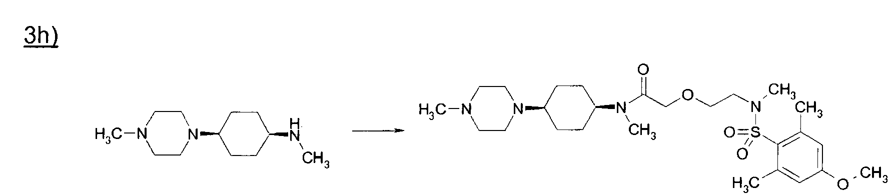

Analog Beispiel 1g) wurde die Titelverbindung aus Produkt 1d) und Produkt 3g) hergestellt.
C₂₆H₄₄N₄O₅S x 2C₂HF₃O₂ (752.76)
[M+H]+ = 525
analytische HPLC (Methode 3): Retentionszeit = 1.41 min

### Beispiel 4

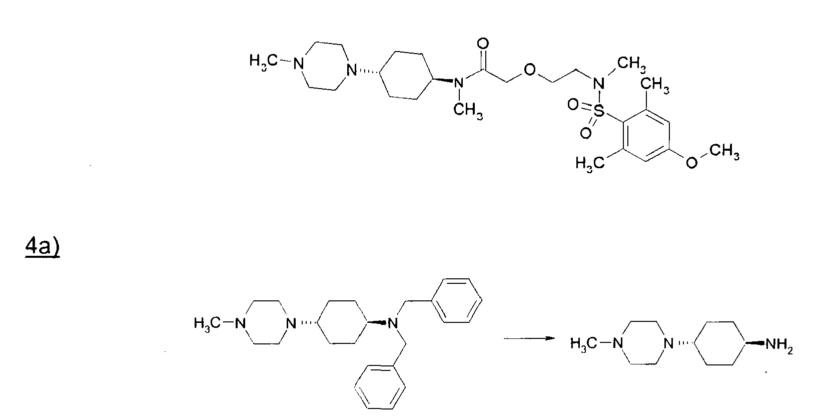

Analog Beispiel 3e) wurde die Titelverbindung aus Produkt 3d) (trans-Isomer) hergestellt. C₁₁H₂₃N₃ x 2C₂HF₃O₂ (425.37)
[M+H]⁺ = 198

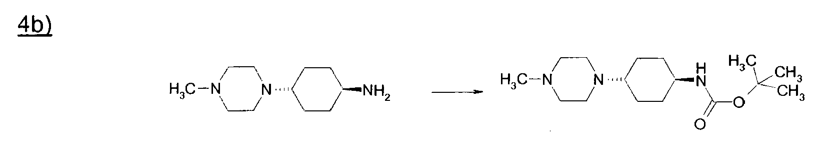

Analog Beispiel 3f) wurde die Titelverbindung aus Produkt 4a) hergestellt.
C₁₆H₃₁N₃O₂ (297.44)
[M+H]⁺ = 298

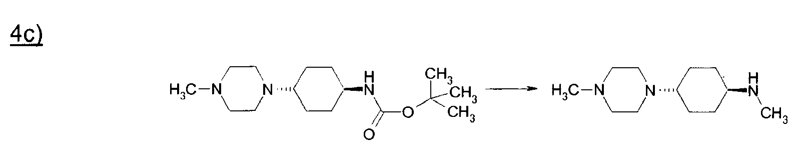

Analog Beispiel 3g) wurde die Titelverbindung aus Produkt 4b) hergestellt. C₁₂H₂₅N₃ (211.35)
[M+H]⁺ = 212

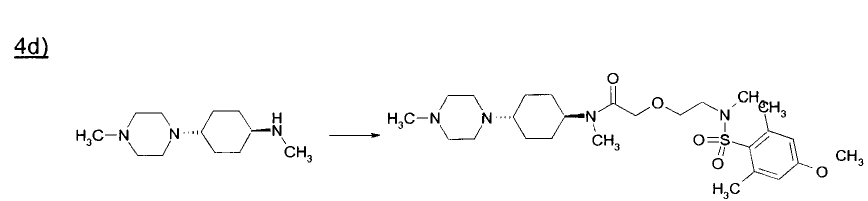

Analog Beispiel 1g) wurde die Titelverbindung aus Produkt 1d) und Produkt 4c) hergestellt.
C₂₆H₄₄N₄O₅S (524.72)
[M+H]+ = 525
analytische HPLC (Methode 3): Retentionszeit = 1.32 min

### Beispiel 5

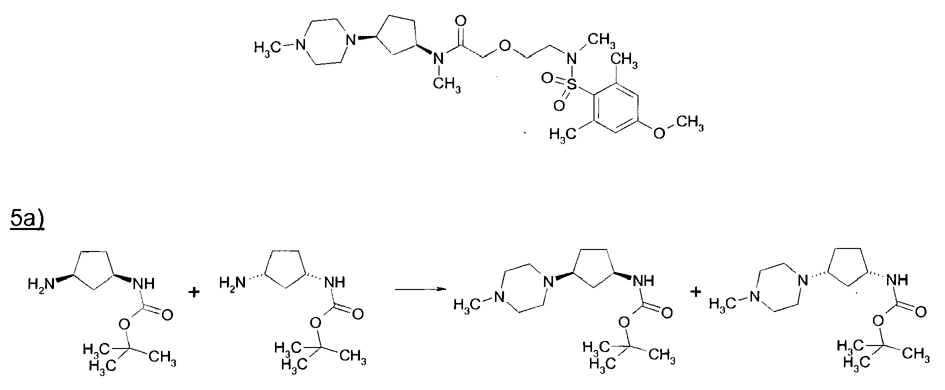

0.5 g (2.5 mmol) 3-Amino-cyclopentyl-carbaminsäure-tert-butylester (racemisch *cis*), 1.73 g (12.5 mmol) Kaliumcarbonat und 0.01 g Kaliumjodid wurden in 20 ml Acetonitril suspendiert. Anschließend wurde 0.48 g (2.5 mmol) Bis-(2-chlor-ethyl)-methylamin-Hydrochlorid zugegeben und 4 h unter Rückfluß gekocht. Nachdem das Reaktionsgemisch abgekühlt war, wurde mit DCM verdünnt und mit 1 M HCl extrahiert. Die organische Phase wurde mit Natriumsulfat getrocknet und einrotiert. Der Rückstand wurde über präparative HPLC (Methode 8) gereinigt.
C₁₅H₂₉N₃O₂ (283.41)
[M+H]⁺ = 201

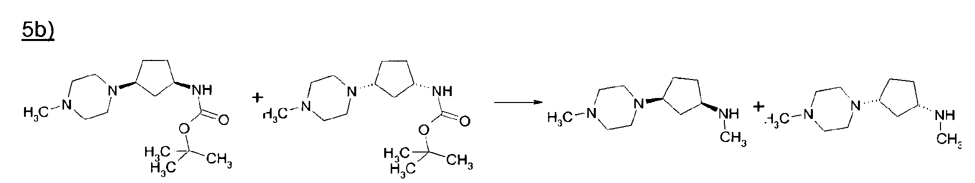

Analog Beispiel 3g) wurde die Titelverbindung aus Produkt 5a) hergestellt. C₁₁H₂₃N₃ (197.32)
[M+H]⁺ = 198

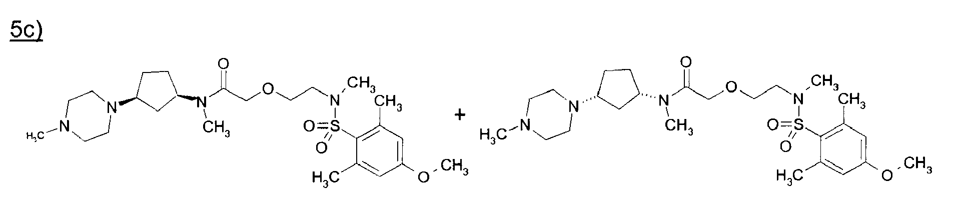

Analog Beispiel 1g) wurde die Titelverbindung aus Produkt 5b) und Produkt 1d) als racemisches Gemisch der beiden *cis*-Isomeren hergestellt.
C₂₅H₄₂N₄O₅S x C₂HF₃O₂ (624.71)
[M+H]⁺ = 511
analytische HPLC (Methode 3): Retentionszeit = 1.43 min

### Beispiel 6

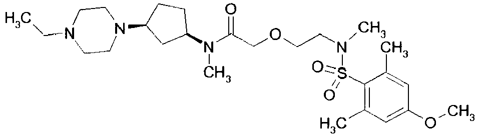

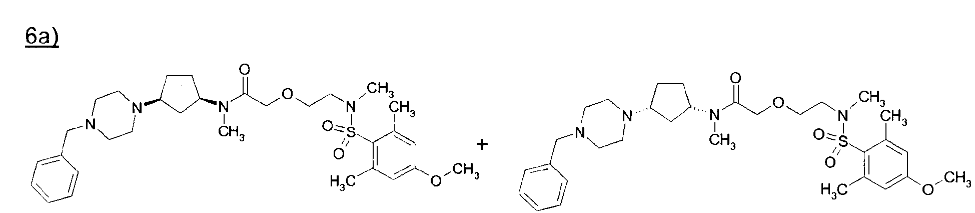

Die Titelverbindung wurde analog Beispiel 1g) als racemisches Gemisch der *cis*-Isomeren hergestellt.
C₃₁H₄₆N₄O₅S (586.79)
[M+H]⁺ = 587

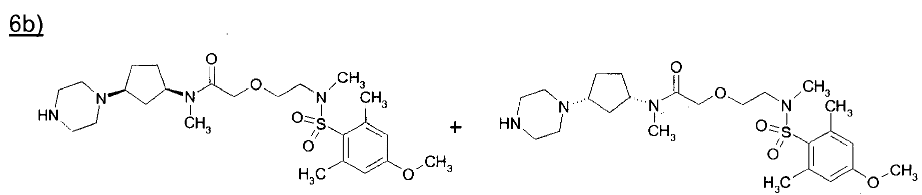

156 mg (0.266 mmol) Produkt 6c) wurden in 5 ml MeOH gelöst und mit 50 mg Katalysator Palladium/C versetzt. Das Reaktionsgemisch wurde über Nacht bei RT und 50 psi in einer Wasserstoffatmosphäre geschüttelt. Anschließend wurde der Katalysator abfiltriert und das Filtrat eingedampft. Man erhielt so die Titelverbindung als racemisches Gemisch der *cis*-Isomeren.
C₂₄H₄₀N₄O₅S (496.66)
analytische HPLC (Methode 3): Retentionszeit = 1.39 min

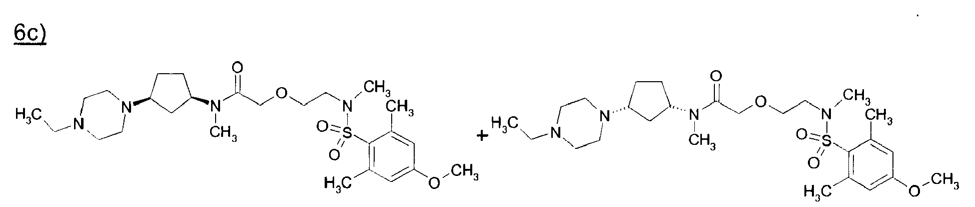

75 mg (0.15 mmol) Produkt 6b) wurden in 1 ml Acetonitril gelöst und mit 0.054 ml (0.39 mmol) TEA versetzt. Das Reaktionsgemisch wurde 15 min bei RT gerührt, dann 16.5 mg (0.15 mmol) Bromethan zugetropft und anschließend 24 h bei RT gerührt. Der Ansatz wurde über präparative HPLC gereinigt (Methode 8) gereinigt. Man erhielt so die Titelverbindung als racemisches Gemisch der *cis*-Isomeren.
C₂₆H₄₄N₄O₅S x C₂HF₃O₂ (638.74)
[M+H]⁺ = 525
analytische HPLC (Methode 3): Retentionszeit = 1.41 min

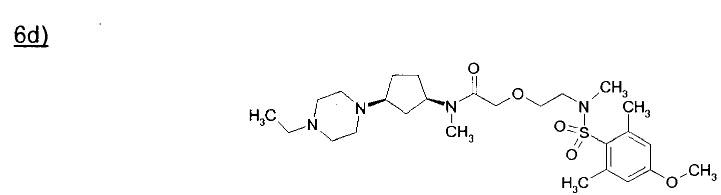

Produkt 6c) (racemisches Gemisch der *cis-*Isomeren) wurde nach HPLC-Methode 13 an chiraler Phase in die Enantiomere aufgetrennt.Man erhielt so die Titelverbindung als langsam eluierendes Enantiomer.
C₂₆H₄₄N₄O₅S x C₂HF₃O₂ (638.74)
[M+H]⁺ = 525
analytische HPLC (Methode 3): Retentionszeit = 17.92 min

### Beispiel 7

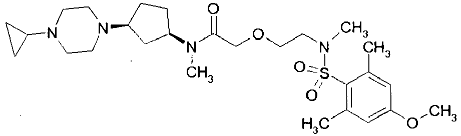

Die Titelverbindung kann analog Beispiel 5 hergestellt werden.

### Beispiel 8

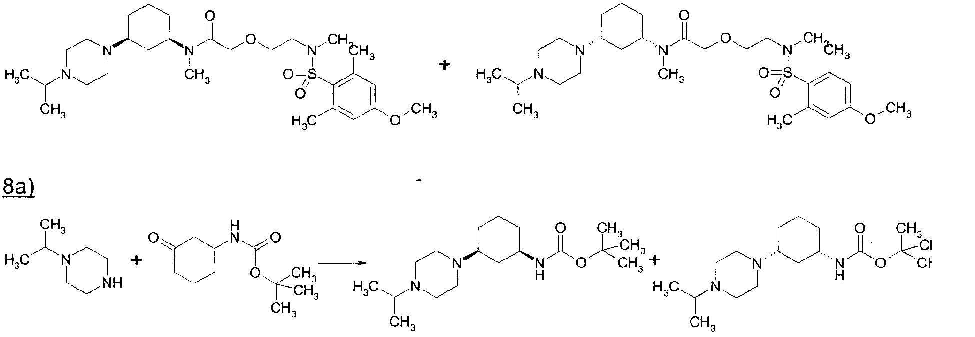

Analog zu Beispiel 1 e) wurde die Titelverbindung als Diastereomerengemisch aus 1-Isopropylpiperazin und tert-Butyl-3-oxo-cyclohexylcarbamat hergestellt. Mittels präparativer HPLC (Methode 11) wurden die *cis*-Isomeren als Racemat isoliert und gereinigt. C₁₈H₃₅N₃O₂ (325.49)
[M+H]⁺ = 326

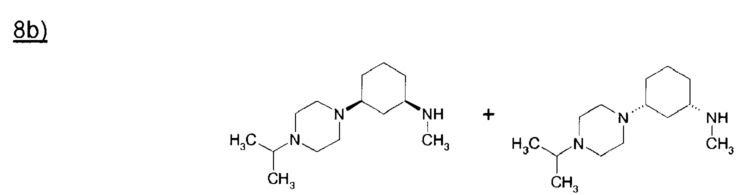

Analog Beispiel 3g) wurde die Titelverbindung aus Produkt 8a) hergestellt. C₁₄H₂₉N₃ (239.40)
[M+H]⁺ = 240

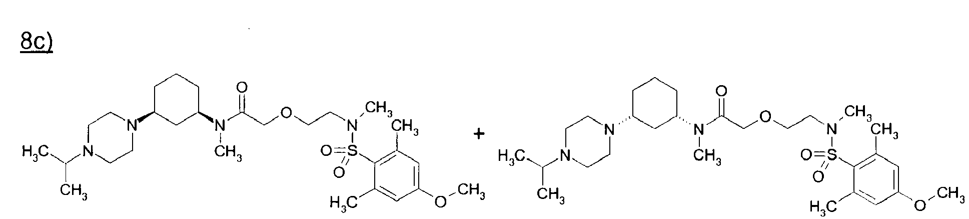

Analog Beispiel 1g) wurde die Titelverbindung als Gemisch der *cis*-Isomeren aus Produkt 8b) und Produkt 1d) hergestellt.
C₂₈H₄₈N₄O₅S x C₂HF₃O₂ (666.79)
[M+H]⁺ = 553
analytische HPLC (Methode 3): Retentionszeit = 1.44 min

### Beispiel 9

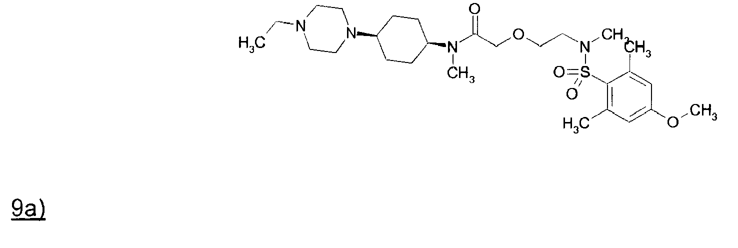

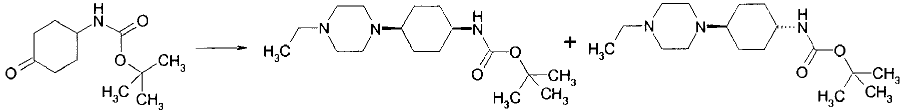

0.3 g (1.41 mmol) *N*-4-BOC-Aminocyclohexanon und 0.18 ml (1.41 mmol) N-Ethylpiperazin wurden in 5 ml THF vorgelegt und mit 0.08 ml (1.41 mmol) Eisessig versetzt. Nach ca. 30 Minuten wurden 0.6 g (2.81 mmol) Natriumtriacetoxyborhydrid zugegeben und über Nacht bei RT gerührt. Danach wurde der Ansatz mit Wasser verdünnt und eingeengt. Der Rückstand wurde mit Acetonitril verrieben und vom ausgefallenen Niederschlag abgesaugt. Das Filtrat wurde eingeengt.
C₁₇H₃₃N₃O₂ (311.46)
[M+H]⁺ = 312
analytische HPLC (Methode 3): Retentionszeit = 0.45 min

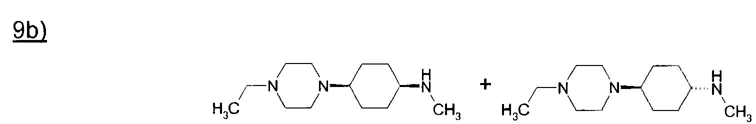

Analog Beispiel 3g) wurde die Titelverbindung als *cis*/*trans*-Isomerengemisch aus Produkt 9a) hergestellt.
C₁₃H₂₇N₃ (225.37)
[M+H]⁺ = 226

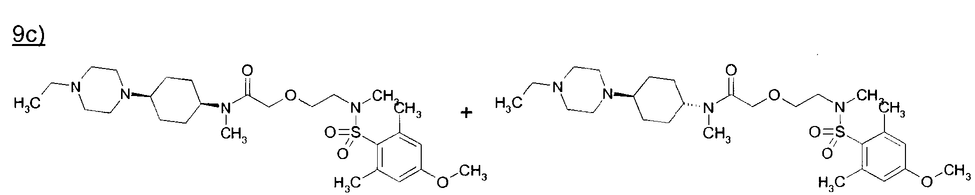

Analog Beispiel 1g) wurde die Titelverbindung aus Produkt 9b) und Produkt 1d) als *cis*/*trans*-Isomerengemisch hergestellt.

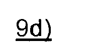

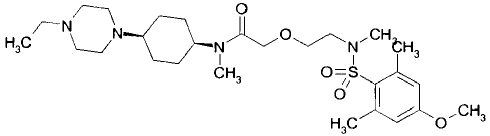

Aus dem Gemisch 9c) wurde die Titelverbindung mittels präparativer HPLC (Methode 9) abgetrennt.
C₂₇H₄₆N₄O₅S x C₂HF₃O₂ (652.77) *cis*-Produkt
analytische HPLC (Methode 3): Retentionszeit = 1.43 min

### Beispiel 10

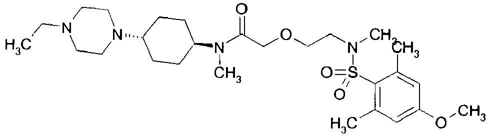

Aus dem Gemisch 9c) wurde die Titelverbindung mittels präparativer HPLC (Methode 9) abgetrennt.
C₂₇H₄₆N₄O₅S x C₂HF₃O₂ (652.77) *trans*-Produkt
analytische HPLC (Methode 3): Retentionszeit = 1.33 min

### Beispiel 11

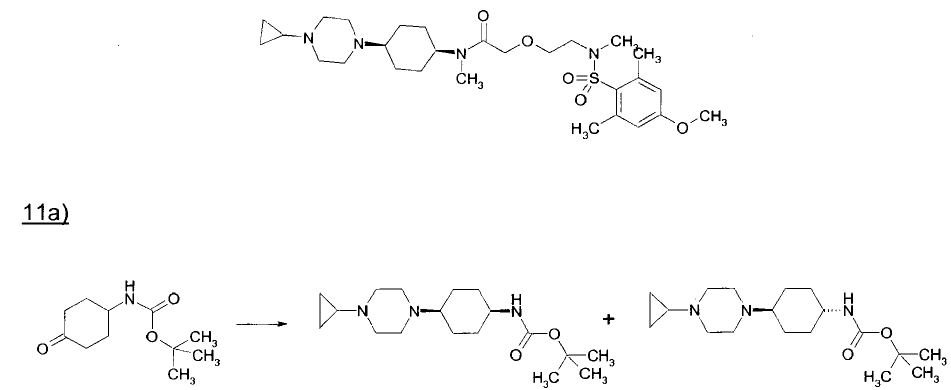

Analog Beispiel 9a) wurde die Titelverbindung aus *N*-4-BOC-Aminocyclohexanon und *N*-Cyclopropylpiperazin als *cis*/*trans*-Isomerengemisch hergestellt.
C₁₈H₃₃N₃O₂(323.47)
[M+H]⁺ = 324
analytische HPLC (Methode 3): Retentionszeit = 1.08 min

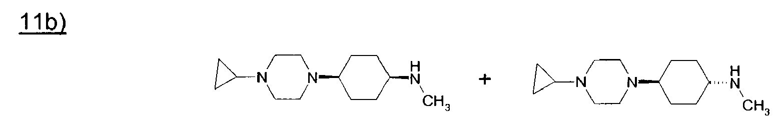

Analog Beispiel 3g) wurde die Titelverbindung aus Produkt 11a) als *cis*/*trans-*Isomerengemisch hergestellt.
C₁₄H₂₇N₃ (237.38)

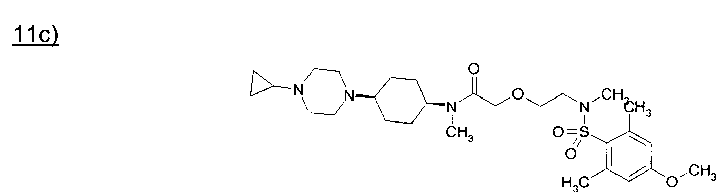

Analog Beispiel 1 g) wurde die Titelverbindung aus Produkt 11b) und Produkt 1 d) hergestellt. Das *cis*-Isomer wurde mittels HPLC (Methode 9) isoliert.
C₂₈H₄₆N₄O₅S (550.76)
[M+H]⁺ = 551
analytische HPLC (Methode 3): Retentionszeit = 1.57 min

### Beispiel 12

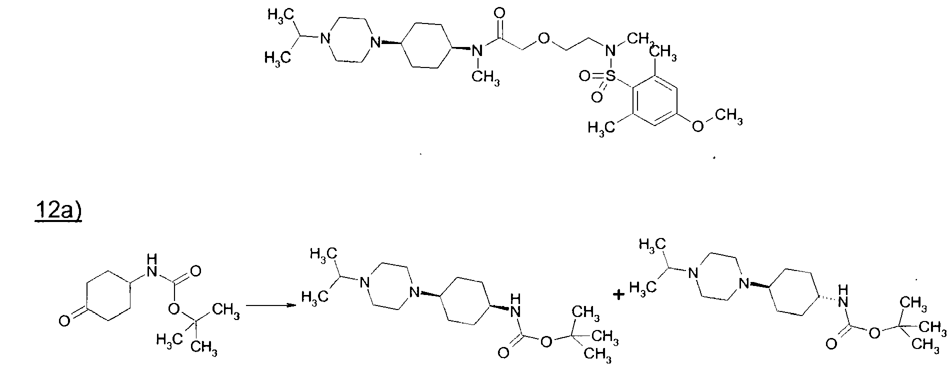

Analog Beispiel 9a) wurde die Titelverbindung aus *N*-4-BOC-Aminocyclohexanon und *N*-Isopropylpiperazin als *cis*/*trans*-Isomerengemisch hergestellt.
C₁₈H₃₅N₃O₂ (325.49)
[M+H]⁺ = 326

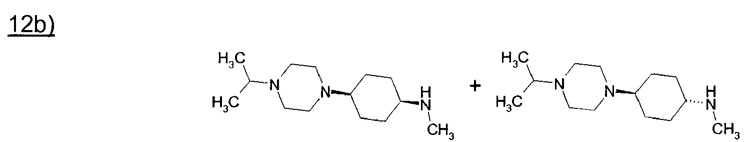

Analog Beispiel 3g) wurde die Titelverbindung aus Produkt 12a) als *cis*/*trans*-Isomerengemisch hergestellt.
C₁₄H₂₉N₃ (239.40)
[M+H]⁺ = 240

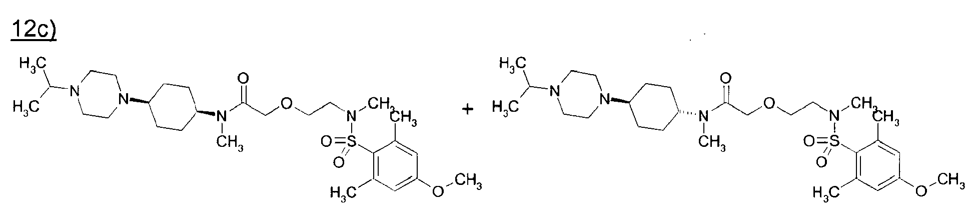

Analog Beispiel 1g) wurde die Titelverbindung aus Produkt 12b) und Produkt 1d) als *cis*/*trans*-Isomerengemisch hergestellt.

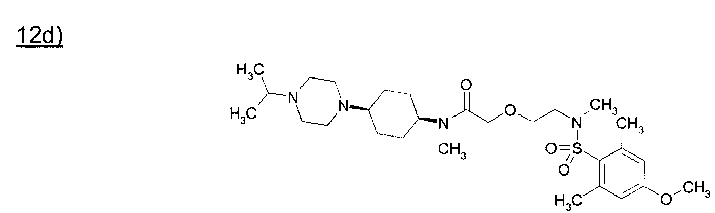

Aus dem Gemisch 12c) wurde die Titelverbindung mittels HPLC isoliert. C₂₈H₄₈N₄O₅S (55277) *cis*-Produkt
[M+H]⁺ = 553
analytische HPLC (Methode 5): Retentionszeit = 1.87 min

### Beispiel 13

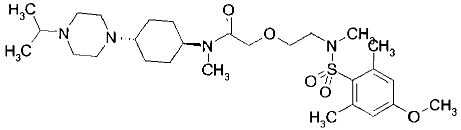

Aus dem Gemisch 12c) wurde die Titelverbindung mittels HPLC isoliert.
C₂₈H₄₈N₄O₅S (552.77) *trans*-Produkt
[M+H]⁺ = 553
analytische HPLC (Methode 5): Retentionszeit = 1.66 min

### Beispiel 14

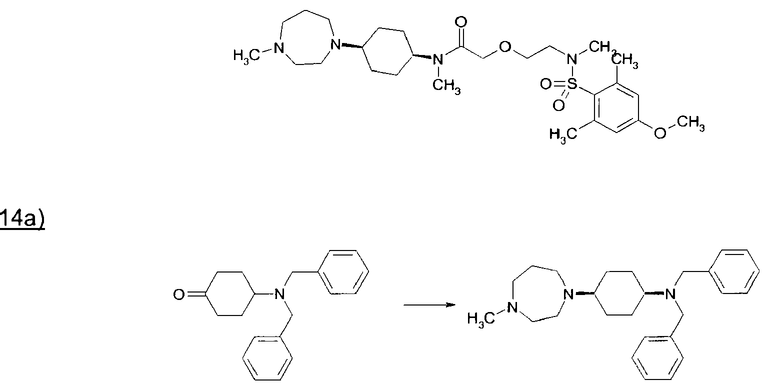

Zu 90 mg (2.38 mmol) Natriumborhydrid in ca. 3 ml THF wurden 1,1 ml (6.87 mmol) 2-Ethylenhexansäure langsam zugetropft und anschließend über Nacht bei RT gerührt. Die so hergestellte Hydridlösung wurde zu einer Lösung von 0.27 ml (2.19 mmol) N-Methylhomopiperazin und 642 mg (2.19 mmol) 4-Dibenzylamino-cyclohexanon in ca. 27 ml THF langsam zugetropft und 2 h bei RT gerührt. Danach wurde das Gemisch eingeengt und aus dem so erhaltenen Rohprodukt die Titelverbindung als *cis*-Isomer mittels HPLC (Methode 11) isoliert.
C₂₆H₃₇N₃ (391.59)
[M+H]⁺ = 392
analytische HPLC (Methode 3): Retentionszeit = 1.30 min

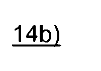

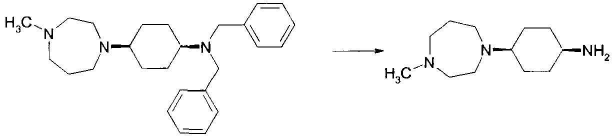

Analog Beispiel 3e) wurde die Titelverbindung aus Produkt 14a) hergestellt.
C₁₂H₂₅N₃ (211.35)
[M+H]⁺ = 212

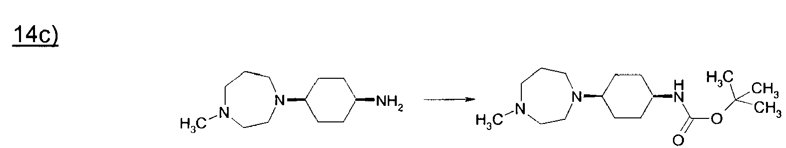

Eine Lösung von 135 mg (0.64 mmol) Produkt 14b), 160 mg (0.73 mmol) BOC-Anhydrid und 0.09 ml (0.65 mmol) TEA in 10 ml Methanol wurde 48 h bei RT gerührt. Dananch wurde das Gemisch eingeengt und das Produkt mittels HPLC isoliert.
C₁₇H₃₃N₃O₂ (311.46)
[M+H]⁺ = 312
analytische HPLC (Methode 4): Retentionszeit = 1.21 min

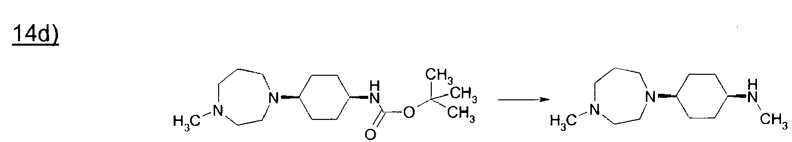

Zu 0.18 g (0.58 mmol) Produkt 14c) in 10 ml THF wurde 1 ml (2 mmol) 2M Lithiumaluminiumhydridlösung in THF langsam zugetropft und anschließend 3 h unter Rückfluß erhitzt. Danach wurde der Ansatz abgekühlt und langsam mit wenig Wasser versetzt. Der ausgefallene Rückstand wurde abgesaugt, mit Acetonitril gewaschen und das Filtrat im Vakuum eingedampft.
C₁₃H₂₇N₃ (225.37)
[M+H]⁺ = 226

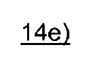

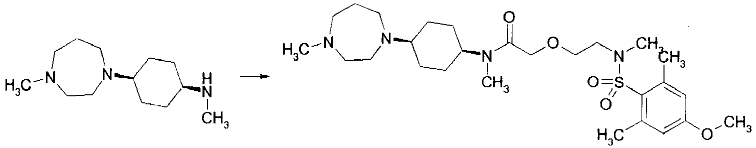

Analog Beispiel 1g) wurde die Titelverbindung aus Produkt 14d) und Produkt 1d) hergestellt.
C₂₇H₄₆N₄O₅S x C₂HF₃O₂ (652.77)
[M+H]⁺ = 539
analytische HPLC (Methode 3): Retentionszeit = 1.29 min

### Beispiel 15

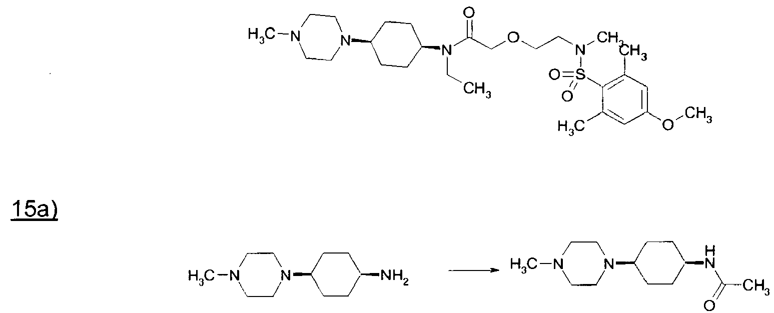

844 mg (1.57 mmol) Produkt 3e), 0.11 ml (1.57 mmol) Acetylchlorid und 1.36 ml (7.82 mmol) DIPEA wurden in 20 ml DCM gelöst und 2 h bei RT gerührt. Dann wurde das Lösungsmittel eingeengt und der Rückstand über präparative HPLC (Methode 10) gereinigt.
C₁₃H₂₅N₃O (239.36)
[M+H]⁺ = 240

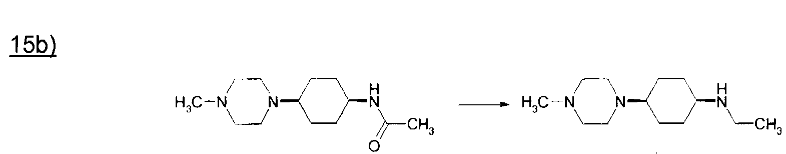

Zu 0.69 ml (1.4 mmol) einer 2M Lithiumaluminiumhydridlösung in THF wurden 124 mg (0.52 mmol) Produkt 15a), gelöst in 10 ml THF, langsam zugetropft. Der Ansatz wurde 4 h bei 75°C gerührt. Anschließend wurde die Lösung mit etwas Wasser versetzt, der ausgefallene Niederschlag abfiltriert und das Filtrat eingeengt.
C₁₃H₂₇N₃ (225.37)
[M+H]⁺ = 226

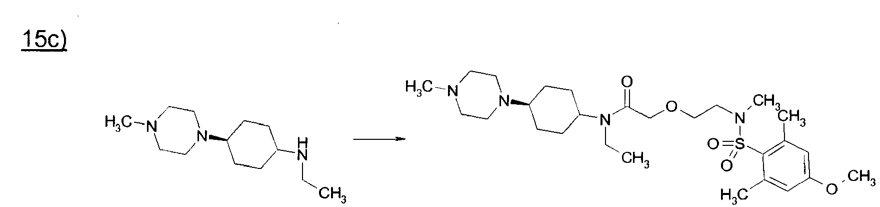

Analog Beispiel 1g) wurde die Titelverbindung aus Produkt 1d) und Produkt 15g) hergestellt.
C₂₇H₄₆N₄O₅S (538.74)
[M+H]⁺ = 539
analytische HPLC (Methode 3): Retentionszeit = 1.47 min

### Beispiel 16

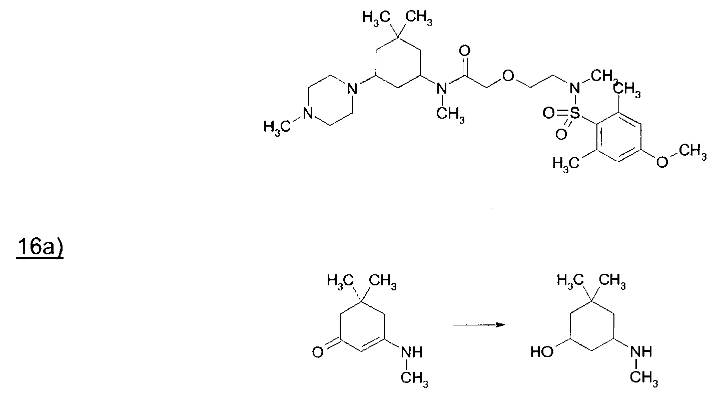

1 g (6.53 mmol) 5,5-Dimethyl-3-(Methylamino)-2-cyclohexen-1-on, gelöst in 15 ml EtOH, wurden mit 100 mg Raney-Nickel versetzt und 24 h bei RT hydriert. Dann wurde der Ansatz auf 50°C erwärmt und weitere 24 h hydriert. Danach wurde Palladium/Kohle zugesetzt und weitere 24 h bei RT hydriert. Zuletzt wurden 3 ml 6 M NaOH zugefügt und wieder 24 h hydriert. Anschließend wurde der Katalysator abgesaugt und das Filtrat eingeengt. Das so erhaltene Rohprodukt wurde ohne Reinigung weiter umgesetzt. C₉H₁₇NO (155.24)
analytische HPLC (Methode 3): Retentionszeit = 0.61 min

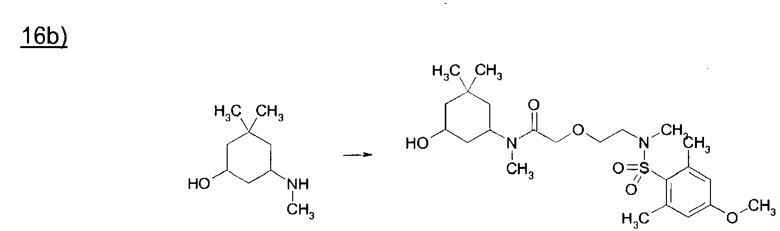

Analog Beispiel 1g) wurde die Titelverbindung aus Produkt 16a) und Produkt 1d) hergestellt.
C₂₃H₃₈N₂O₆S (470.62)
[M+H]⁺ = 471

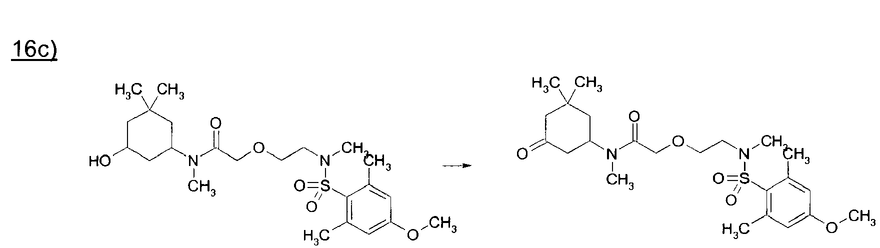

Zu einer Lösung von 42 mg (0.09 mmol) Produkt 16b) in 5 ml Acetonitril wurden 42 mg (0.1 mmol) Dess-Martin-Periodinan zugesetzt und bei RT gerührt. Nach beendeter Oxidation wurde der Ansatz mit etwas Wasser versetzt und eingeengt. Der Rückstand wurde mit DCM verrieben und vom ausgefallenen Feststoff abgesaugt. Das Filtrat wurde eingeengt.
C₂₃H₃₆N₂O₆S (468.61)
analytische HPLC (Methode 3): Retentionszeit = 2.22 min

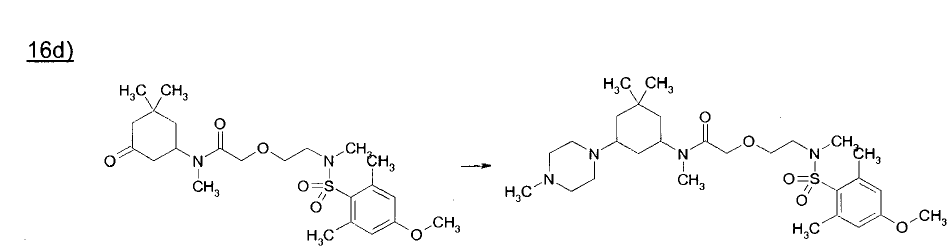

Zu einer Lösung von 43 mg (0.092 mmol) Produkt 16c) und 10.2 µl (0.092 mmol) 1-Methylpiperazin in 5 ml wasserfreiem THF wurden 5.25 µl (0.092 mmol) Essigsäure gegeben und 30 min bei RT gerührt. Danach wurden 58.34 mg (0.28 mmol) Natriumtriacetoxyborhydrid zugefügt und 24 h bei RT weitergerührt. Anschließend wurden innerhalb einer Woche mehrmals einige Tropfen 1-Methylpiperazin, Essigsäure, Natriumtriacetoxyborhydrid und nach 4 Tagen Molekularsieb zugefügt. Danach wurden das Molekularsieb und der ausgefallene Feststoff abgesaugt, mit Acetonitril gewaschen und das Filtrat eingeengt. Der Rückstand wurde mit DCM und Natriumhydrogencarbonatlösung ausgeschüttelt. Die organische Phase wurde abgetrennt, mit Magnesiumsulfat getrocknet und einrotiert. Der Rückstand wurde über präparative HPLC (Methode 9) gereinigt. C₂₈H₄₈N₄O₅S (552.77)
[M+H]⁺ = 553
analytische HPLC (Methode 3): Retentionszeit = 1.76 min

### Beispiel 17

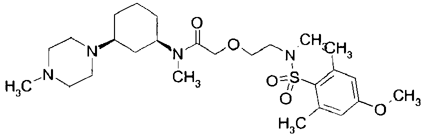

Produkt 1g) (racemisches Gemisch der *cis-*Isomeren) wurde nach HPLC-Methode 13 an chiraler Phase in die Enantiomere aufgetrennt. Man erhielt so die Titelverbindung als das schnell eluierende Enantiomer.
C₂₆H₄₄N₄O₅S (524.72
[M+H]⁺ = 525
HPLC (Methode 13): Retentionszeit = 7.3 min

### Beispiel 18

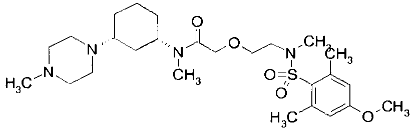

Produkt 1g) (racemisches Gemisch der *cis-*Isomeren) wurde nach HPLC-Methode 13 an chiraler Phase in die Enantiomere aufgetrennt. Man erhielt so die Titelverbindung als das langsam eluierende Enantiomer.
C₂₆H₄₄N₄O₅S (524.72
[M+H]⁺ = 525
HPLC (Methode 13): Retentionszeit = 9.7 min

### Beispiel 19

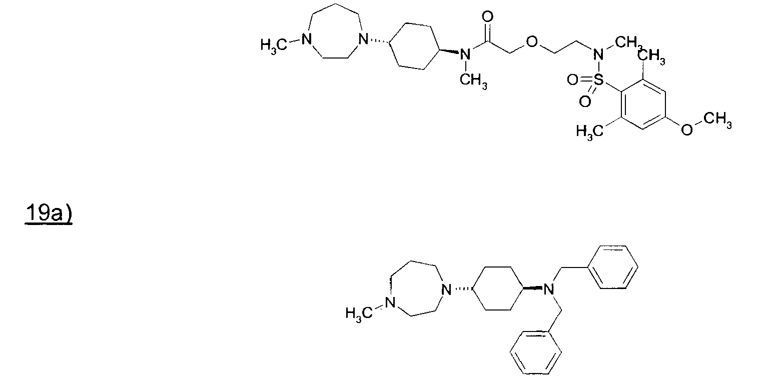

Aus dem Rohprodukt 14a) wurde das *trans*-Isomer chromatographisch isoliert (präparative HPLC-Methode 11).
C₂₆H₃₇N₃ (391.59) *trans*-Verbindung
[M+H]⁺ = 392

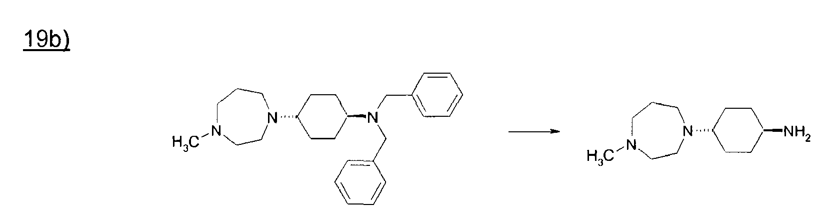

Analog Beispiel 3e) wurde die Titelverbindung aus Produkt 19a) hergestellt. C₁₂H₂₅N₃ x 3 HCl (320.73)
[M+H]⁺ = 212

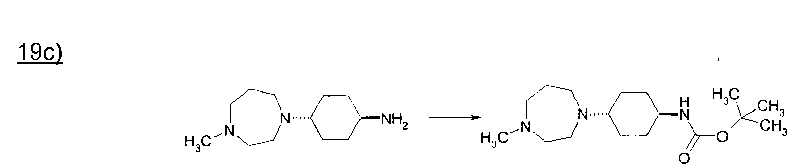

Analog Beispiel 14c) wurde die Titelverbindung aus Produkt 19b) hergestellt. C₁₇H₃₃N₃O₂ x 3 HCl (420.85)
[M+H]⁺ = 312

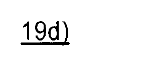

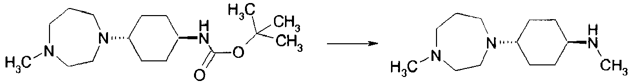

Analog Beispiel 14d) wurde die Titelverbindung aus Produkt 19c) hergestellt. C₁₇H₃₃N₃O₂ x 3 HCl (420.85)
C₁₃H₂₇N₃ (225.37)
analytische HPLC (Methode 3): Retentionszeit = 0.304 min

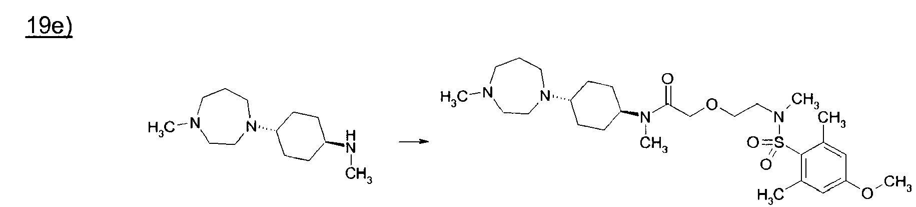

Analog Beispiel 1g) wurde die Titelverbindung aus Produkt 19d) und Produkt 1d) hergestellt.
C₂₇H₄₆N₄O₅S x C₂HF₃O₂ (652.77)
[M+H]⁺ = 539
HPLC (Methode 3): Retentionszeit = 1.30 min

### Beispiel 20

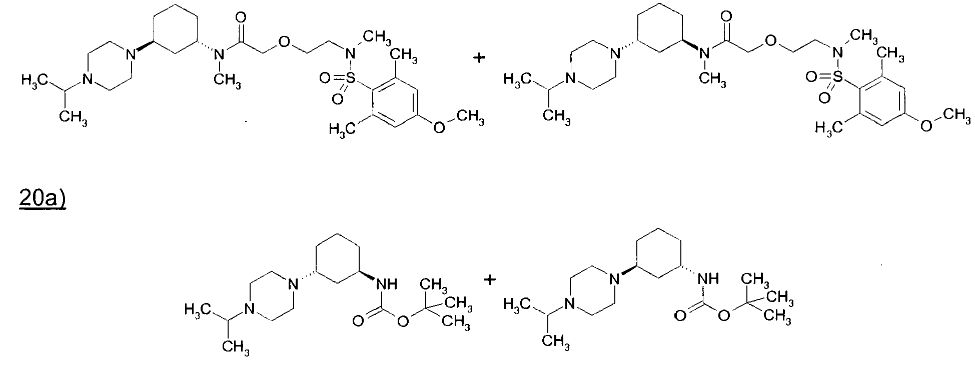

Aus dem Rohprodukt 8a) wurden mittels präparativer HPLC (Methode 11) die *trans-*Isomeren als racemisches Gemisch isoliert.
C₁₈H₃₅N₃O₂ (325.49) *trans*-Verbindung
[M+H]⁺ = 326

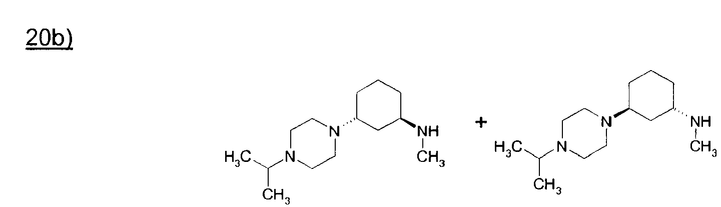

Analog Beispiel 3g) wurde die Titelverbindung als Gemisch der *trans-*Isomeren aus Produkt 20a) hergestellt.
C₁₄H₂₉N₃ (239.40)
[M+H]⁺ = 240

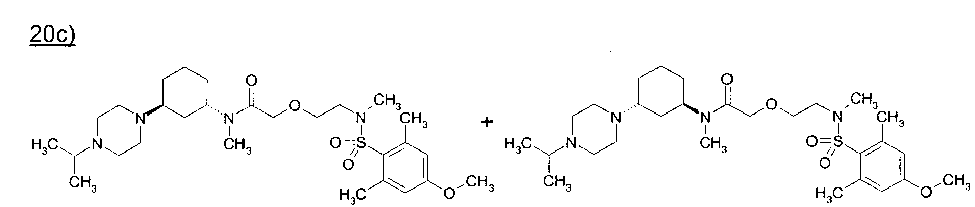

Analog Beispiel 1g) wurde die Titelverbindung aus Produkt 20b) und Produkt 1d) hergestellt.
C₂₈H₄₈N₄O₅S x 2C₂HF₃O₂ (780.82)
[M+H]⁺ = 553
analytische HPLC (Methode 3): Retentionszeit = 1.53 min

### Beispiel 21

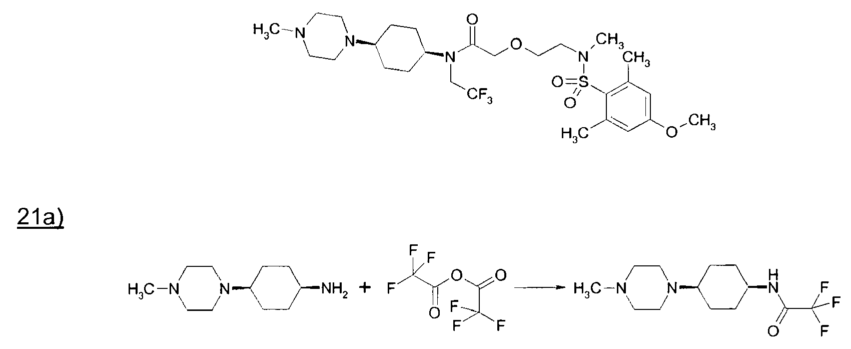

Analog Beispiel 15a) wurde die Titelverbindung aus Produkt 3e) und Trifluoressigsäureanhydrid hergestellt.
C₁₃H₂₂F₃N₃O (293.33)
[M+H]⁺ = 294
analytische HPLC (Methode 5): Retentionszeit = 1.16 min

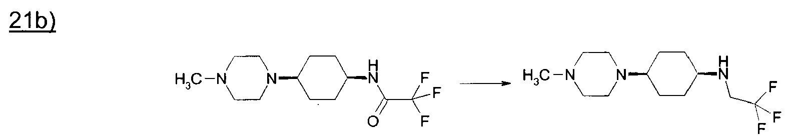

Analog Beispiel 15b) wurde die Titelverbindung aus Produkt 21 a) hergestellt.
C₁₃H₂₄ F₃N₃ (279.35)
[M+H]⁺ = 280
analytische HPLC (Methode 5): Retentionszeit = 1.36 min

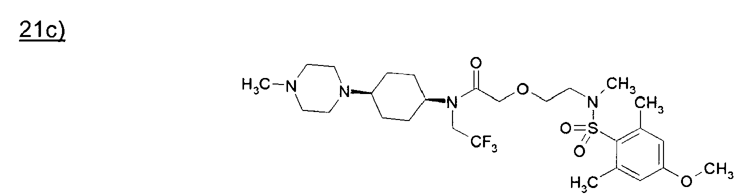

Analog Beispiel 15c) wurde die Titelverbindung aus Produkt 21b) und Produkt 1d) hergestellt und mittels HPLC (Methode 9) gereinigt.
C₂₇H₄₃F₃N₄O₅S x C₂HF₃O₂ (706.74)
[M+H]⁺ = 593

### Beispiel 22

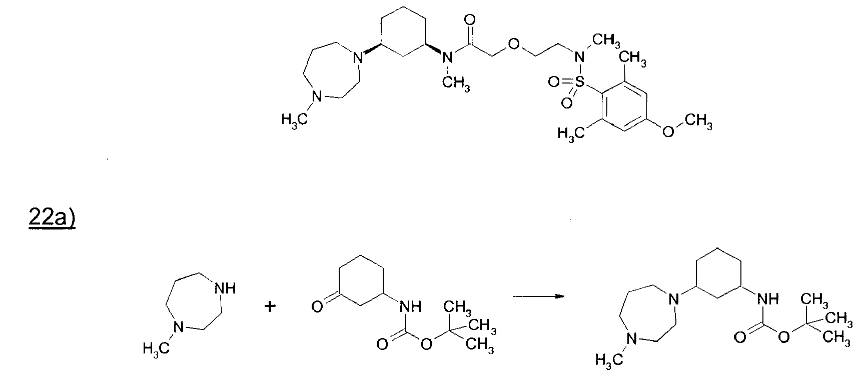

Analog Beispiel 1e) wurde die Titelverbindung aus N-Methyl-homopiperazin und (3-Oxo-cyclohexyl)-carbaminsäure-tert-butylester als Diastereomerengemisch hergestellt. C₁₇H₃₃N₃O₂ (311.46)
analytische HPLC (Methode 3): Retentionszeit = 0.42 min

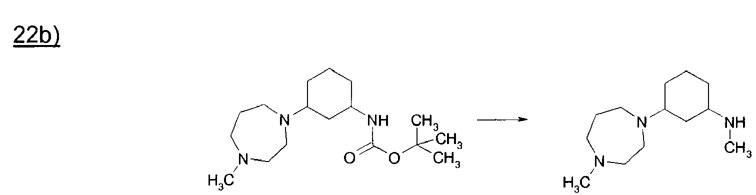

Analog Beispiel 1f) wurde die Titelverbindung aus Produkt 22a) als Diastereomerengemisch hergestellt.
C₁₃H₂₇N₃ (225.37)
analytische HPLC (Methode 3): Retentionszeit = 0.26 min

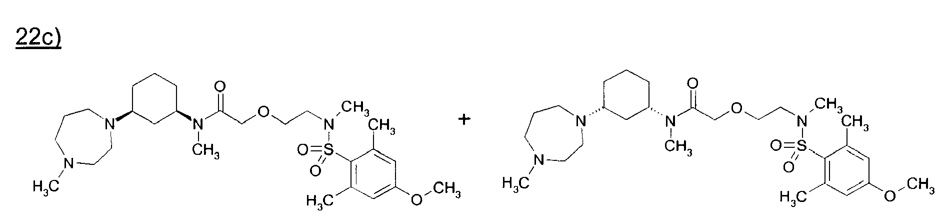

Analog Beispiel 1g) wurde die Titelverbindung als racemisches Gemisch der *cis*-Isomeren hergestellt.
C₂₇H₄₆N₄O₅S (538.74)
[M+H]⁺ = 539

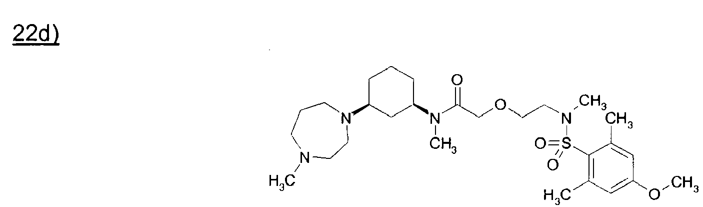

Das racemische Gemisch aus Beispiel 22c) wurde nach HPLC-Methode 14 an chiraler Phase in die Enatiomere aufgetrennt. Man erhielt so die Titelverbindung als schnell eluierendes Enantiomer.
C₂₇H₄₆N₄O₅S (538.74)
[M+H]⁺ = 539
HPLC (Methode 14): Retentionszeit = 1.31 min

### Beispiel 23

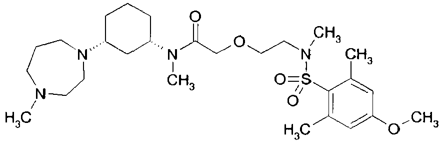

Das racemische Gemisch aus Beispiel 22c) wurde nach HPLC-Methode 14 an chiraler Phase in die Enatiomere aufgetrennt. Man erhielt so die Titelverbindung als langsam eluierendes Enantiomer.
C₂₇H₄₆N₄O₅S (538.74)
[M+H]⁺ = 539
HPLC (Methode 14): Retentionszeit = 1.57 min

### Beispiel 24

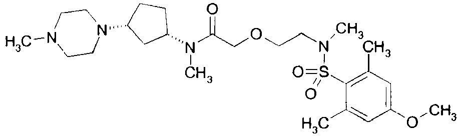

Das racemische Gemisch der *cis*-Isomeren aus Beispiel 5c) wurde nach HPLC-Methode 13 an chiraler Phase in die Enatiomere aufgetrennt. Man erhielt so die Titelverbindung als langsam eluierendes Enantiomer.
C₂₅H₄₂N₄O₅S (510.69)
[M+H]⁺ = 511
HPLC (Methode 13): Retentionszeit = 12.37 min

### Beispiel 25

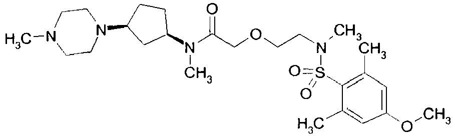

Das racemische Gemisch der *cis*-Isomeren aus Beispiel 5c) wurde nach HPLC-Methode 13 an chiraler Phase in die Enatiomere aufgetrennt. Man erhielt so die Titelverbindung als schnell eluierendes Enantiomer.
C₂₅H₄₂N₄O₅S (510.69)
[M+H]⁺ = 511
HPLC (Methode 13): Retentionszeit = 6.96 min

### Beispiel 26

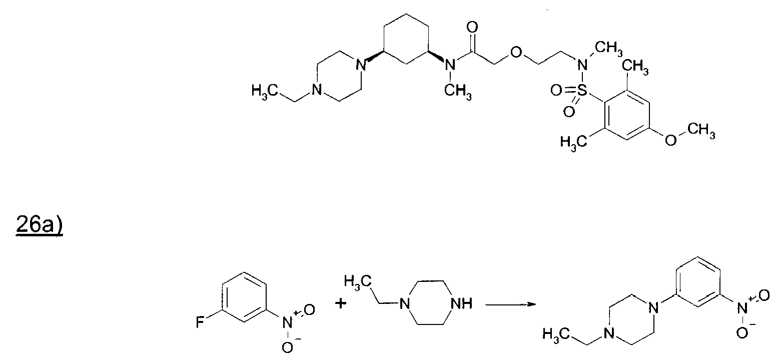

1.0 ml (9.4 mmol) 3-Fluornitrobenzol, 2 g (14.1 mmol) Kaliumcarbonat und 1.2 ml (9.4 mmol) N-Ethylpiperazin in 20 ml DMF wurden 48 h bei 185°C unter Rückfluß gekocht. Danach wurden nochmals 1.2 ml (9.4 mmol) *N*-Ethylpiperazin zugefügt und weitere 30 h unter Rückfluß erhitzt. Anschließend wurde das Carbonat abfiltriert und das Filtrat eingeengt. Der Rückstand wurde über präparative HPLC (Methode 10) gereinigt. C₁₂H₁₇N₃O₂ (235.28)
[M+H]⁺ = 236
analytische HPLC (Methode 3): Retentionszeit = 0.77 min

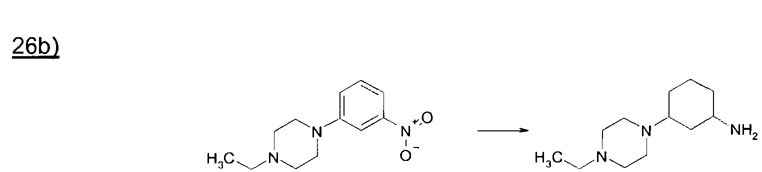

550 mg (2.34 mmol) Produkt 26a) und 300 mg Nishimura-Katalysator (Rh/Pt) wurden in 25 ml MeOH suspendiert und 24 h bei RT und 5 bar in Wasserstoffatmosphäre geschüttelt. Danach wurde der Katalysator abfiltriert und das Filtrat eingeengt. Man erhielt so die Titelverbindung als Diastereomerengemisch.
C₁₂H₂₅N₃ (211.35)
[M+H]⁺ = 212
analytische HPLC (Methode 5): Retentionszeit = 1.19 min

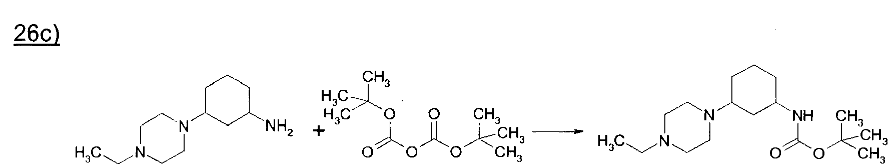

Zu einer Lösung von 550 mg (2.34 mmol) Produkt 26a) in 15 ml DCM wurden 613 mg (2.81 mmol) BOC-Anhydrid und 0.71 ml (5.15 mmol) TEA zugefügt und 48 h bei RT gerührt. Danach wurde der entstandene Niederschlag abgesaugt und mit DCM gewaschen. Das Filtrat wurde eingeengt und über präparative HPLC (Methode 10) gereinigt. Beide Produkte wurden vereinigt.Man erhielt so die Titelverbindung als Diastereomerengemisch.
C₁₇H₃₃N₃O₂ (311.46)
[M+H]⁺ = 312
analytische HPLC (Methode 5): Retentionszeit = 1.50 min

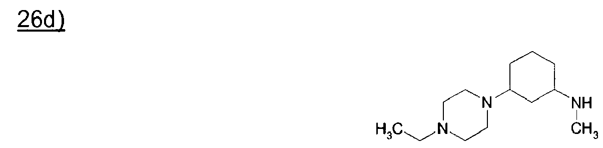

Analog Beispiel 14d) wurde die Titelverbindung aus 26c) als Diastereomerengemisch hergestellt.
C₁₃H₂₇N₃ (225.37)
[M+H]⁺ = 225

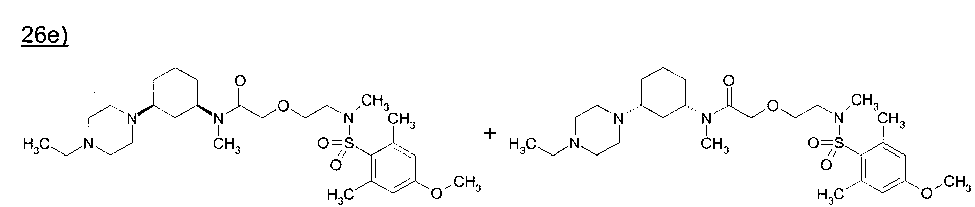

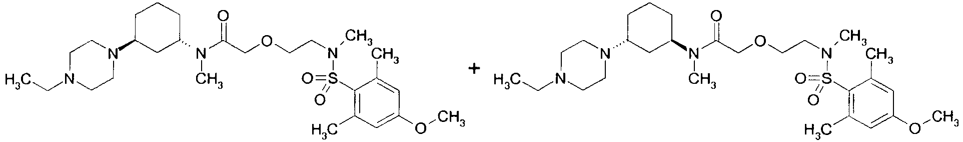

Analog Beispiel 1g) wurde die Titelverbindung aus Produkt 1d) und Produkt 26d) als Diastereomerengemisch hergestellt. Die Reinigung und die Abtrennung der *cis*-Isomeren von den *trans*-Isomeren erfolgte über präparative HPLC (Methode 10).
C₁₄H₂₁NO₆S (538.74)
[M+H]⁺ = 539
analytische HPLC (Methode 5): Retentionszeit = 1.56 min (*cis*-Diastereomer)
analytische HPLC (Methode 5): Retentionszeit = 1.75 min (trans-Diastereomer)

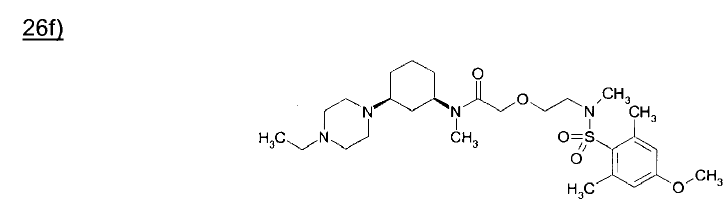

Das Gemisch der *cis*-Isomeren aus Produkt 26e) wurde nach HPLC-Methode 13 an chiraler Phase in die Enantiomere aufgetrennt. Man erhielt so die Titelverbindung als das schnell eluierende Enantiomer.
C₁₄H₂₁NO₆S (538.74)
[M+H]⁺ = 539
HPLC (Methode 13): Retentionszeit = 26.85 min

### Beispiel 27

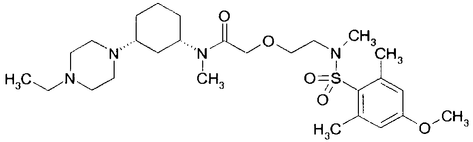

Das Gemisch der *cis*-Isomeren aus Produkt 26e) wurde nach HPLC-Methode 13 an chiraler Phase in die Enantiomere aufgetrennt. Man erhielt so die Titelverbindung als das langsam eluierende Enantiomer.
C₁₄H₂₁NO₆S (538.74)
[M+H]⁺ = 539
HPLC (Methode 13): Retentionszeit = 31.90 min

### Beispiel 28

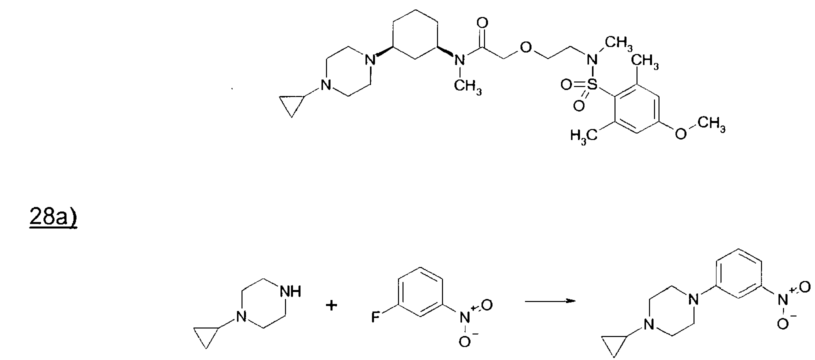

Die Titelverbindung wurde analog Beispiel 26a) hergestellt und über präparative HPLC (Methode 9) gereinigt.
C₁₃H₁₇N₃O₂ x C₂HF₃O₂ (361.32)
[M+H]⁺ = 248
analytische HPLC (Methode 3): Retentionszeit = 0.99 min

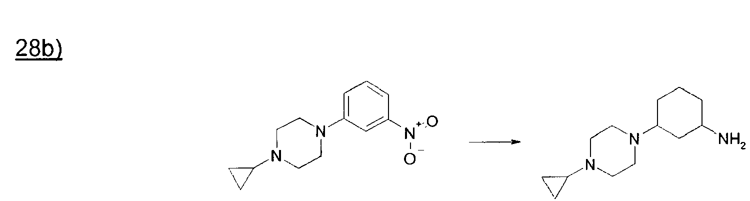

Analog Beispiel 26b) wurde die Titelverbindung als Diastereomerengemisch aus Produkt 28a) hergestellt.
C₁₃H₂₅N₃ (223.36)
[M+H]⁺ = 224
analytische HPLC (Methode 5): Retentionszeit = 1.30 min

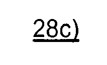

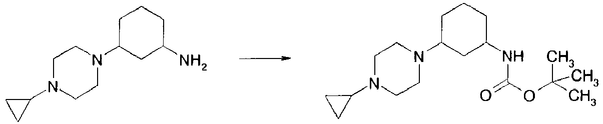

Analog Beispiel 26c) wurde die Titelverbindung als Diastereomerengemisch aus Produkt 28b) hergestellt.
C₁₈H₃₃N₃O₂ (323.47)
analytische HPLC (Methode 2): Retentionszeit = 1.58 min

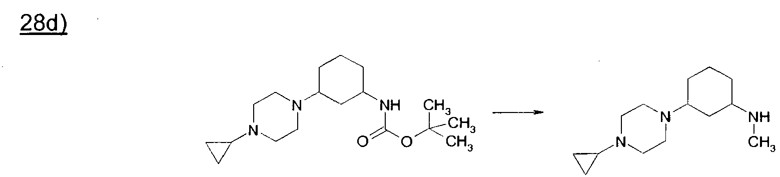

Analog Beispiel 14d) wurde die Titelverbindung als Diastereomerengemisch aus Produkt 28c) hergestellt.
C₁₄H₂₇N₃ (237.38)
[M+H]⁺ = 238
analytische HPLC (Methode 5): Retentionszeit = 1.58 min

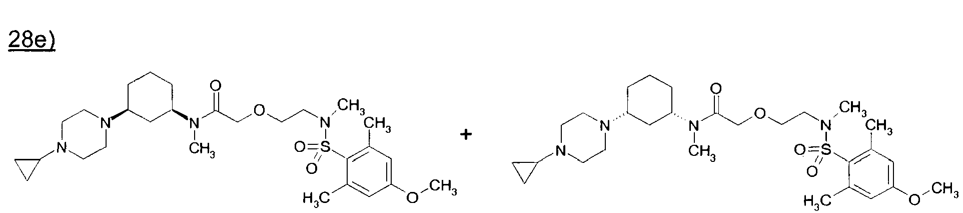

Analog Beispiel 12c) wurde die Titelverbindung aus Produkt 1d) und Produkt 28d) hergestellt. Nach chromatographischer Reinigung erhielt man das Produkt als racemisches Gemisch der *cis*-Isomeren.
C₂₈H₄₆N₄O₅S (550.76)
[M+H]⁺ = 551
analytische HPLC (Methode 5): Retentionszeit = 1.65 min

### Beispiel 29

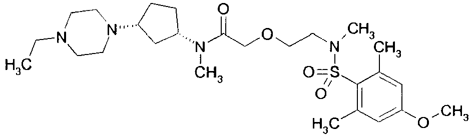

Produkt 6c) (racemisches Gemisch der *cis-*Isomeren) wurde nach HPLC-Methode 13 an chiraler Phase in die Enatiomere aufgetrennt. Man erhielt so die Titelverbindung als schnell eluierendes Enantiomer.
C₂₆H₄₄N₄O₅S x C₂HF₃O₂ (638.74)
[M+H]⁺ = 525
HPLC (Methode 13): Retentionszeit = 14.83 min

### Beispiel 30

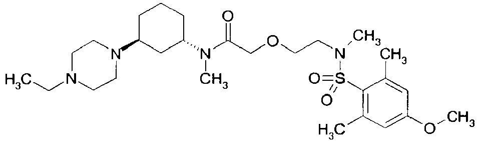

### Beispiel 31

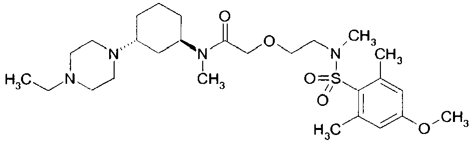

Das Diastereomerengemisch aus Beispiel 26e) wurde mittels präparativer HPLC (Methode 10) in die Diastereomerenpaare getrennt. Man erhielt so die Titelverbindungen 30) und 31) als racemisches Gemisch der trans-Isomeren.
C₁₄H₂₁NO₆S (538.74)
[M+H]⁺ = 539
analytische HPLC (Methode 5): Retentionszeit = 1.75 min

### Beispiel 32

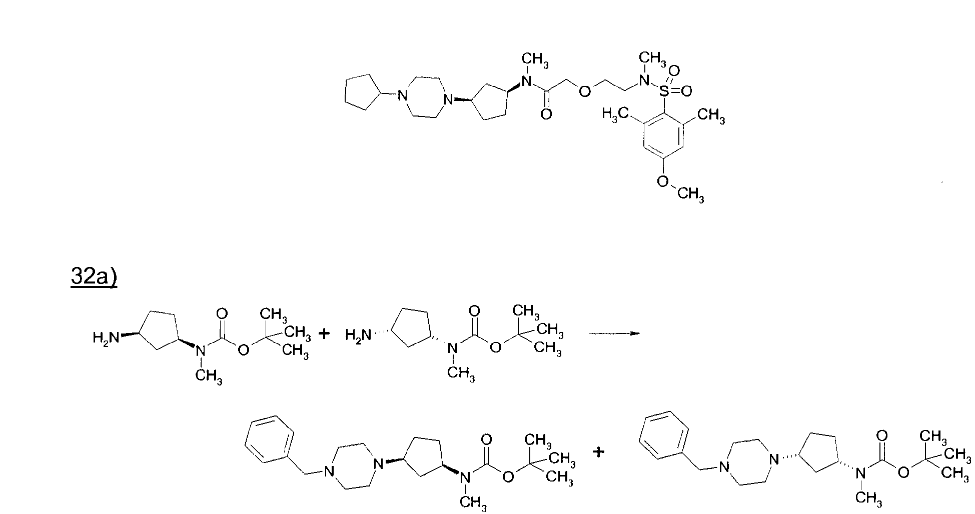

Zu einer Suspension aus 1.5 g (6.34 mmol) (3-Amino-cyclopentyl)-carbaminsäure-*tert-*butylester Hydrochlorid, 4.38 g (31.68 mmol) Kaliumcarbonat und 0.11 g (0.63 mmol) Kaliumjodid in 36 ml Acetonitril wurden 1.5 g (6.34 mmol) N,N-Bis(2-chlorethyl)benzylamin hinzugefügt und 4 h unter Rückfluß gekocht. Dann wurde das abgekühlte Reaktionsgemisch mit DCM verdünnt und mit Wasser gewaschen. Die organische Phase wurde mit Natriumsulfat getrocknet und eingeengt. Man erhielt das racemische Gemisch der *cis-*Isomeren.
C₂₁H₃₃N₃O₂ (359.51)
[M+H]⁺ = 360

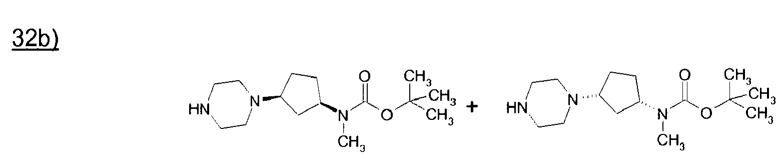

1.8 g (5.01 mmol) Produkt 32a) und 0.2 g Palladium/Kohle in 20 ml MeOH wurden zunächst 48 h bei 50°C und 50 psi unter Wasserstoffatmosphäre hydriert. Danach wurden der Ansatz weitere 48 h bei 70°C und 50 psi hydriert. Anschließend wurde der Katalysator abgesaugt und das Lösungsmittel abgedampft. Man erhielt die Titelverbindung als racemisches Genisch der *cis*-Isomeren.
C₁₄H₂₇N₃O₂ (269.38)
[M+H]⁺ = 270

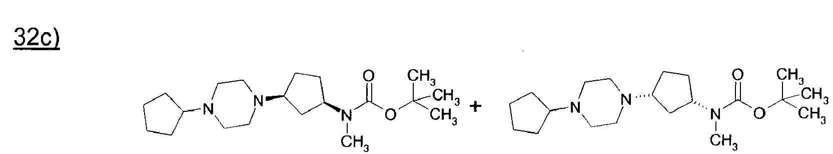

450 mg (1.67 mmol) Produkt 32a), 140.5 mg (1.67 mmol) Cyclopentanon und 0.18 ml (3.34 mmol) Essigsäure wurden in 8 ml THF gelöst und im Eisbad gekühlt. Danach wurden portionsweise 531 mg (2.51 mmol) Natriumtriacetoxyborhydrid zugegeben und 2 h bei RT gerührt. Die Suspension wurde filtriert und eingeengt. Man erhielt die Titelverbindung als racemisches Genisch der *cis*-Isomeren.
C₁₉H₃₅N₃O₂ (337.5)
[M+H]⁺ = 338

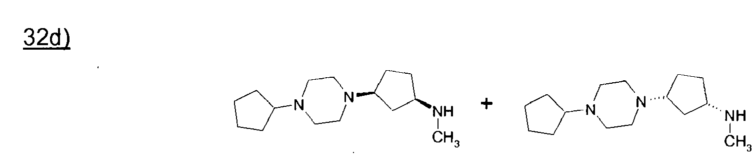

Analog Beispiel 14d) wurde dieTitelverbindung aus Produkt 32c) hergestellt. Man erhielt die Titelverbindung als racemisches Genisch der *cis*-Isomeren.
C₁₅H₂₉N₃ (251.41)
[M+H]⁺ = 252
analytische HPLC (Methode 3): Retentionszeit = 0.30 min

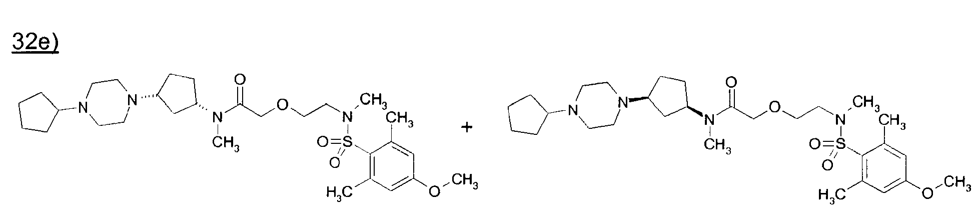

Analog Beispiel 1g) wurde die Titelverbindung als racemisches Gemisch der *cis*-Isomeren aus Produkt 1d) und Produkt 32d) hergestellt.
C₂₉H₄₈N₄O₅S x C₂HF₃O₂ (678.81)
[M+H]⁺ = 565 analytische HPLC (Methode 2): Retentionszeit = 1.53 min

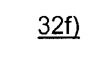

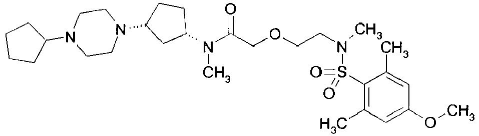

Das racemische Gemisch aus Beispiel 32e) wurde nach HPLC-Methode 14 an chiraler Phase in die *cis-*Enantiomere aufgetrennt. Man erhielt so die Titelverbindung als das schnell eluierende Enantiomer.
C₂₉H₄₈N₄O₅S (564.78)
[M+H]⁺ = 565
HPLC (Methode 14): Retentionszeit = 1.66 min (schnell eluierendes Enantiomer)

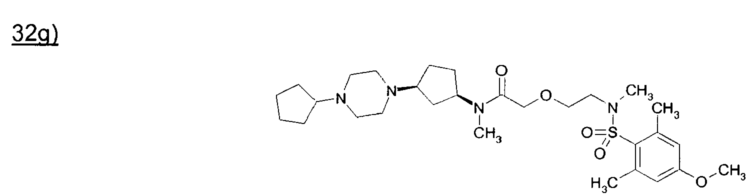

Das racemische Gemisch aus Beispiel 32e) wurde nach HPLC-Methode 14 an chiraler Phase in die *cis*-Enantiomere aufgetrennt. Man erhielt so die Titelverbindung als das langsam eluierende Enantiomer.
C₂₉H₄₈N₄O₅S (564.78)
[M+H]⁺ = 565
HPLC (Methode 14): Retentionszeit = 1.77 min (langsam eluierendes Enantiomer)

### Beispiel 33

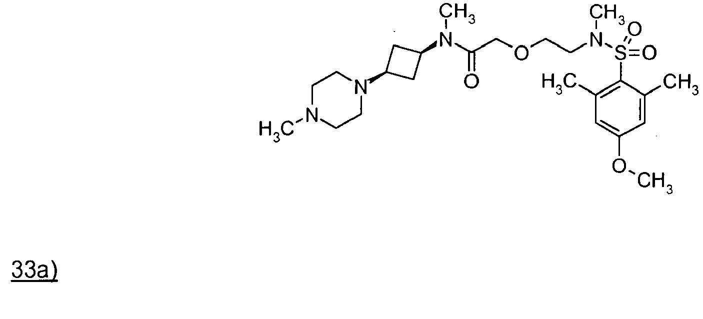

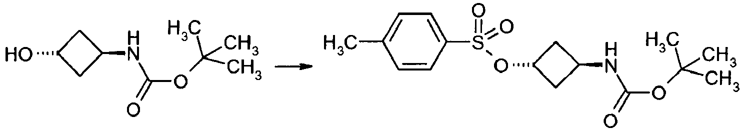

Zu einer Lösung aus 440 mg (2.35 mmol) *trans*-*tert*-Butyl-3-hydroxycyclobutylcarbamat und 0.74 ml (9.4 mmol) wasserfreiem Pyridin in 7 ml DCM wurden unter Eiskühlung und Stickstoffatmosphäre 498 mg (2.59 mmol) *p*-Toluolsulfonsäurechlorid zugefügt und bei RT gerührt. Nach einigen Stunden wurde noch etwas DMAP zugefügt und weitere 24 h bei RT gerührt. Das Reaktionsgemisch wurde mit EE verdünnt, einmal mit 20%iger Zitronensäurelösung ausgeschüttelt, fünfmal mit Wasser und einmal mit gesättigter Natriumchloridlösung gewaschen. Die organische Phase wurde über Magnesiumsulfat getrocknet und das Filtrat eingeengt.
C₁₆H₂₃NO₅S (341.42)
[M+NH₄]⁺ = 359
analytische HPLC (Methode 3): Retentionszeit = 2.50 min

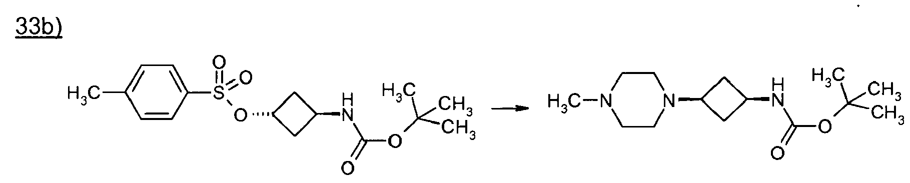

800 mg (2.34 mmol) Produkt 33a) wurden in 2 ml (18.03 mmol) 1-Methylpiperazin gelöst und mit 20 mg (0.16 mmol) DMAP versetzt. Dann wurde das Reaktionsgemisch über Nacht im Mikrowellengerät auf 100°C erhitzt. Anschließend wurde das Lösungsmittel abgedampft und der Rückstand über präparative HPLC (Methode 11) gereinigt.
C₁₄H₂₇N₃O₂ (269.38)
[M+H]⁺ = 270

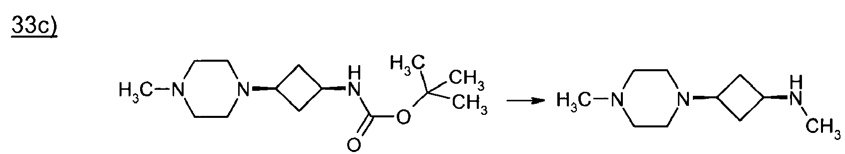

Zu 500 mg (1.3 mmol) Produkt 33b) in 12 ml wasserfreiem THF wurden 3.9 ml (7.8 mmol) 2M Lithiumaluminiumhydridlösung in THF langsam zugetropft und anschließend 4 h unter Rückfluß gerührt. Danach wurde der Ansatz abgekühlt und langsam mit wenig Wasser (3 bis 4 ml) versetzt. Der ausgefallene Feststoff wurde abgesaugt, mit Acetonitril gewaschen und das Filtrat eingedampft. Der Rückstand wurde mit 2 ml 2M etherischer Salzsäure verrieben und das Lösungsmittel abgedampft.
C₁₀H₂₁N₃ x 3 HCl (292.68)
analytische HPLC (Methode 4): Retentionszeit = 096 min

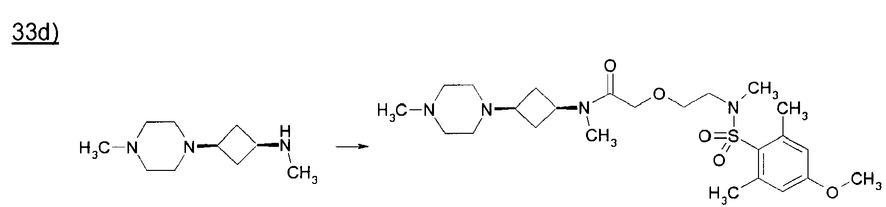

Analog Beispiel 1g) wurde die Titelverbindung aus Produkt 1d) und Produkt 33c) hergestellt.
C₂₄H₄₀N₄O₅S x C₂HF₃O₂ (610.69)
[M+H]⁺ = 497
analytische HPLC (Methode 3): Retentionszeit = 1.56 min

### Beispiel 34

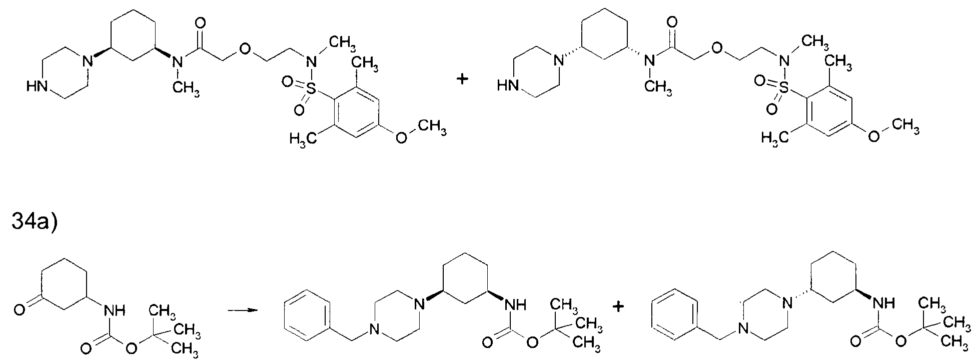

Analog Beispiel (1e) wurde 3-(tert-Butyloxycarbonyl-amino)-cyclohexanon mit 1-Benzylpiperazin umgesetzt. Man erhielt das Produkt als Diastereomeren-Gemisch, das chromatographisch in die racemischen *cis*- und trans-Gemische getrennt wurde (HPLC-Methode 10).
*cis-*Racemat:
C₂₂H₃₅N₃O₂ (373.5)
[M+H]⁺ = 374
analytische HPLC (Methode 4): Retentionszeit = 1.24 min
*trans*-Racemat:
C₂₂H₃₅N₃O₂ (373.5) [M+H]⁺ = 374
analytische HPLC (Methode 4): Retentionszeit = 1.29 min

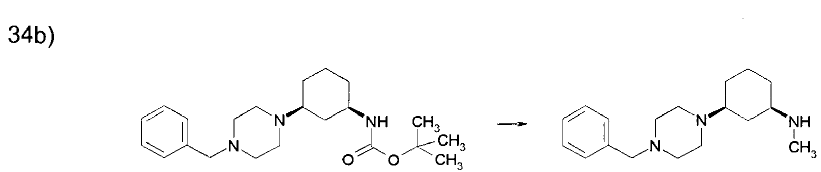

Das *cis*-Racemat aus Beispiel (34a) wurde analog Beispiel (1f) mit Lithiumaluminiumhydrid reduziert. Man erhielt das Produkt als racemisches Gemisch der *cis*-Isomeren.
C₁₈H₂₉N₃ (287.4)
[M+H]⁺ = 288
analytische HPLC (Methode 6): Retentionszeit = 1.69 min

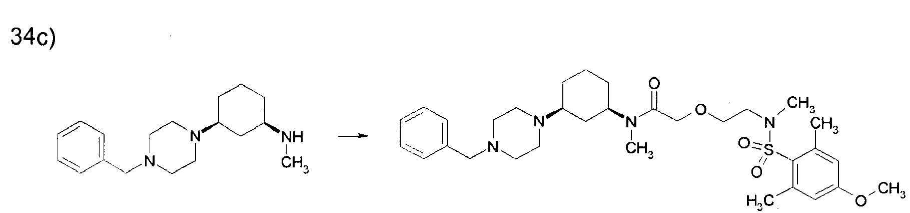

Das Produkt aus Beispiel (34b) wurde analog Beispiel (1 g) weiter umgesetzt. Man erhielt das Produkt als racemisches Gemisch der *cis*-Isomeren.
C₃₂H₄₈N₄O₅S (600.8)
[M+H]⁺ = 601
analytische HPLC (Methode 6): Retentionszeit = 1.79 min

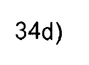

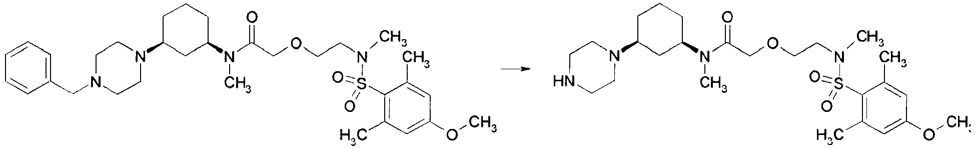

Das Produkt aus (34c) (86 mg, 0.14 mmol) wurde in 10 ml Methanol gelöst, mit 50 mg Pd/Kohle (10%) versetzt und zwei Stunden bei Raumtemperatur hydriert. Man erhielt das Produkt als racemisches Gemisch der *cis*-Isomeren.
Ausbeute: 63 mg (60% der Theorie)
C₂₅H₄₂N₄O₅S (510.7)
[M+H]⁺ = 511
analytische HPLC (Methode 6): Retentionszeit = 1.62 min

### Beispiel 35

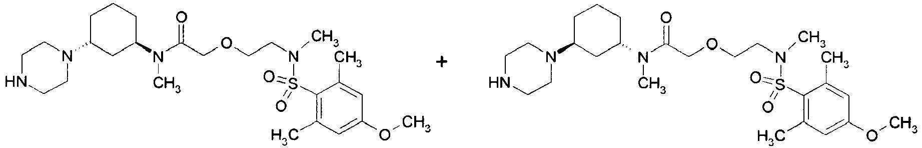

Analog Beispiel (34) wurde das Produkt, ausgehend vom *trans*-Racemat aus Beispiel (34a) in drei Syntheseschritten hergestellt.
C₂₅H₄₂N₄O₅S (510.7)
[M+H]⁺ = 511
analytische HPLC (Methode 6): Retentionszeit = 1.44 min

### Beispiel 36

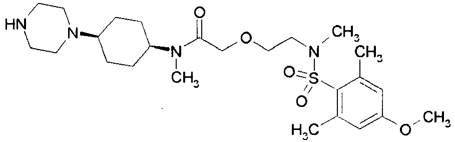

Analog Beispiel (34) wurde das Produkt, ausgehend von 4-(tert-Butyloxycarbonyl-amino)-cyclohexanon, in vier Syntheseschritten hergestellt
C₂₅H₄₂N₄O₅S (510.7)
[M+H]⁺ = 511
analytische HPLC (Methode 4): Retentionszeit = 1.68 min

Die nachfolgenden Beispiele beschreiben pharmazeutischer Darreichungsformen, die als Wirkstoff eine beliebige Verbindung der allgemeinen Formel I enthalten:

### Beispiel I

### Trockenampulle mit 75 mg Wirkstoff pro 10 ml

### Zusammensetzung:

| | |
|---|---|
| Wirkstoff | 75.0 mg |
| Mannitol | 500 mg |
| Wasser für Injektionszwecke ad | 10.0 ml |

### Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

### Beispiel II

### Tablette mit 50 mg Wirkstoff

### Zusammensetzung:

| | |
|---|---|
| (1) Wirkstoff | 50.0 mg |
| (2) Milchzucker | 98.0 mg |
| (3) Maisstärke | 50.0 mg |
| (4) Polyvinylpyrrolidon | 15.0 mg |
| (5) Magnesiumstearat | 2.0 mg |
| | 215.0 mg |

### Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe.
Durchmesser der Tabletten: 9 mm.

### Beispiel III

### Tablette mit 350 mg Wirkstoff

### Zusammensetzung:

| | |
|---|---|
| (1) Wirkstoff | 350.0 mg |
| (2) Milchzucker | 136.0 mg |
| (3) Maisstärke | 80.0 mg |
| (4) Polyvinylpyrrolidon | 30.0 mg |
| (5) Magnesiumstearat | 4.0 mg |
| | 600.0 mg |

### Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe.
Durchmesser der Tabletten: 12 mm.

### Beispiel IV

### Kapseln mit 50 mg Wirkstoff

### Zusammensetzung:

| | |
|---|---|
| (1) Wirkstoff | 50.0 mg |
| (2) Maisstärke getrocknet | 58.0 mg |
| (3) Milchzucker pulverisiert | 50.0 mg |
| (4) Magnesiumstearat | 2.0 mg |
| | 160.0 mg |

### Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 3 abgefüllt.

### Beispiel V

### Kapseln mit 350 mg Wirkstoff

### Zusammensetzung:

| | |
|---|---|
| (1) Wirkstoff | 350.0 mg |
| (2) Maisstärke getrocknet | 46.0 mg |
| (3) Milchzucker pulverisiert | 30.0 mg |
| (4) Magnesiumstearat | 4.0 mg |
| | 430.0 mg |

### Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Gr6Be 0 abgefüllt.

### Beispiel VI

### Suppositorien mit 100 mg Wirkstoff

1 Zäpfchen enthält:

| | |
|---|---|
| Wirkstoff | 100.0 mg |
| Polyethylenglykol (M.G. 1500) | 600.0 mg |
| Polyethylenglykol (M.G. 6000) | 460.0 mg |

| | |
|---|---|
| Polyethylensorbitanmonostearat | 840.0 mg |
| | 2000.0 mg |

## Patentansprüche

1. Verbindungen der allgemeinen Formel I 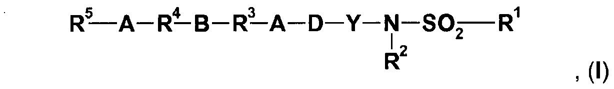 in der
**A** eine Bindung,
**B** eine Bindung,
**D-Y** zusammen eine Gruppe ausgewählt aus 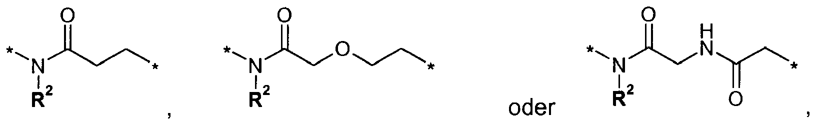
**R¹** die Gruppe 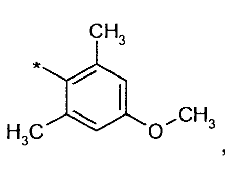
**R²** H oder C₁₋₃-Alkyl-, wobei jede Methylengruppe mit bis zu zwei und jede Methylgruppe mit bis zu drei Fluoratomen substituiert sein kann, oder auch H₃C-C(O)-,
**R³** eine C₄₋₆-Cycloalkylengruppe, die durch einen, zwei oder drei Reste **R^{3.1}** substituiert sein kann,
**R^{3.1}** -CH₃, -C₂H₅, *iso*-Propyl, *tert-*Butyl, -OH, F, Cl, Br, I,
**R⁴** einen gesättigten 6- oder 7-gliedrigen Diaza-Heterocyclus,
**R⁵** C₁₋₃-Alkyl- oder C₃₋₅-Cycloalkyl bedeutet,
deren Enantiomere, deren Diastereomere, deren Gemische und deren Salze, insbesondere deren physiologisch verträgliche Salze mit organischen oder anorganischen Säuren oder Basen.

2. Verbindungen der allgemeinen Formel **I** gemäß Anspruch 1, in denen
**A** eine Bindung,
**B** eine Bindung,
**D-Y** zusammen eine Gruppe ausgewählt aus 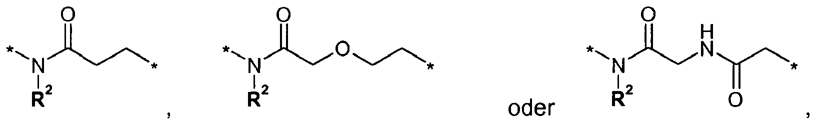
**R¹** die Gruppe 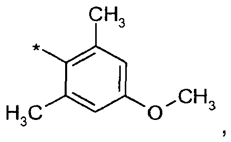
**R²** H oder C₁₋₃-Alkyl-, wobei jede Methylengruppe mit bis zu zwei und jede Methylgruppe mit bis zu drei Fluoratomen substituiert sein kann, oder auch H₃C-C(O)-,
**R³** eine C₄₋₆-Cycloalkylengruppe,
**R⁴** einen gesättigten 6- oder 7-gliedrigen Diaza-Heterocyclus,
**R⁵** C₁₋₃-Alkyl- oder C₃₋₅-Cycloalkyl bedeutet,
deren Enantiomere, deren Diastereomere, deren Gemische und deren Salze, insbesondere deren physiologisch verträgliche Salze mit organischen oder anorganischen Säuren oder Basen.

3. Verbindungen der allgemeinen Formel **I** gemäß Anspruch 1, in denen **A, B, D, Y,**
**R¹, R², R⁴** und **R⁵** wie in Anspruch 1 definiert sind und
**R³** eine C₄₋₆-Cycloalkylengruppe, die durch einen, zwei oder drei Reste **R^{3.1}** substituiert sein kann, und
**R^{3.1}** -CH₃, -C₂H₅, *iso*-Propyl, *tert*-Butyl, -OH, F, Cl, Br oder I bedeutet,
mit der Maßgabe, dass die voranstehend erwähnte C₄₋₆-Cycloalkylengruppe in 1,3-Stellung mit dem restlichen Molekül verknpüft ist,
deren Enantiomere, deren Diastereomere, deren Gemische und deren Salze, insbesondere deren physiologisch verträgliche Salze mit organischen oder anorganischen Säuren oder Basen.

4. Verbindungen der allgemeinen Formel **I** gemäß Anspruch 1, in denen **A, B, D, Y,**
**R¹, R², R⁴** und **R⁵** wie in Anspruch 2 definiert sind und
**R³** eine C₄₋₆-Cycloalkylengruppe, die durch einen, zwei oder drei Reste **R^{3.1}** substituiert sein kann, und
**R^{3.1}** -CH₃, -C₂H₅, *iso*-Propyl, *tert*-Butyl, -OH, F, Cl, Br oder I bedeutet,
mit der Maßgabe, dass die voranstehend erwähnte C₄₋₆-Cycloalkylengruppe in 1,3-Stellung mit dem restlichen Molekül verknpüft ist,
deren Enantiomere, deren Diastereomere, deren Gemische und deren Salze, insbesondere deren physiologisch verträgliche Salze mit organischen oder anorganischen Säuren oder Basen.

5. Folgende Verbindungen der allgemeinen Formel **I** gemäß Anspruch 1:
| **Nr.** | **Struktur** |
|---|---|
| (1) | 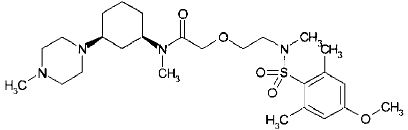 |
| (2) | 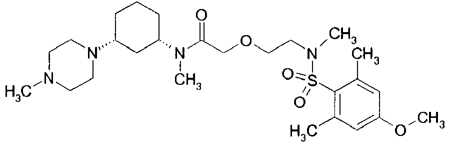 |
| (3) | 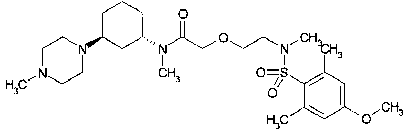 |
| (4) | 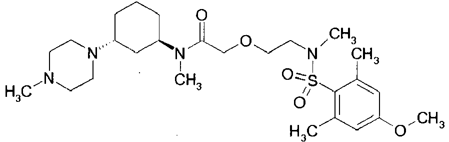 |
| (5) | 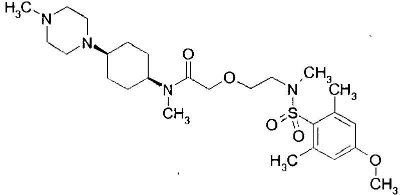 |
| (6) | 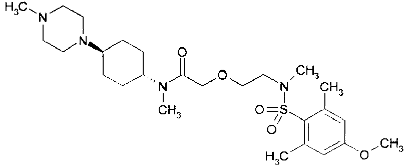 |
| (7) | 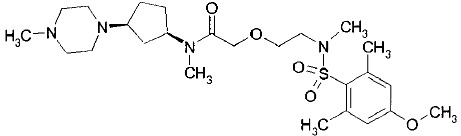 |
| (8) | 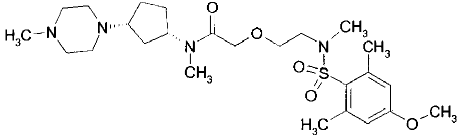 |
| (9) | 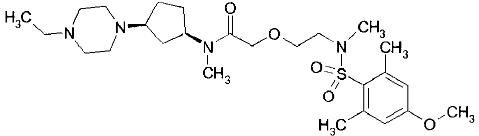 |
| (10) | 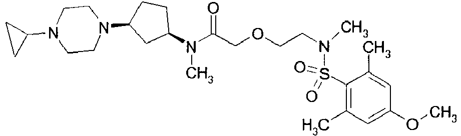 |
| (11) | 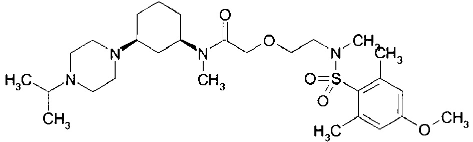 |
| (12) | 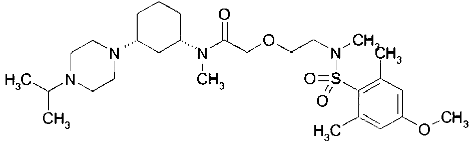 |
| (13) | 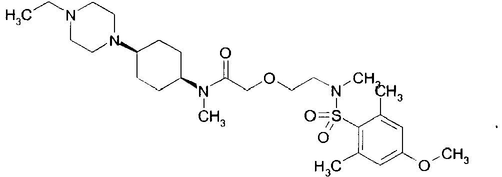 |
| (14) | 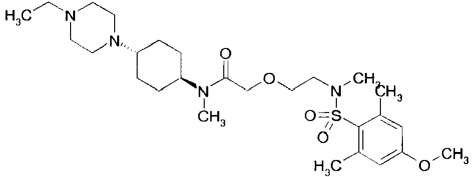 |
| (15) | 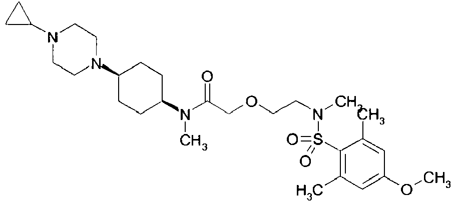 |
| (16) | 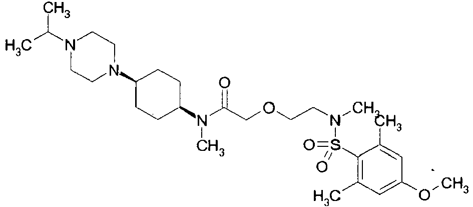 |
| (17) | 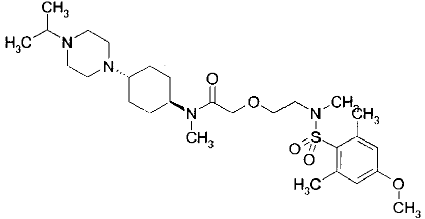 |
| (18) | 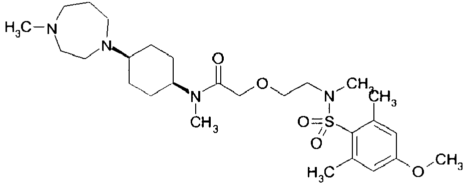 |
| (19) | 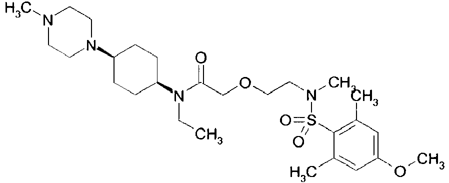 |
| (20) | 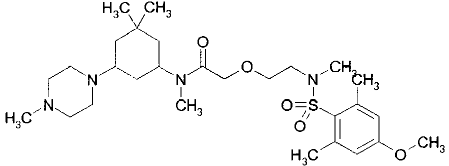 |
| (21) | 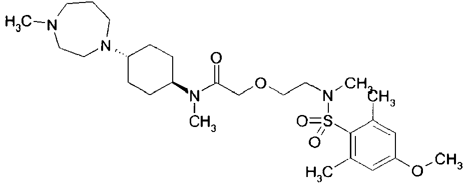 |
| (22) | 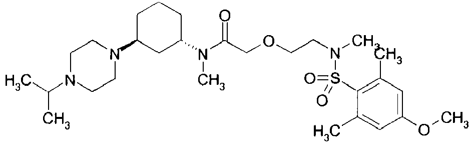 |
| (23) | 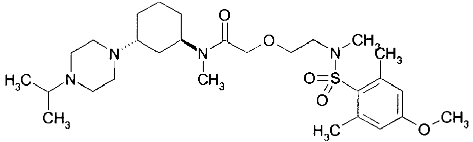 |
| (24) | 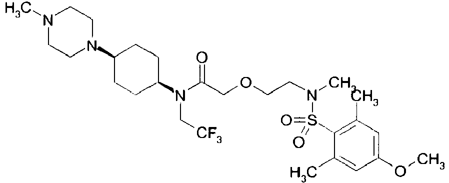 |
| (25) | 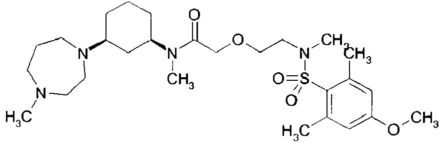 |
| (26) | 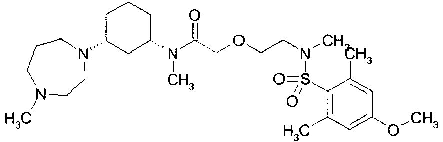 |
| (27) | 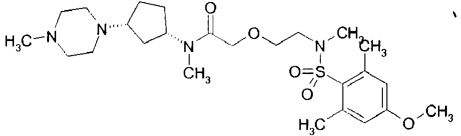 |
| (28) | 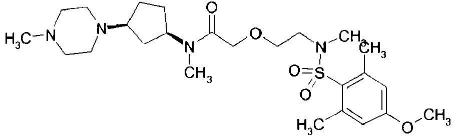 |
| (29) | 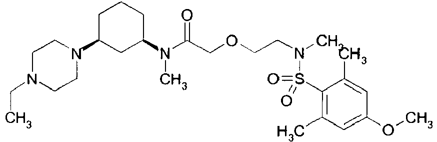 |
| (30) | 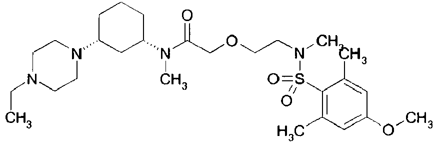 |
| (31) | 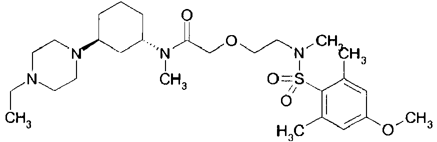 |
| (32) | 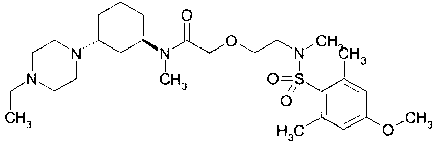 |
| (33) | 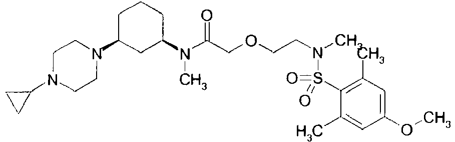 |
| (34) | 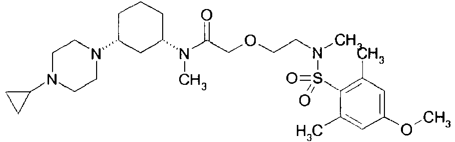 |
| (35) | 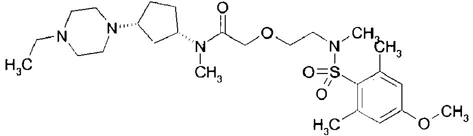 |
| (36) | 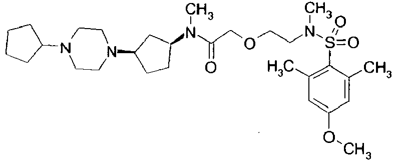 |
| (37) | 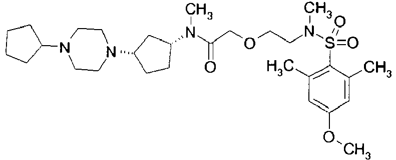 |
| (38) | 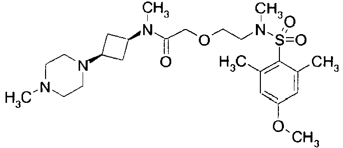 |
| (39) | 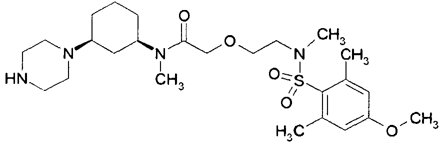 |
| (40) | 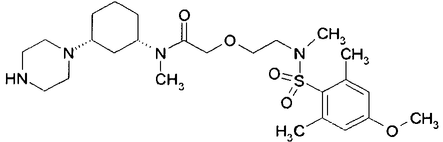 |
| (41) | 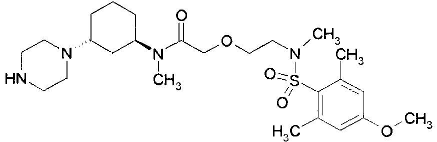 |
| (42) | 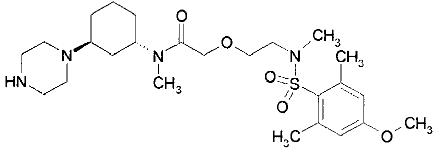 |
| (43) | 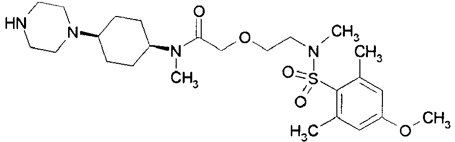 |
deren Enantiomere, deren Diastereomere, deren Gemische und deren Salze, insbesondere deren physiologisch verträgliche Salze mit organischen oder anorganischen Säuren oder Basen.

6. Folgende Verbindungen der allgemeinen Formel **I** gemäß Anspruch 1:
| **Nr.** | **Struktur** |
|---|---|
| (1) | 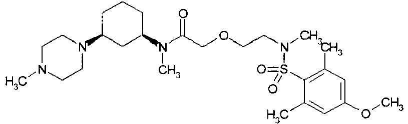 |
| (2) | 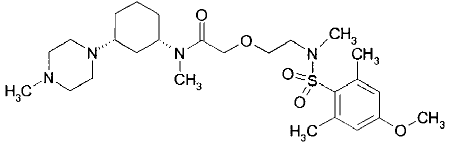 |
| (3) | 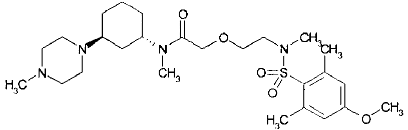 |
| (4) | 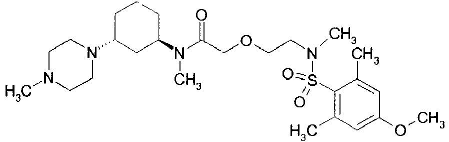 |
| (5) | 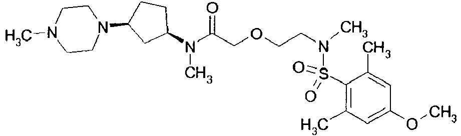 |
| (6) | 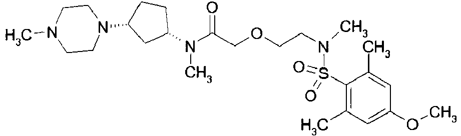 |
| (7) | 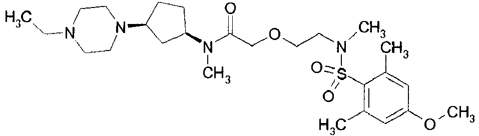 |
| (8) | 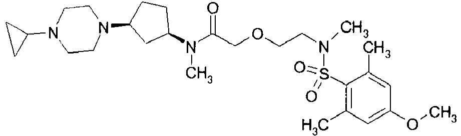 |
| (9) | 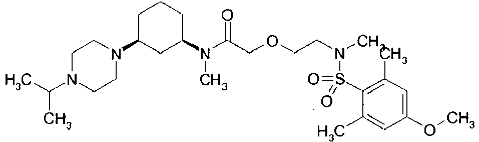 |
| (10) | 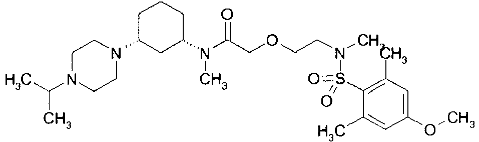 |
| (11) | 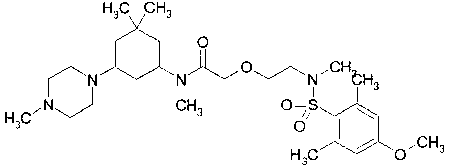 |
| (12) | 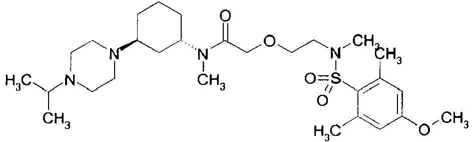 |
| (13) | 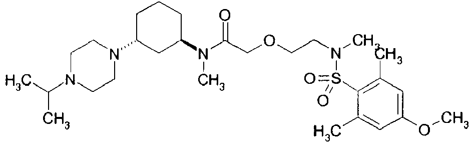 |
| (14) | 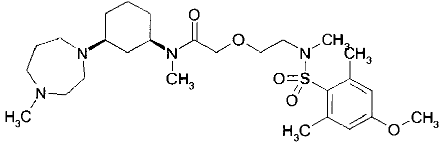 |
| (15) | 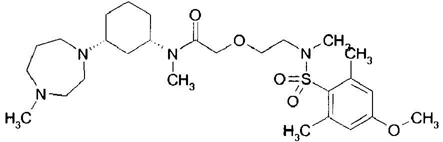 |
| (16) | 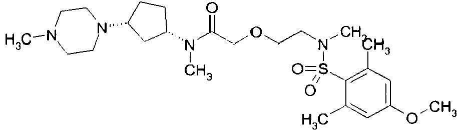 |
| (17) | 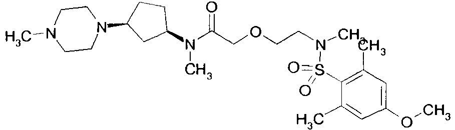 |
| (18) | 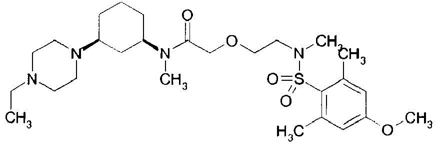 |
| (19) | 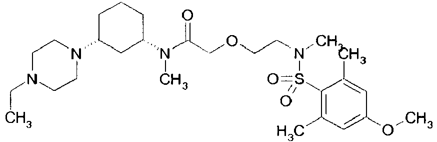 |
| (20) | 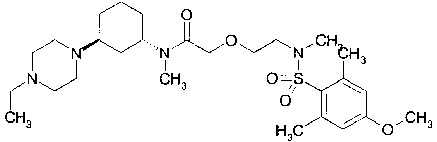 |
| (21) | 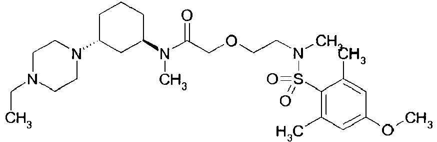 |
| (22) | 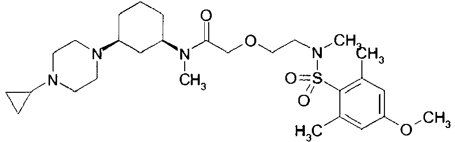 |
| (23) | 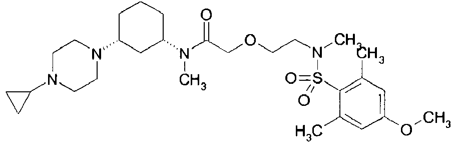 |
| (24) | 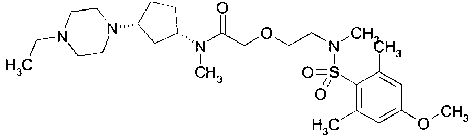 |
| (25) | 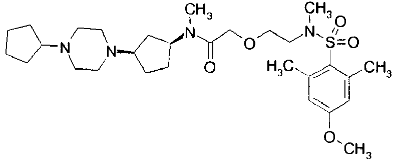 |
| (26) | 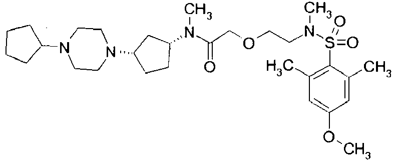 |
| (27) | 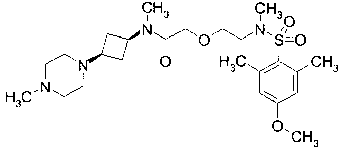 |
| (28) | 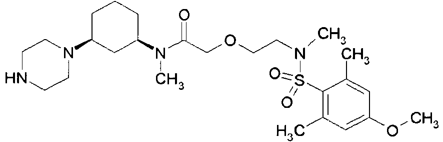 |
| (29) | 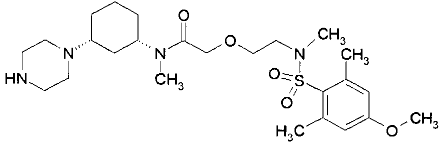 |
| (30) | 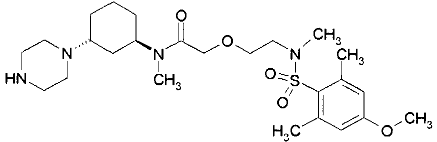 |
| (31) | 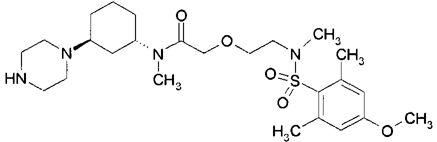 |
deren Enantiomere, deren Diastereomere, deren Gemische und deren Salze, insbesondere deren physiologisch verträgliche Salze mit organischen oder anorganischen Säuren oder Basen.

7. Physiologisch verträgliche Salze der Verbindungen nach einem der Ansprüche 1 bis 6 mit anorganischen oder organischen Säuren oder Basen.

8. Arzneimittel, enthaltend eine Verbindung nach mindestens einem der Ansprüche 1 bis 6 oder ein physiologisch verträgliches Salz gemäß Anspruch 7 neben gegebenenfalls einem oder mehreren inerten Trägerstoffen und/oder Verdünnungsmitteln.

9. Verwendung einer Verbindung nach mindestens einem der Ansprüche 1 bis 7 zur Herstellung eines Arzneimittels zur akuten und prophylaktischen Behandlung von akuten Schmerzen, Eingeweideschmerzen, neuropathischen Schmerzen, entzündlichen / Schmerzrezeptor-vermittelten Schmerzen, Tumorschmerzen und Kopfschmerz-Erkrankungen.

10. Verfahren zur Herstellung eines Arzneimittels gemäß Anspruch 8, **dadurch gekennzeichnet, dass** auf nichtchemischem Weg eine Verbindung nach mindestens einem der Ansprüche 1 bis 7 in einen oder mehrere inerte Trägerstoffe und/oder Verdünnungsmittel eingearbeitet wird.

## Claims

1. Compounds of general formula I 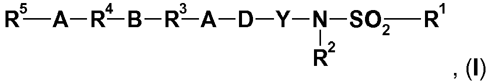 wherein
**A** denotes a bond,
**B** denotes a bond,
**D-Y** together denote a group selected from 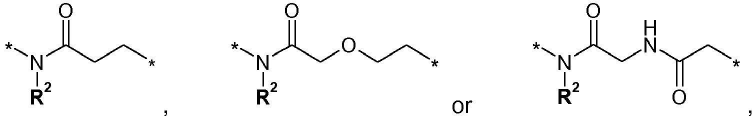
**R¹** denotes the group 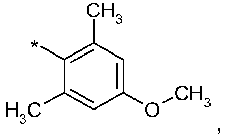
**R²** denotes H or C₁₋₃-alkyl, while each methylene group may be substituted by up to two fluorine atoms and each methyl group may be substituted by up to three fluorine atoms, or also H₃C-C(O),
**R³** denotes a C₄₋₆-cycloalkylene group which may be substituted by one, two or three **R^{3.1}** groups,
**R^{3.1}** denotes -CH₃, -C₂H₅, *iso*-propyl, *tert*-butyl, -OH, F, Cl, Br, I,
**R⁴** denotes a saturated 6- or 7-membered diaza heterocycle,
**R⁵** denotes C₁₋₃-alkyl or C₃₋₅-cycloalkyl,
the enantiomers, the diastereomers, the mixtures thereof and the salts thereof, particularly the physiologically acceptable salts thereof with organic or inorganic acids or bases.

2. Compounds of general formula **I** according to claim 1, wherein
**A** denotes a bond,
**B** denotes a bond,
**D-Y** together denote a group selected from 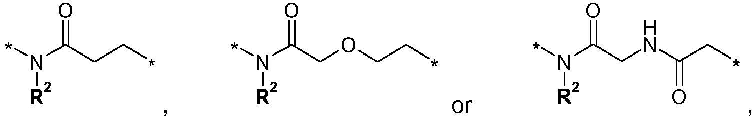
**R¹** denotes the group 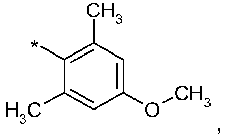
**R²** denotes H or C₁₋₃-alkyl , while each methylene group may be substituted by up to two fluorine atoms and each methyl group may be substituted by up to three fluorine atoms, or also H₃C-C(O),
**R³** denotes a C₄₋₆-cycloalkylene group,
**R⁴** denotes a saturated 6- or 7-membered diaza heterocycle,
**R⁵** denotes C₁₋₃-alkyl or C₃₋₅-cycloalkyl,
the enantiomers, the diastereomers, the mixtures thereof and the salts thereof, particularly the physiologically acceptable salts thereof with organic or inorganic acids or bases.

3. Compounds of general formula **I** according to claim 1, wherein **A, B, D, Y, R¹, R²,**
**R⁴** and **R⁵** are defined as in claim 1 and
**R³** denotes a C₄₋₆-cycloalkylene group which may be substituted by one, two or three **R^{3.1}** groups, and
**R^{3.1}** denotes -CH₃, -C₂H₅, *iso*-propyl, *tert*-butyl, -OH, F, Cl, Br or I,
with the proviso that the above-mentioned C₄₋₆-cycloalkylene group is linked in the 1,3 position to the remainder of the molecule,
the enantiomers, the diastereomers, the mixtures thereof and the salts thereof, particularly the physiologically acceptable salts thereof with organic or inorganic acids or bases.

4. Compounds of general formula **I** according to claim 1, wherein A, B, D, Y, **R¹, R²,**
**R⁴** and **R⁵** are defined as in claim 2 and
**R³** denotes a C₄₋₆-cycloalkylene group which may be substituted by one, two or three **R^{3.1}** groups, and
**R^{3.1}** denotes -CH₃, -C₂H₅, *iso*-propyl, *tert*-butyl, -OH, F, Cl, Br or I,
with the proviso that the above-mentioned C₄₋₆-cycloalkylene group is linked in the 1,3 position to the remainder of the molecule,
the enantiomers, the diastereomers, the mixtures thereof and the salts thereof, particularly the physiologically acceptable salts thereof with organic or inorganic acids or bases.

5. The following compounds of general formula **I** according to claim 1:
| **No.** | **Structure** |
|---|---|
| (1) | 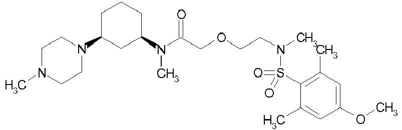 |
| (2) | 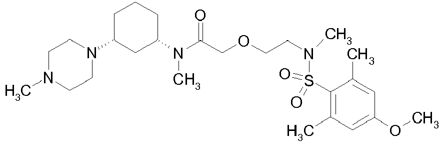 |
| (3) | 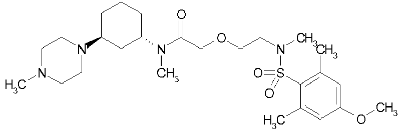 |
| (4) | 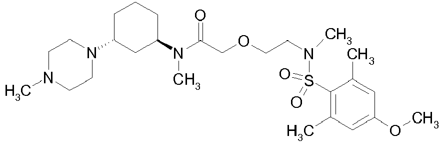 |
| (5) | 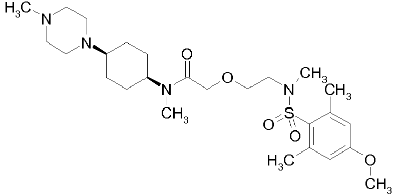 |
| (6) | 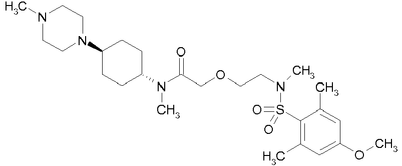 |
| (7) | 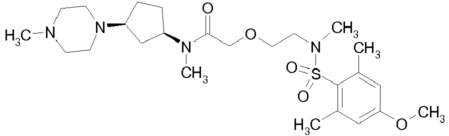 |
| (8) | 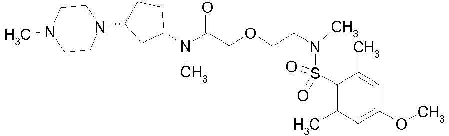 |
| (9) | 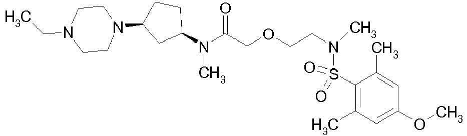 |
| (10) | 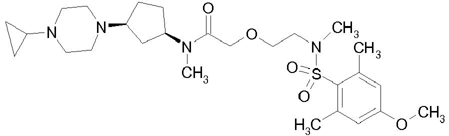 |
| (11) | 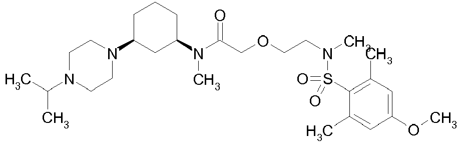 |
| (12) | 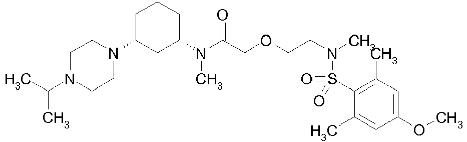 |
| (13) | 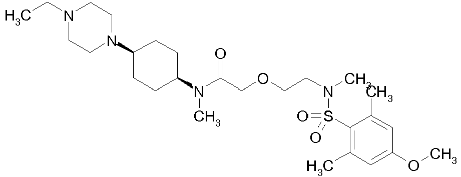 |
| (14) | 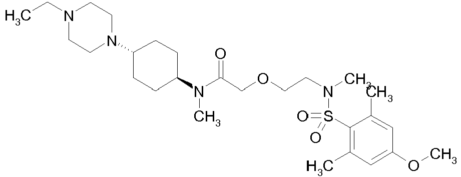 |
| (15) | 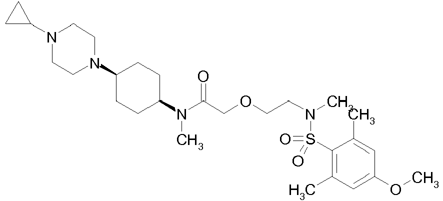 |
| (16) | 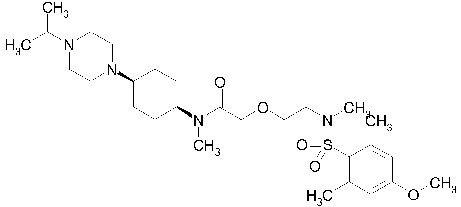 |
| (17) | 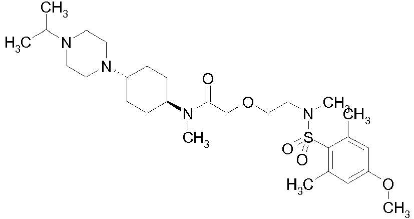 |
| (18) | 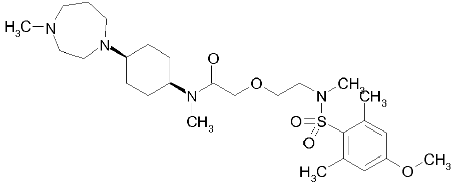 |
| (19) | 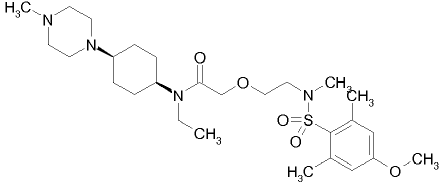 |
| (20) | 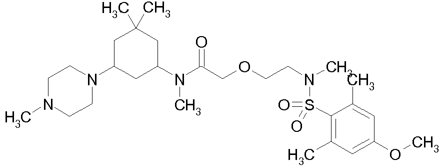 |
| (21) | 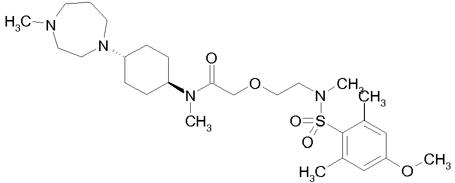 |
| (22) | 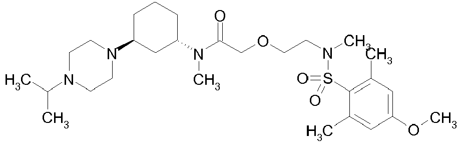 |
| (23) | 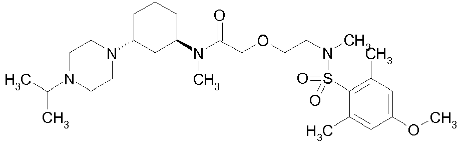 |
| (24) | 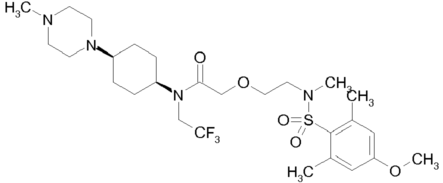 |
| (25) | 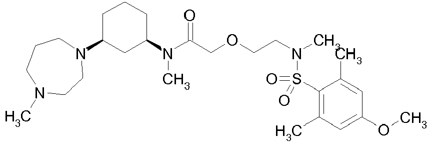 |
| (26) | 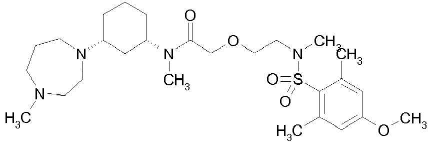 |
| (27) | 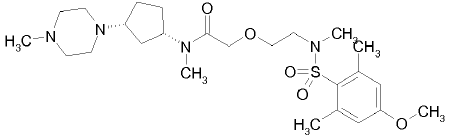 |
| (28) | 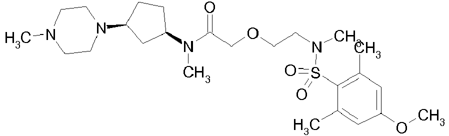 |
| (29) | 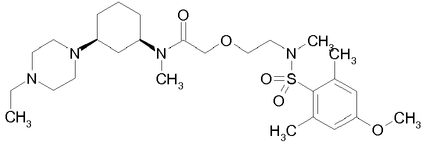 |
| (30) | 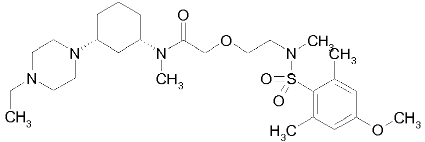 |
| (31) | 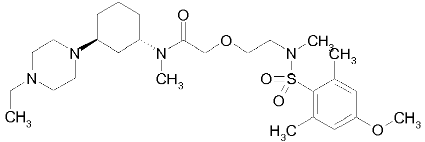 |
| (32) | 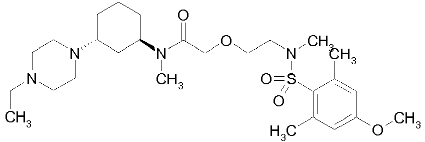 |
| (33) | 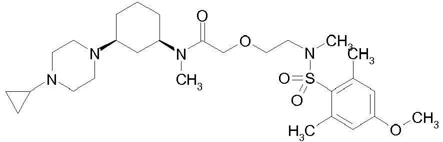 |
| (34) | 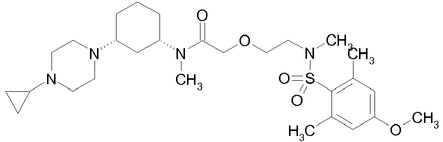 |
| (35) | 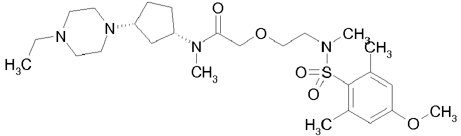 |
| (36) | 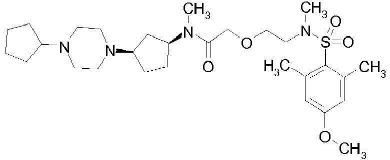 |
| (37) | 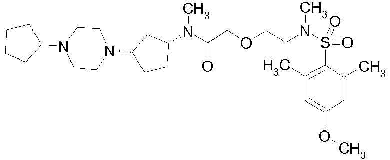 |
| (38) | 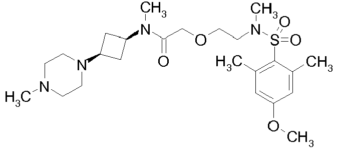 |
| (39) | 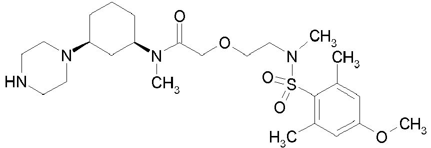 |
| (40) | 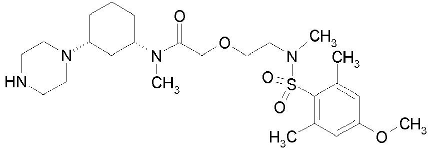 |
| (41) | 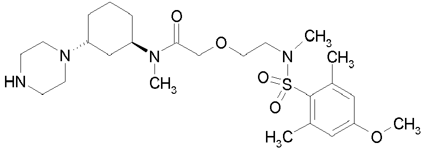 |
| (42) | 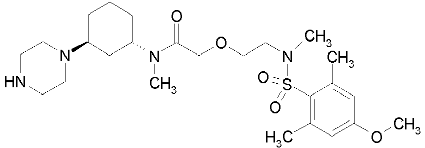 |
| (43) | 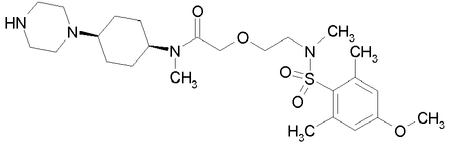 |
the enantiomers, the diastereomers, the mixtures thereof and the salts thereof, particularly the physiologically acceptable salts thereof with organic or inorganic acids or bases.

6. The following compounds of general formula **I** according to claim 1:
| **No.** | **Structure** |
|---|---|
| (1) | 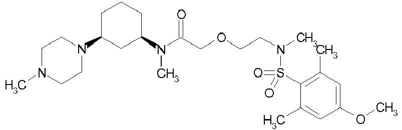 |
| (2) | 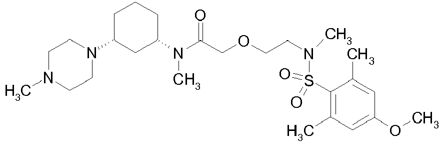 |
| (3) | 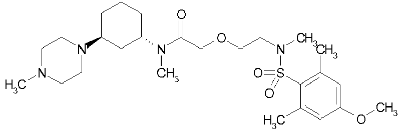 |
| (4) | 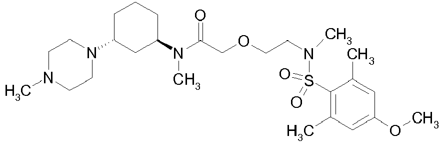 |
| (5) | 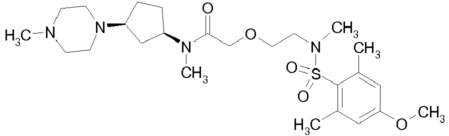 |
| (6) | 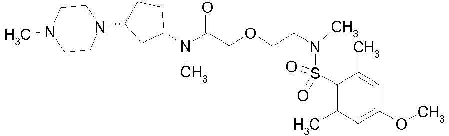 |
| (7) | 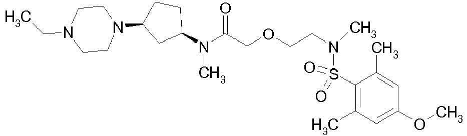 |
| (8) | 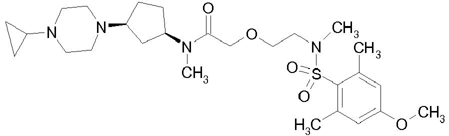 |
| (9) | 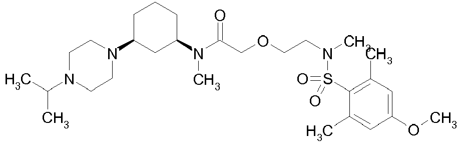 |
| (10) | 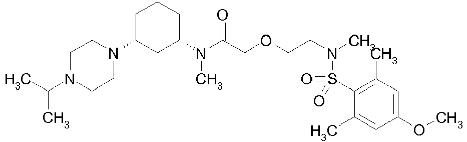 |
| (11) | 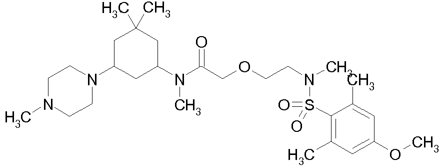 |
| (12) | 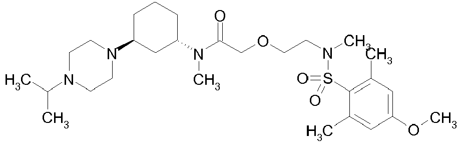 |
| (13) | 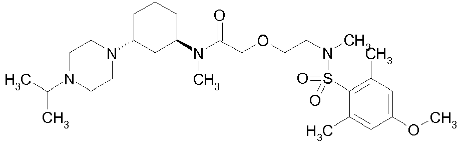 |
| (14) | 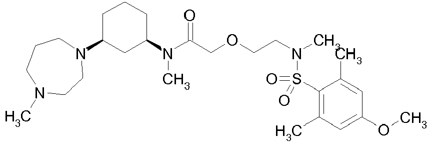 |
| (15) | 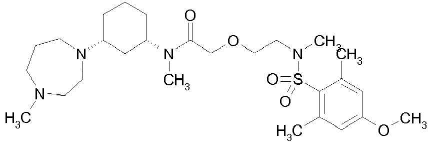 |
| (16) | 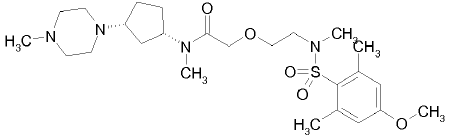 |
| (17) | 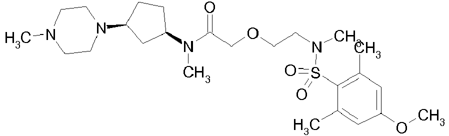 |
| (18) | 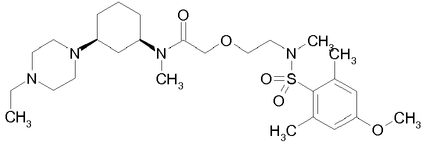 |
| (19) | 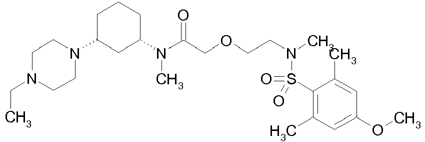 |
| (20) | 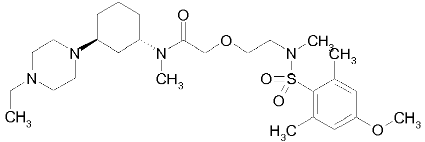 |
| (21) | 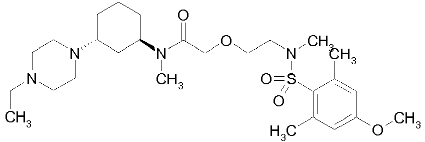 |
| (22) | 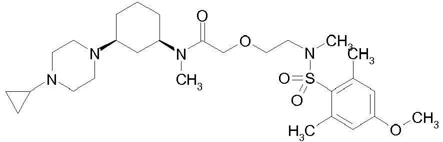 |
| (23) | 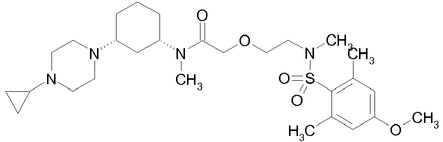 |
| (24) | 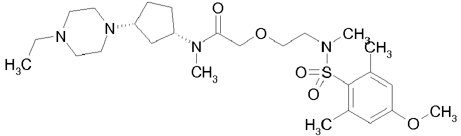 |
| (25) | 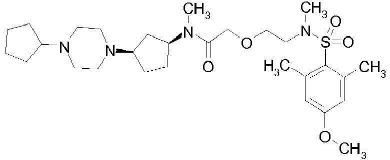 |
| (26) | 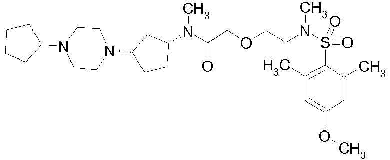 |
| (27) | 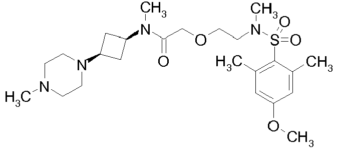 |
| (28) | 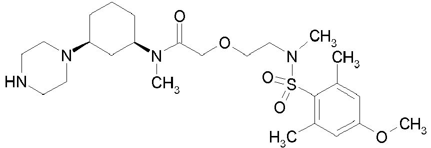 |
| (29) | 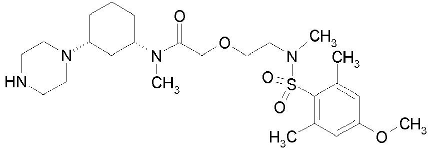 |
| (30) | 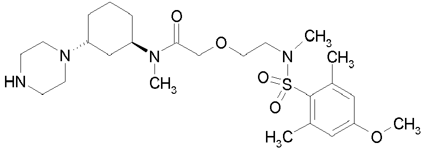 |
| (31) | 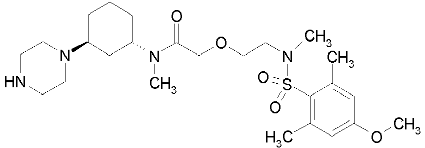 |
the enantiomers, the diastereomers, the mixtures thereof and the salts thereof, particularly the physiologically acceptable salts thereof with organic or inorganic acids or bases.

7. Physiologically acceptable salts of the compounds according to one of claims 1 to 6 with inorganic or organic acids or bases.

8. Medicament, containing a compound according to at least one of claims 1 to 6 or a physiologically acceptable salt according to claim 7 optionally together with one or more inert carriers and/or diluents.

9. Use of a compound according to at least one of claims 1 to 7 for preparing a medicament for the acute and prophylactic treatment of acute pain, visceral pain, neuropathic pain, inflammatory/pain receptor-mediated pain, tumour pain and headache diseases.

10. Process for preparing a medicament according to claim 8, **characterised in that** by a non-chemical method a compound according to at least one of claims 1 to 7 is incorporated in one or more inert carriers and/or diluents.

## Revendications

1. Composés de formule générale I 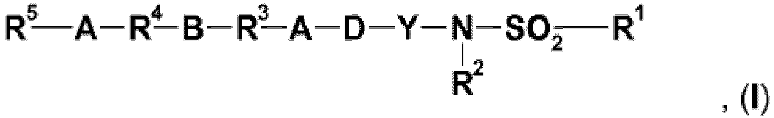 dans laquelle
A désigne une liaison
B désigne une liaison
D-Y désignent conjointement un groupe choisi parmi 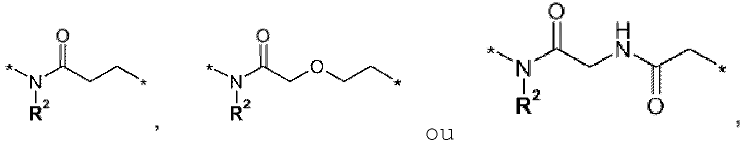
R¹ désigne le groupe 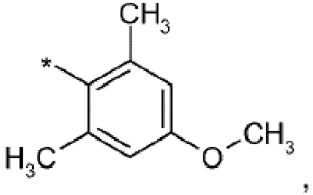
R² désigne H ou un groupe alkyle en C₁ à C₃, dans lequel chaque groupe méthylène peut être substitué par jusqu'à deux, et chaque groupe méthyle par jusqu'à trois atomes de fluor, ou également H₃C-C(O)-,
R³ désigne un groupe cycloalkylène en C₄ à C₆ qui peut être substitué par un, deux ou trois radicaux R^{3.1},
R^{3.1} désigne -CH₃, -CH₂H₅, un groupe *iso*-propyle, *tert-*butyle, -OH, F, Cl, Br, I,
R⁴ désigne un diaza-hétérocycle saturé à 6 ou 7 chaînons,
R⁵ désigne un groupe alkyle en C₁ à C₃ ou cycloalkyle en C₃ à C₅,
leurs énantiomères, leurs diastéréomères, leurs mélanges et leurs sels, en particulier leurs sels physiologiquement acceptables avec des acides ou bases organiques ou inorganiques.

2. Composés de formule générale I selon la
revendication 1, dans laquelle
A désigne une liaison
B désigne une liaison
D-Y désignent conjointement un groupe choisi parmi 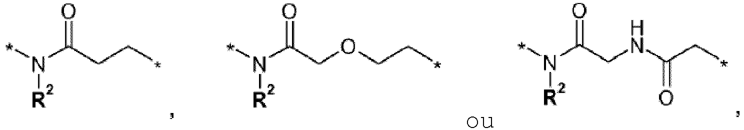
R¹ désigne le groupe 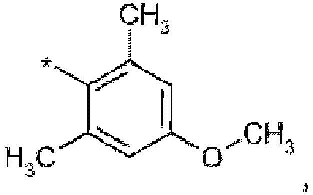
R² désigne H ou un groupe alkyle en C₁ à C₃, dans lequel chaque groupe méthylène peut être substitué par jusqu'à deux, et chaque groupe méthyle par jusqu'à trois atomes de fluor, ou également H₃C-C(O)-,
R³ désigne un groupe cycloalkylène en C₄ à C₆,
R⁴ désigne un diaza-hétérocycle saturé à 6 ou 7 chaînons,
R⁵ désigne un groupe alkyle en C₁ à C₃ ou cycloalkyle en C₃ à C₅,
leurs énantiomères, leurs diastéréomères, leurs mélanges et leurs sels, en particulier leurs sels physiologiquement acceptables avec des acides ou bases organiques ou inorganiques.

3. Composés de formule générale I selon la revendication 1, dans laquelle A, B, D, Y, R¹, R², R⁴ et R⁵ sont tels que définis dans la revendication 1 et
R³ désigne un groupe cycloalkylène en C₄ à C₆ qui peut être substitué par un, deux ou trois radicaux R^{3.1},
R^{3.1} désigne -CH₃, -CH₂H₅, un groupe *iso*-propyle, *tert-*butyle, -OH, F, Cl, Br ou I,
à condition que le groupe cycloalkylène en C₄ à C₆ mentionné précédemment en position 1,3 soit relié au reste de la molécule,
leurs énantiomères, leurs diastéréomères, leurs mélanges et leurs sels, en particulier leurs sels physiologiquement acceptables avec des acides ou bases organiques ou inorganiques.

4. Composés de formule générale I selon la revendication 1, dans laquelle A, B, D, Y, R¹, R², R⁴ et R⁵ sont tels que définis dans la revendication 2 et
R³ désigne un groupe cycloalkylène en C₄ à C₆ qui peut être substitué par un, deux ou trois radicaux R^{3.1}, et
R^{3.1} désigne -CH₃, -CH₂H₅, un groupe *iso*-propyle, *tert-*butyle, -OH, F, Cl, Br ou I,
à condition que le groupe cycloalkylène en C₄ à C₆ mentionné précédemment en position 1,3 soit relié au reste de la molécule,
leurs énantiomères, leurs diastéréomères, leurs mélanges et leurs sels, en particulier leurs sels physiologiquement acceptables avec des acides ou bases organiques ou inorganiques.

5. Composés de formule générale I suivants, selon la revendication 1 :
| N° | Structure |
|---|---|
| (1) | 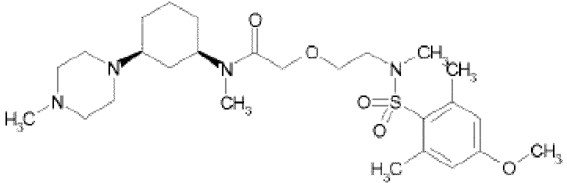 |
| (2) | 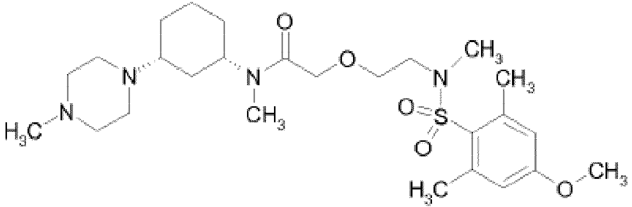 |
| (3) | 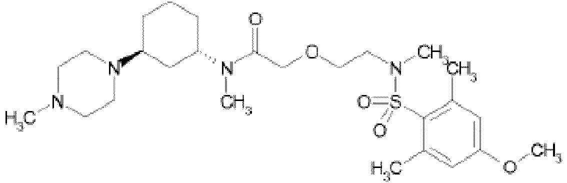 |
| (4) | 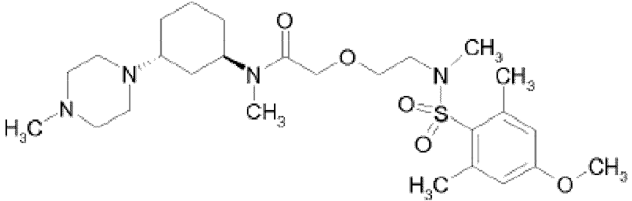 |
| (5) | 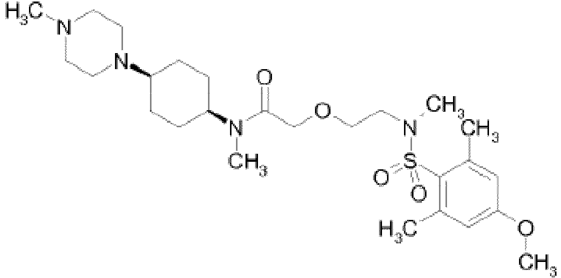 |
| (6) | 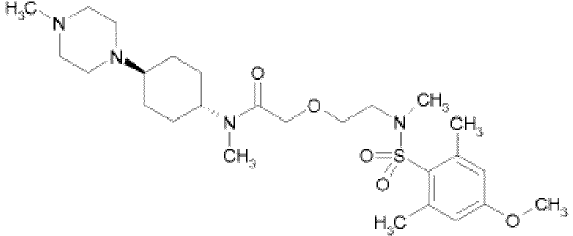 |
| (7) | 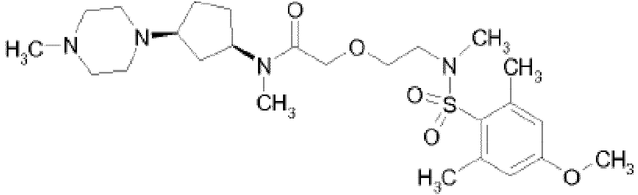 |
| (8) | 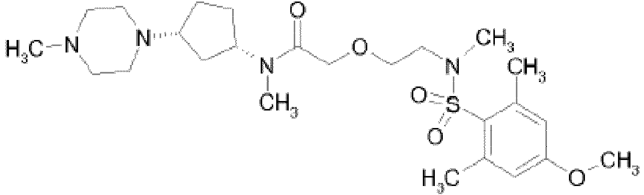 |
| (9) | 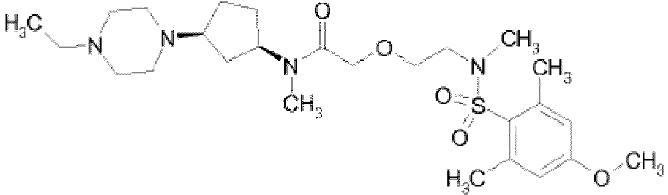 |
| (10) | 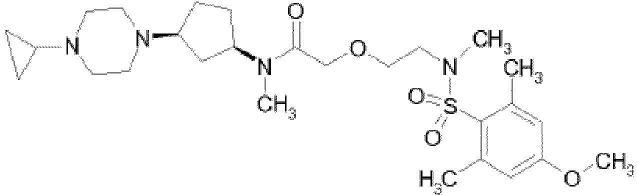 |
| (11) | 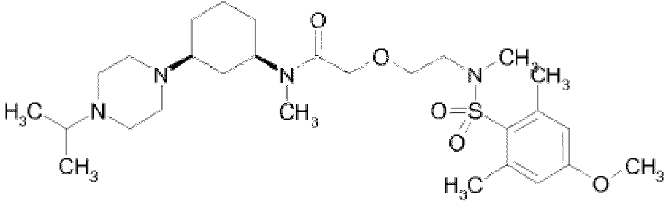 |
| (12) | 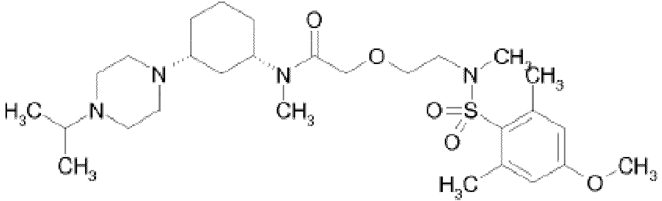 |
| (13) | 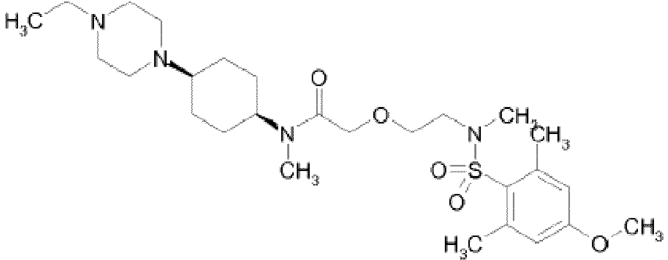 |
| (14) | 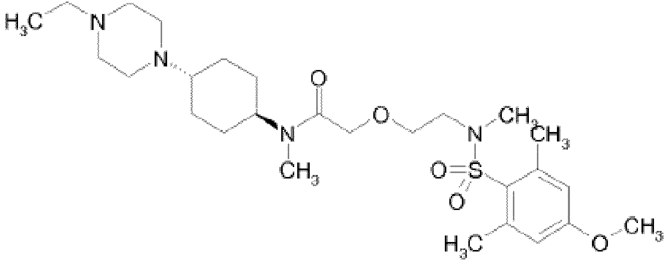 |
| (15) | 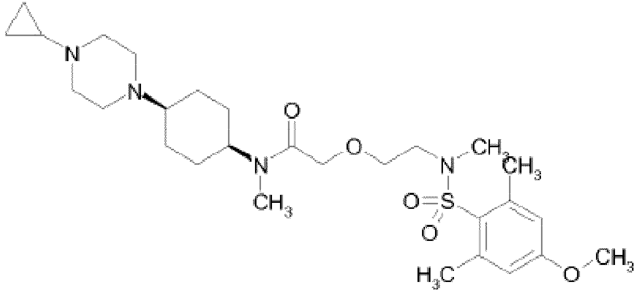 |
| (16) | 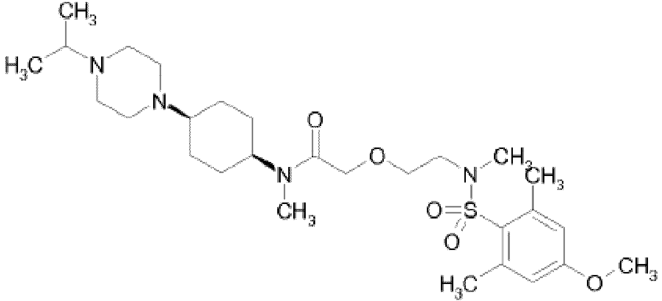 |
| (17) | 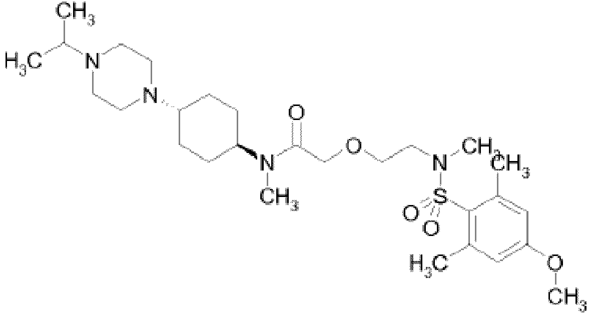 |
| (18) | 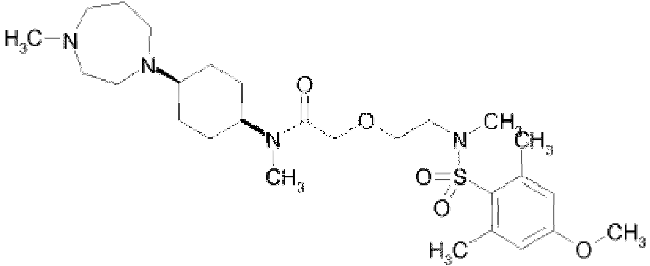 |
| (19) | 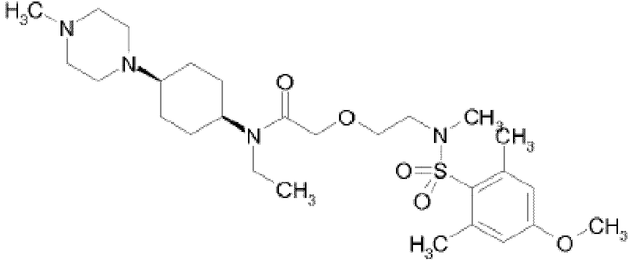 |
| (20) | 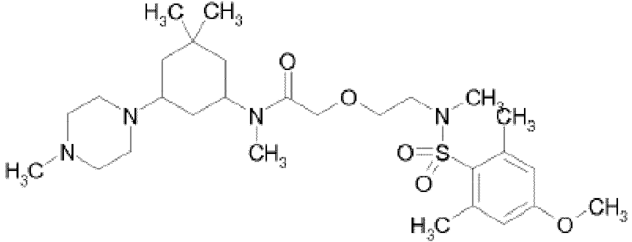 |
| (21) | 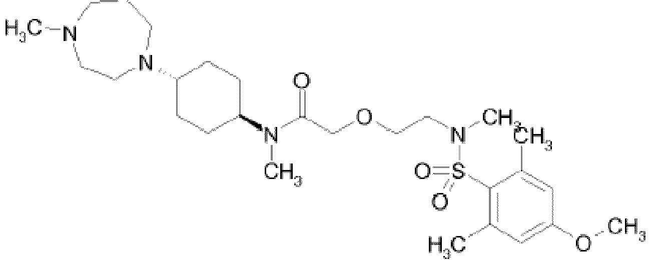 |
| (22) | 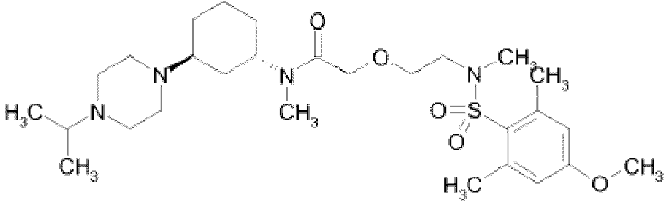 |
| (23) | 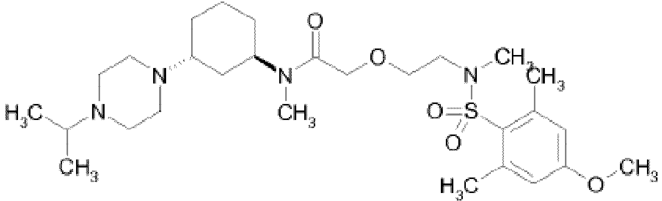 |
| (24) | 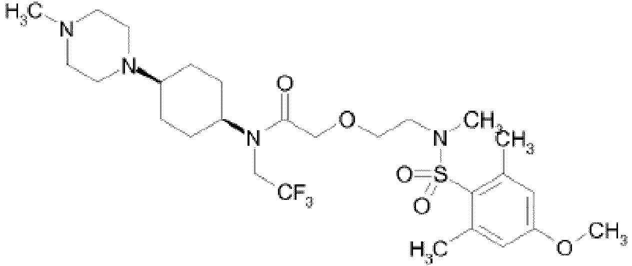 |
| (25) | 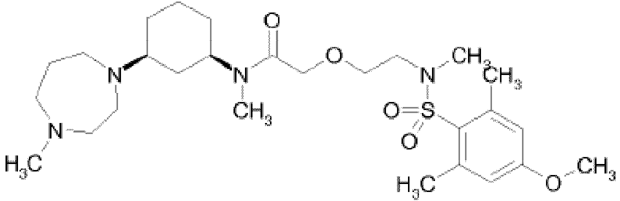 |
| (26) | 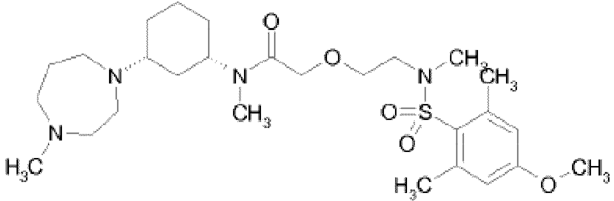 |
| (27) | 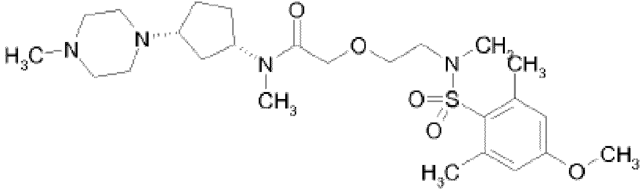 |
| (28) | 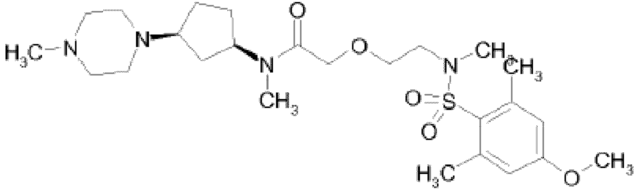 |
| (29) | 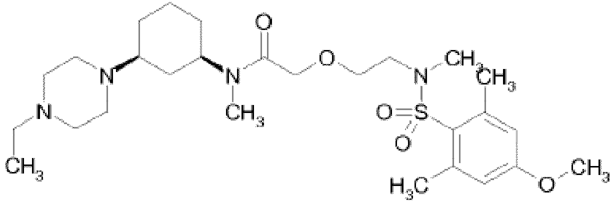 |
| (30) | 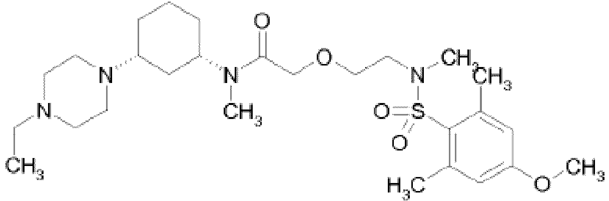 |
| (31) | 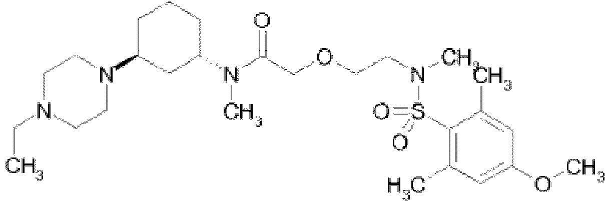 |
| (32) | 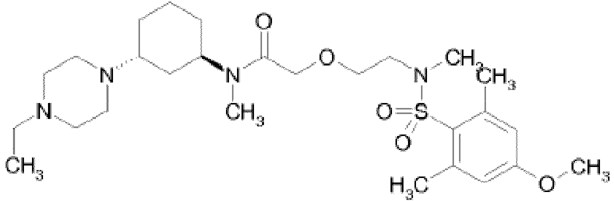 |
| (33) | 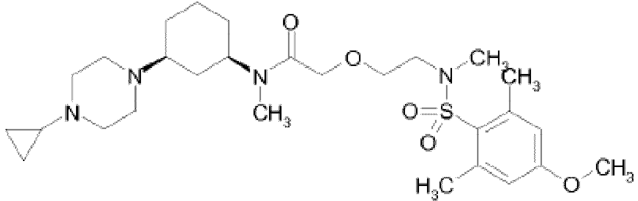 |
| (34) | 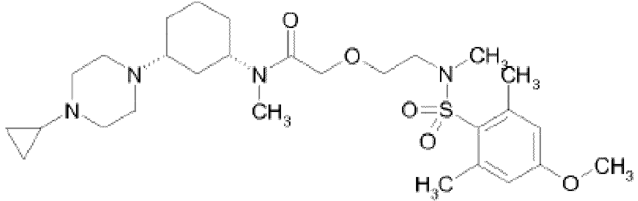 |
| (35) | 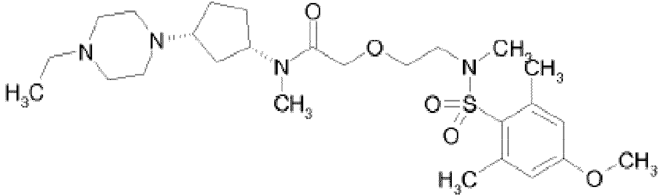 |
| (36) | 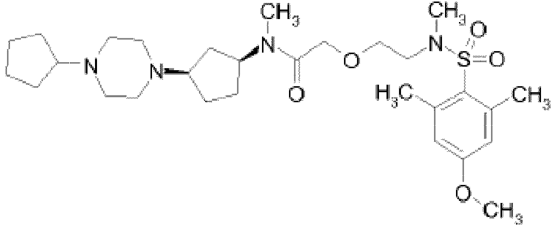 |
| (37) | 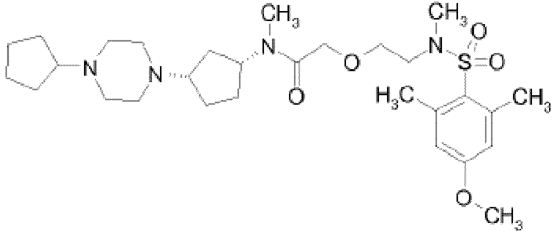 |
| (38) | 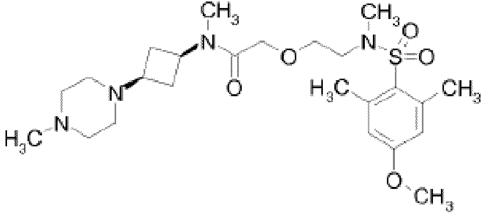 |
| (39) | 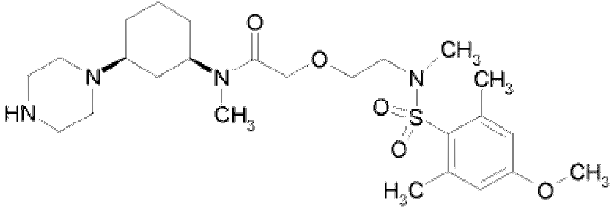 |
| (40) | 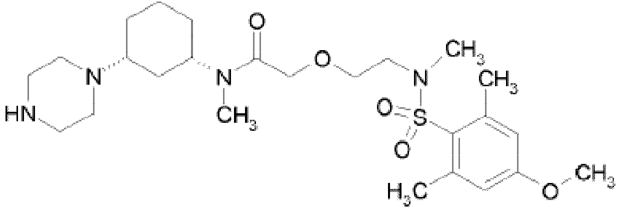 |
| (41) | 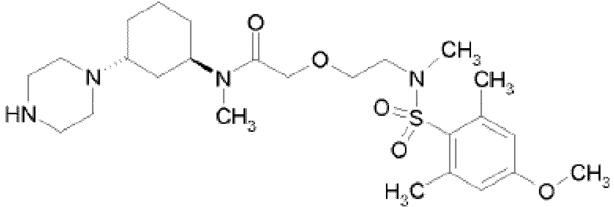 |
| (42) | 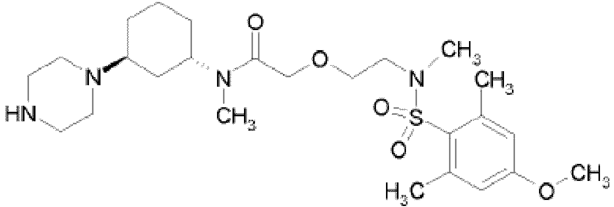 |
| (43) | 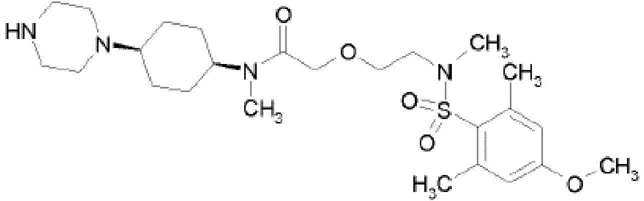 |
leurs énantiomères, leurs diastéréomères, leurs mélanges et leurs sels, en particulier leurs sels physiologiquement acceptables avec des acides ou bases organiques ou inorganiques.

6. Composés de formule générale I suivants selon la revendication 1 :
| N° | Structure |
|---|---|
| (1) | 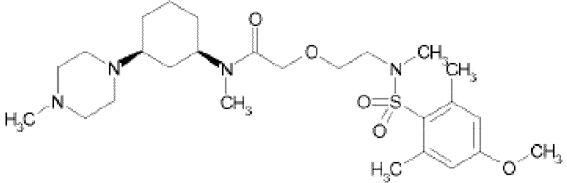 |
| (2) | 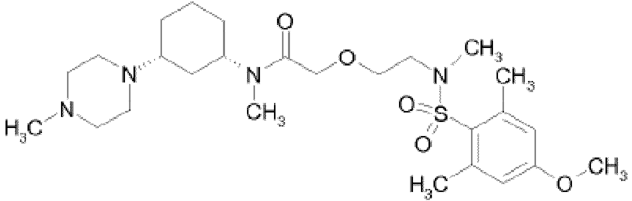 |
| (3) | 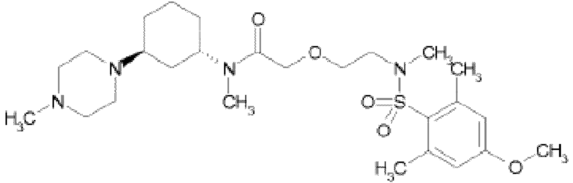 |
| (4) | 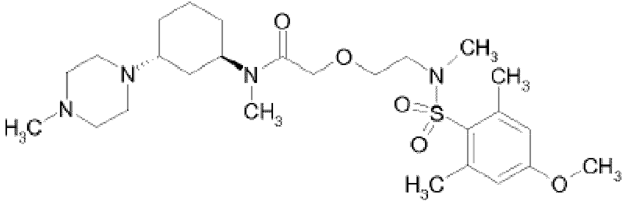 |
| (5) | 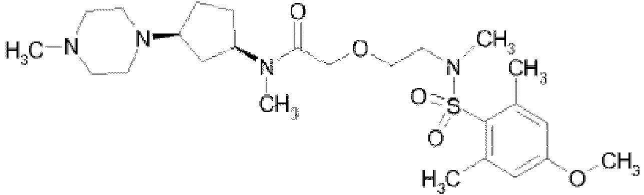 |
| (6) | 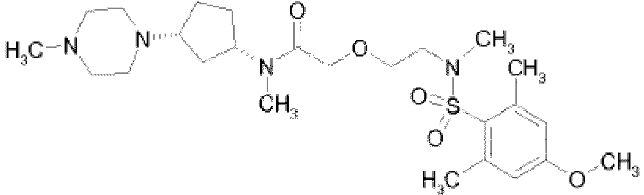 |
| (7) | 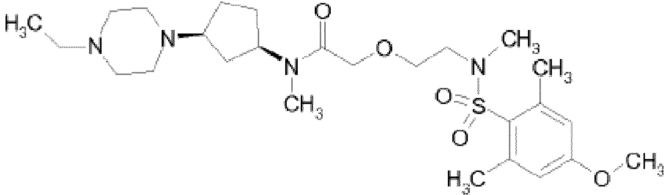 |
| (8) | 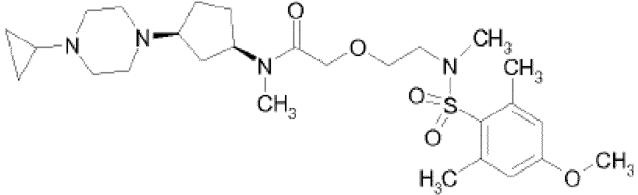 |
| (9) | 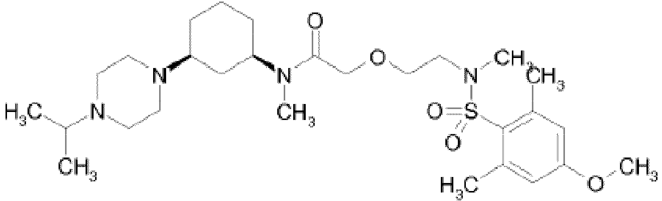 |
| (10) | 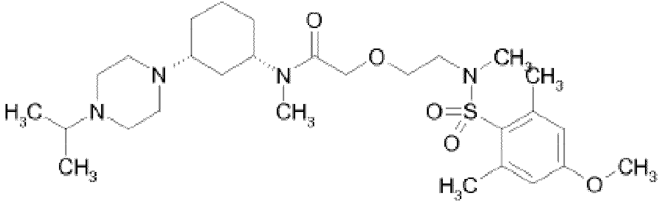 |
| (11) | 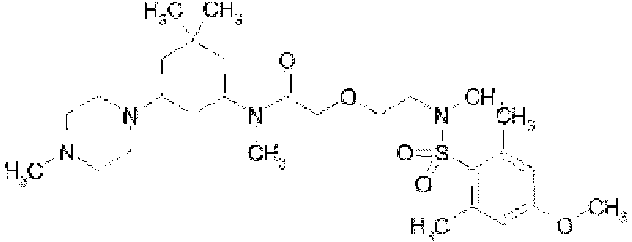 |
| (12) | 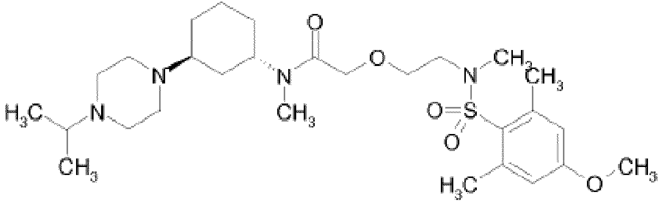 |
| (13) | 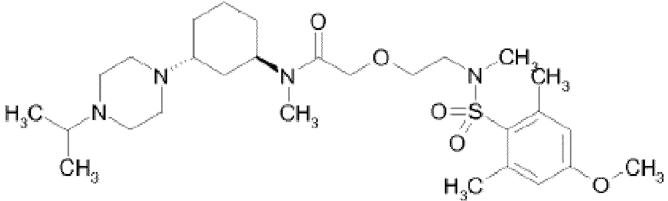 |
| (14) | 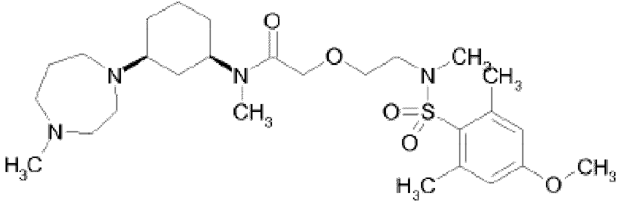 |
| (15) | 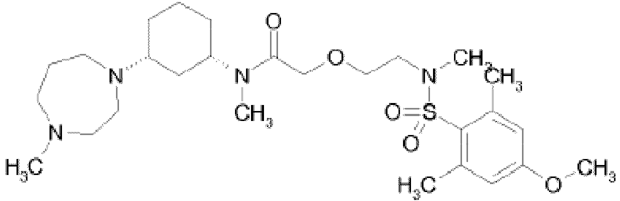 |
| (16) | 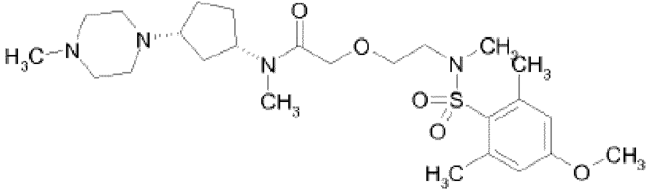 |
| (17) | 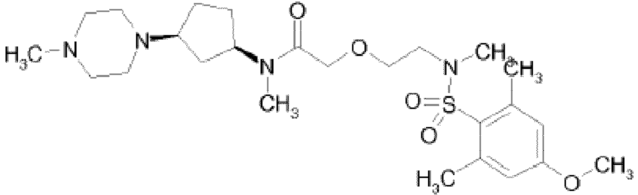 |
| (18) | 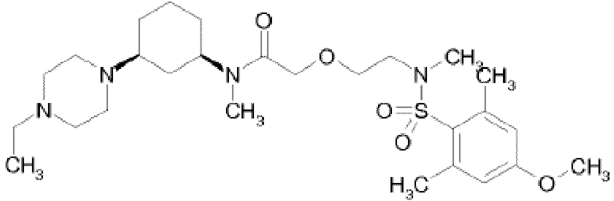 |
| (19) | 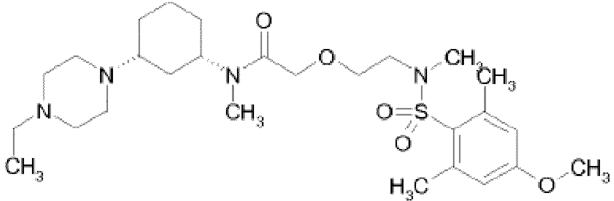 |
| (20) | 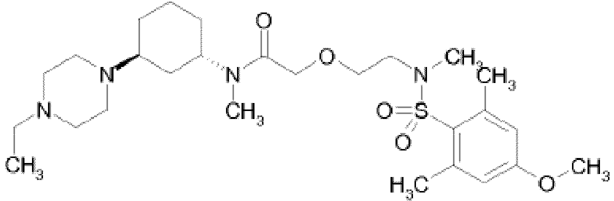 |
| (21) | 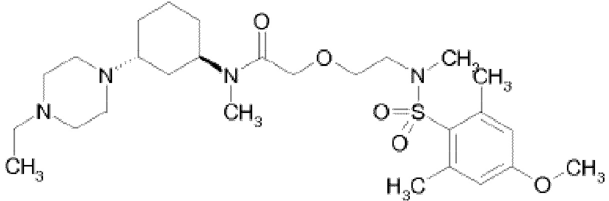 |
| (22) | 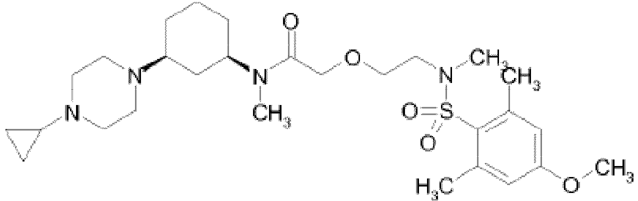 |
| (23) | 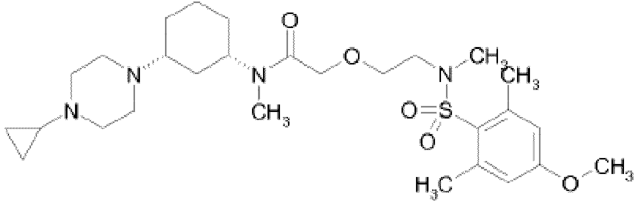 |
| (24) | 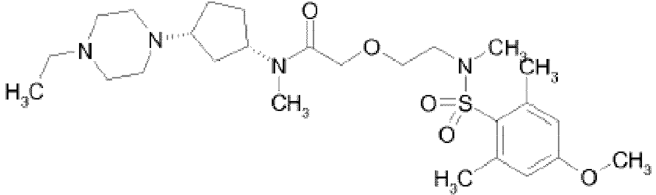 |
| (25) | 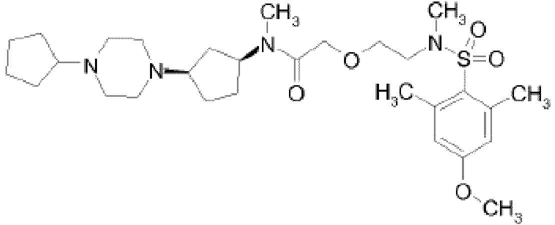 |
| (26) | 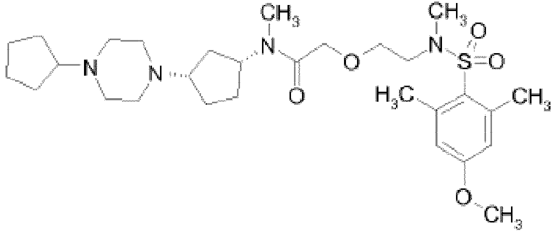 |
| (27) | 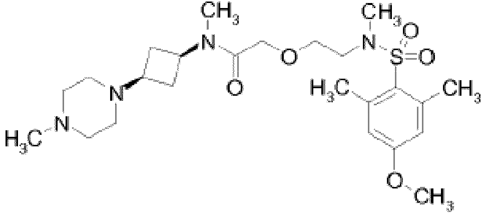 |
| (28) | 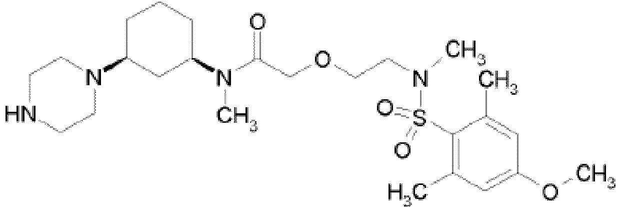 |
| (29) | 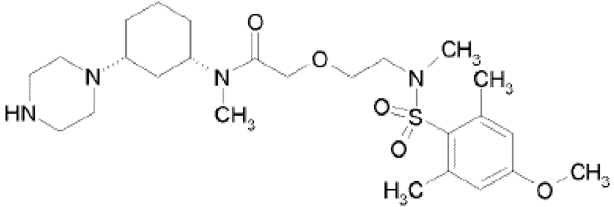 |
| (30) | 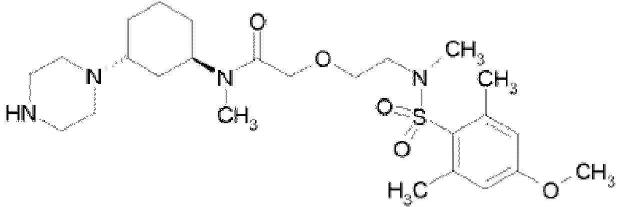 |
| (31) | 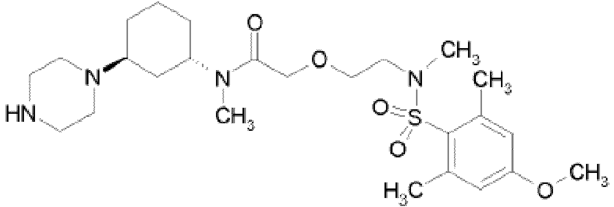 |
leurs énantiomères, leurs diastéréomères, leurs mélanges et leurs sels, en particulier leurs sels physiologiquement acceptables avec des acides ou bases organiques ou inorganiques.

7. Sels physiologiquement acceptables des composés selon l'une des revendications 1 à 6, avec des acides ou des bases inorganiques ou organiques.

8. Médicament, contenant un composé selon au moins l'une des revendications 1 à 6, ou sel physiologiquement acceptable selon la revendication 7, avec éventuellement un ou plusieurs véhicules et/ou diluants inertes.

9. Utilisation d'un composé selon au moins l'une des revendications 1 à 7, pour la production d'un médicament pour le traitement aigu et prophylactique de douleurs aiguës, de douleurs viscérales, de douleurs neuropathiques, de douleurs inflammatoires/ médiées par un récepteur nociceptif, de douleurs tumorales et de céphalées.

10. Procédé de production d'un médicament selon la revendication 8, **caractérisé en ce que**, de façon non chimique, un composé selon au moins l'une des revendications 1 à 7 est introduit dans un ou plusieurs véhicules et/ou diluants inertes.
